(19) Europäisches Patentamt / European Patent Office / Office européen des brevets

(11) **EP 4 483 895 A2**

(12) **EUROPEAN PATENT APPLICATION**

(43) Date of publication:
**01.01.2025   Bulletin 2025/01**

(21) Application number: **24211210.0**

(22) Date of filing: **26.11.2020**

(51) International Patent Classification (IPC):
**A61K 39/00** $^{(2006.01)}$

(52) Cooperative Patent Classification (CPC):
**C07K 16/2809; C07K 16/2878;** A61K 2039/505;
C07K 2317/24; C07K 2317/31; C07K 2317/33;
C07K 2317/35; C07K 2317/55; C07K 2317/64;
C07K 2317/73; C07K 2317/74; C07K 2317/76;
C07K 2317/92

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 26.11.2019   PCT/CN2019/120991
27.08.2020   PCT/CN2020/111796

(62) Document number(s) of the earlier application(s) in
accordance with Art. 76 EPC:
**20892443.1 / 4 065 164**

(71) Applicant: **Shanghai Epimab Biotherapeutics Co.,
Ltd.
Shanghai 201210 (CN)**

(72) Inventors:
- **WU, Chengbin**
**Shanghai 201210 (CN)**
- **WU, Danqing**
**Shanghai  201210 (CN)**
- **HUANG, Lini**
**Shanghai 201210 (CN)**

- **ZHANG, Amin**
**Shanghai 201210 (CN)**
- **SHUAI, Zhengrong**
**Shanghai  201210 (CN)**
- **ZHANG, Rui**
**Shanghai 201210 (CN)**
- **GONG, Shiyong**
**Shanghai 201210 (CN)**
- **WU, Xuan**
**Shanghai 201210 (CN)**

(74) Representative: **Mewburn Ellis LLP
Aurora Building
Counterslip
Bristol BS1 6BX (GB)**

Remarks:
•The complete document including Reference
Table(s) and the Sequence Listing(s) can be
downloaded from the EPO website
•This application was filed on 06-11-2024 as a
divisional application to the application mentioned
under INID code 62.

(54)   **ANTIBODIES TO CD3 AND BCMA, AND BISPECIFIC BINDING PROTEINS MADE THEREFROM**

(57)   High-affinity antibodies recognizing CD3 and B Cell Maturation Factor protein (BCMA) are disclosed. Binding sites from humanized anti-CD3 and anti-BCMA antibodies are incorporated into a Fabs-in-Tandem Immunoglobulin format without significant loss of binding affinity, and the resultant bispecific, multivalent binding proteins are able to bind to both CD3 and BCMA simultaneously. Such antibodies, antigen-binding portions thereof, and bispecific FIT-Ig binding proteins are useful for treating cancer.

EP 4 483 895 A2

## Description

### Field of the Invention

The present invention relates to new antibodies recognizing CD3, new antibodies recognizing B Cell Maturation Antigen (BCMA), and bispecific BCMA/CD3 binding proteins such as FIT-Ig binding proteins and MAT-Fab binding proteins made using those antibodies. The antibodies and bispecific binding proteins are useful for treatment of immunological diseases and hematological cancers.

### Background of the Invention

### Cluster of Differentiation 3 (CD3)

The T cell receptor (TCR) binds to antigens (Ags) displayed by major histocompatibility complexes (MHCs) and plays critical roles in T cell function. But the TCR does not possess intracellular signaling by itself. Instead, TCR non-covalently associates with the Cluster of Differentiation 3 (CD3) complex and triggers intracellular signaling through immunoreceptor tyrosine-based activation motifs (ITAM) of CD3. The CD3 T cell co-receptor helps to activate both the cytotoxic T cell (CD8+ naive T cells) and also T helper cells (CD4+ naive T cells). It consists of a protein complex and is composed of four distinct chains. In mammals, the complex contains a CD3$\gamma$ chain, a CD3$\delta$ chain, and two CD3$\epsilon$ chains. These chains associate with the T cell receptor (TCR) and the CD3$\zeta$ chain (zeta-chain) to generate an activation signal in T lymphocytes. The TCR, $\zeta$-chain, and CD3y, $\delta$, and $\epsilon$ chains together constitute the TCR complex. The CD3 four-chain complex then forms CD3$\epsilon\gamma$, CD3$\epsilon\delta$ and $\zeta\zeta$ dimers in 1:1:1 stoichiometry.

CD3 is initially expressed in the cytoplasm of pro-thymocytes, the stem cells from which T cells arise in the thymus. The pro-thymocytes differentiate into common thymocytes, and then into medullary thymocytes, and it is at this latter stage that CD3 antigen begins to migrate to the cell membrane. The antigen is found bound to the membranes of all mature T cells, and in virtually no other cell type, although it does appear to be present in small amounts in Purkinje cells.

This high specificity, combined with the presence of CD3 at all stages of T cell development, makes it a useful immunohistochemical marker for T cells in tissue sections. The antigen remains present in almost all T cell lymphomas and leukemias, and can therefore be used to distinguish them from superficially similar B cell and myeloid neoplasms. Some antibodies against CD3$\epsilon$ chain have been shown to activate TCR-CD3 complex, possibly by clustering CD3 complex on T cells. In addition, bispecific antibodies targeting both CD3 and a tumor specific antigen have been studied for redirected tumor eradication by T cells. Because CD3 is required for T cell activation, drugs (often monoclonal antibodies) that target it are being investigated as immunosuppressant therapies (e.g., otelixizumab) for type 1 diabetes and other autoimmune diseases.

### B Cell Maturation Antigen (BCMA)

B cell maturation antigen (BCMA, TNFRSF17, CD269) is a member of the TNF receptor superfamily. BCMA expression is restricted to the B cell lineage, mainly expressed on plasma cells and plasmablasts and is absent on naive B cells. BCMA binds to two ligands, A proliferation-inducing ligand (APRIL, TNFSF13, TALL-2, CD256) and B cell activation factor (BAFF, BLYS, TNFSF13B, TALL-1, CD257). BCMA has higher binding affinity for APRIL than for BAFF. Multiple myeloma (MM) cells express high levels of BCMA. Antibodies targeting BCMA with ligand blocking activity could promote cytotoxicity of MM cells both as naked IgG and as drug-conjugates. The restricted expression of BCMA on late-stage matured B cells also makes it an ideal co-target of chimeric antigen receptor T cells (CAR-T cells), which are T cells genetically engineered to exhibit chimeric antigen receptors to target the modified T cells to specific cellular proteins. CAR-T cells provide a promising immunotherapy for B cell cancers, however their mechanism of action is not well understood, and the side effects of CAR-T cell therapy are often severe and include cytokine release syndrome (cytokine storm) and neurological toxicity.

Understanding of the roles of CD3 and BCMA has also led to a related immunotherapy known as bispecific T cell redirecting antibodies. Bispecific antibodies targeting both CD3 and BCMA would be useful for the treatment of multiple myeloma through redirected T cell cytotoxicity (RTCC).

### Summary of the Invention

The present invention provides new antibodies that bind to CD3 with high affinity and new antibodies that bind to BCMA with high affinity. The invention also provides BCMA/CD3 bispecific Fabs-in-Tandem immunoglobulins (FIT-Igs) that are reactive with both CD3 and BCMA. The invention also provides BCMA/CD3 bispecific monovalent asymmetric tandem Fab antibodies (MAT-Fabs) that are reactive with both CD3 and BCMA. Antibodies and bispecific binding proteins of the present invention can activate the TCR-CD3 complex. The bispecific, multivalent binding proteins described herein will be useful as BCMA/CD3 bispecific inhibitors to provide a synergistic combination effect for the treatment of multiple myeloma (MM) cells through redirected T cell cytotoxicity.

The invention also provides methods of making and using the anti-CD3 and anti-BCMA antibodies and BCMA/CD3 bispecific binding proteins described herein as well as various compositions that may be used in methods of detecting CD3 and/or BCMA in a sample or in methods of treating or preventing a disorder in an individual that is associated with CD3 and/or BCMA activity.

In a further embodiment, the invention provides a bispecific Fabs-in-Tandem immunoglobulin (FIT-Ig) binding protein comprising first, second, and third polypeptide chains,

wherein said first polypeptide chain comprises, from amino to carboxyl terminus, (i) $VL_A$-CL-$VH_B$-CH1-Fc wherein CL is directly fused to $VH_B$, or (ii) $VH_B$-CH1-$VL_A$-CL-Fc wherein CH1 is directly fused to $VL_A$;
wherein said second polypeptide chain comprises, from amino to carboxyl terminus, $VH_A$-CH1; and
wherein said third polypeptide chain comprises, from amino to carboxyl terminus, $VL_B$-CL;
wherein VL is a light chain variable domain, CL is a light chain constant domain, VH is a heavy chain variable domain, CH1 is a heavy chain constant domain, Fc is an immunoglobulin Fc region, A is an epitope of CD3 or BCMA and B is an epitope of CD3 or BCMA, with the proviso that A and B are different. In accordance with the present invention, such FIT-Ig binding proteins bind to both CD3 and BCMA.

In one further embodiments, the Fab fragments of such FIT-Ig binding proteins incorporate $VL_A$ and $VH_A$ domains from a parental antibody binding to one of the antigen targets CD3 or BCMA and incorporate $VL_B$ and $VH_B$ domains from a different parental antibody binding to the other of the antigen targets CD3 and BCMA. Thus, $VH_A$-CH1/$VL_A$-CL and $VH_B$-CH1/$VL_B$-CL pairing of the first second and third polypeptide chains will result in tandem Fab moieties recognizing CD3 and BCMA.

In accordance with the present invention, a BCMA/CD3 FIT-Ig binding protein advantageously comprises first, second, and third polypeptide chains, wherein said first polypeptide chain comprises, from amino to carboxyl terminus, $VL_{CD3}$-CL-$VH_{BCMA}$-CH1-Fc wherein CL is directly fused to $VH_{BCMA}$, wherein said second polypeptide chain comprises, from amino to carboxyl terminus, $VH_{CD3}$-CH1; and wherein said third polypeptide chain comprises, from amino to carboxyl terminus, $VL_{BCMA}$-CL; wherein $VL_{CD3}$ is a light chain variable domain of an anti-CD3 antibody, CL is a light chain constant domain, $VH_{CD3}$ is a heavy chain variable domain of an anti-CD3 antibody, CH1 is a heavy chain constant domain, $VL_{BCMA}$ is a light chain variable domain of an anti-BCMA antibody, $VH_{BCMA}$ is a heavy chain variable domain of an anti-BCMA antibody, and Fc is an immunoglobulin Fc region. Advantageously, in the first polypeptide chain, the domains $VL_{CD3}$-CL are the same as the light chain of an anti-CD3 parental antibody, the domains $VH_{CD3}$-CH1 are the same as the heavy chain variable and heavy chain constant domains of an anti-CD3 parental antibody, the domains $VL_{BCMA}$-CL are the same as the light chain of an anti-BCMA parental antibody, and the domains $VH_{BCMA}$-CH1 are the same as the heavy chain variable and heavy chain constant domains of an anti-BCMA parental antibody.

In alternative embodiments, a BCMA/CD3 FIT-Ig binding protein may advantageously comprise first, second, and third polypeptide chains, wherein said first polypeptide chain comprises, from amino to carboxyl terminus, $VL_{BCMA}$-CL-$VH_{CD3}$-CH1-Fc wherein CL is directly fused to $VH_{CD3}$, wherein said second polypeptide chain comprises, from amino to carboxyl terminus, $VH_{BCMA}$-CH1; and wherein said third polypeptide chain comprises, from amino to carboxyl terminus, $VL_{CD3}$-CL; wherein $VL_{CD3}$ is a light chain variable domain of an anti-CD3 antibody, CL is a light chain constant domain, $VH_{CD3}$ is a heavy chain variable domain of an anti-CD3 antibody, CH1 is a heavy chain constant domain, $VL_{BCMA}$ is a light chain variable domain of an anti-BCMA antibody, $VH_{BCMA}$ is a heavy chain variable domain of an anti-BCMA antibody, and Fc is an immunoglobulin Fc region. Advantageously, in the first polypeptide chain, the domains $VL_{BCMA}$-CL are the same as the light chain of an anti-BCMA parental antibody, the domains $VH_{BCMA}$-CH1 are the same as the heavy chain variable and heavy chain constant domains of an anti-BCMA parental antibody, the domains $VL_{CD3}$-CL are the same as the light chain of an anti-CD3parental antibody, and the domains $VH_{CD3}$-CH1 are the same as the heavy chain variable and heavy chain constant domains of an anti-CD3 parental antibody.

In alternative embodiments, a BCMA/CD3 FIT-Ig binding protein may advantageously comprise first, second, and third polypeptide chains, wherein said first polypeptide chain comprises, from amino to carboxyl terminus, $VH_{BCMA}$-CH1-$VL_{CD3}$-CL-Fc wherein CH1 is directly fused to $VL_{CD3}$, wherein said second polypeptide chain comprises, from amino to carboxyl terminus, $VL_{BCMA}$-CL; and wherein said third polypeptide chain comprises, from amino to carboxyl terminus, $VH_{CD3}$-CH1; wherein $VL_{CD3}$ is a light chain variable domain of an anti-CD3 antibody, CL is a light chain constant domain, $VH_{CD3}$ is a heavy chain variable domain of an anti-CD3 antibody, CH1 is a heavy chain constant domain, $VL_{BCMA}$ is a light chain variable domain of an anti-BCMA antibody, $VH_{BCMA}$ is a heavy chain variable domain of an anti-BCMA antibody, and Fc is an immunoglobulin Fc region. Advantageously, in the first polypeptide chain, the domains $VL_{BCMA}$-CL are the same as the light chain of an anti-BCMA parental antibody, the domains $VH_{BCMA}$-CH1 are the same as the heavy chain variable and heavy chain constant domains of an anti-BCMA parental antibody, the domains $VL_{CD3}$-CL are the same as the light chain of an anti-CD3 parental antibody, and the domains $VH_{CD3}$-CH1 are the same as the heavy chain variable and heavy chain constant domains of an anti-CD3 parental antibody.

In alternative embodiments, a BCMA/CD3 FIT-Ig binding protein may advantageously comprise first, second, and third polypeptide chains, wherein said first polypeptide chain comprises, from amino to carboxyl terminus, $VH_{CD3}$-CH1-$VL_{BCMA}$-CL-Fc wherein CH1 is directly fused to $VL_{BCMA}$, wherein said second polypeptide chain comprises, from amino to carboxyl terminus, $VL_{CD3}$-CL; and wherein said third polypeptide chain comprises, from amino to carboxyl terminus, $VH_{BCMA}$-CH1; wherein $VL_{CD3}$ is a light chain variable domain of an anti-CD3 antibody, CL is a light chain constant domain, $VH_{CD3}$ is a heavy chain variable domain of an anti-CD3 antibody, CH1 is a heavy chain constant domain, $VL_{BCMA}$ is a light chain variable domain of an anti-BCMA antibody, $VH_{BCMA}$ is a heavy chain variable domain of an anti-BCMA antibody, and Fc is an immunoglobulin Fc region. Advantageously, in the first polypeptide chain, the domains $VL_{BCMA}$-CL are the same as the light chain of an anti-BCMA parental antibody, the domains $VH_{BCMA}$-CH1 are the same as the heavy chain variable and heavy chain constant domains of an anti-BCMA parental antibody, the domains $VL_{CD3}$-CL are the same as the light chain of an anti-CD3 parental antibody, and the domains $VH_{CD3}$-CH1 are the same as the heavy chain variable and heavy chain constant domains of an anti-CD3 parental antibody.

In the foregoing formulas for the first polypeptide chain of a FIT-Ig binding protein, an Fc region may be a native or a variant Fc region. In particular embodiments, the Fc region is a human Fc region from IgG1, IgG2, IgG3, IgG4, IgA, IgM, IgE, or IgD. In particular embodiments, the Fc is a human Fc from IgG1, such as set forth below:

```
DKTHTCPPCPAPEAAGGPSVFLFPPKPKDTLMISRTPEVTCVVVDVSHEDPEVKFNWYVDGVEVHNAKTKP

REEQYNSTYRVVSVLTVLHQDWLNGKEYKCKVSNKALPAPIEKTISKAKGQPREPQVYTLPPSREEMTKNQ

VSLTCLVKGFYPSDIAVEWESNGQPENNYKTTPPVLDSDGSFFLYSKLTVDKSRWQQGNVFSCSVMHEALH

NHYTQKSLSLSPGK
```
(SEQ ID NO:1).

In an embodiment of the invention, FIT-Ig binding proteins of the present invention retain one or more properties of parental antibodies from which the sequences of their Fab fragments are utilized and incorporated into the FIT-Ig structure. In one further embodiments, the FIT-Ig will retain binding affinity for the target antigens (i.e., CD3 and BCMA) comparable to that of the parental antibodies, meaning that the binding affinity of the FIT-Ig binding protein for the CD3 and BCMA antigen targets does not vary by greater than 10-fold in comparison to the binding affinity of the parental antibodies for their respective target antigens, as measured by surface plasmon resonance or biolayer interferometry.

In one embodiment, a BCMA/CD3 FIT-Ig binding protein of the present invention binds CD3 and BCMA and is comprised of a first polypeptide chain comprising, consisting essentially of, or consisting of the sequence of amino acids of SEQ ID NO:50; a second polypeptide chain comprising, consisting essentially of, or consisting of the sequence of amino acids of SEQ ID NO:51; and a third polypeptide chain comprising, consisting essentially of, or consisting of the sequence of amino acids of SEQ ID NO:52.

In another embodiment, a BCMA/CD3 FIT-Ig binding protein of the present invention binds CD3 and BCMA and is comprised of a first polypeptide chain comprising, consisting essentially of, or consisting of the sequence of amino acids of SEQ ID NO:53; a second polypeptide chain comprising, consisting essentially of, or consisting of the sequence of amino acids of SEQ ID NO:54; and a third polypeptide chain comprising, consisting essentially of, or consisting of the sequence of amino acids of SEQ ID NO:55.

In another embodiment, a BCMA/CD3 FIT-Ig binding protein of the present invention binds CD3 and BCMA and is comprised of a first polypeptide chain comprising, consisting essentially of, or consisting of the sequence of amino acids of SEQ ID NO:80; a second polypeptide chain comprising, consisting essentially of, or consisting of the sequence of amino acids of SEQ ID NO:81; and a third polypeptide chain comprising, consisting essentially of, or consisting of the sequence of amino acids of SEQ ID NO:82.

In another embodiment, a BCMA/CD3 FIT-Ig binding protein of the present invention binds CD3 and BCMA and is comprised of a first polypeptide chain comprising, consisting essentially of, or consisting of the sequence of amino acids of SEQ ID NO:83; a second polypeptide chain comprising, consisting essentially of, or consisting of the sequence of amino acids of SEQ ID NO:84; and a third polypeptide chain comprising, consisting essentially of, or consisting of the sequence of amino acids of SEQ ID NO:85.

In another embodiment, a BCMA/CD3 FIT-Ig binding protein of the present invention binds CD3 and BCMA and is comprised of a first polypeptide chain comprising, consisting essentially of, or consisting of the sequence of amino acids of SEQ ID NO:86; a second polypeptide chain comprising, consisting essentially of, or consisting of the sequence of amino acids of SEQ ID NO:87; and a third polypeptide chain comprising, consisting essentially of, or consisting of the sequence of amino acids of SEQ ID NO:88.

The invention also provides novel antibodies capable of binding human CD3, wherein the antigen-binding domain of the antibody comprises a set of six CDRs, i.e., CDR-H1, CDR-H2, CDR-H3, CDR-L1, CDR-L2, and CDR-L3, selected from the group of CDR sets defined below:

| CDR Set No. | CDR | CDR Amino Acid Sequence | SEQ ID NO: |
|---|---|---|---|
| 1 | CDR-H1 | NYYVH | SEQ ID NO:56 |
| | CDR-H2 | WISPGSDNTKYNEKFKG | SEQ ID NO:57 |
| | CDR-H3 | DDYGNYYFDY | SEQ ID NO:58 |
| | CDR-L1 | KSSQSLLNSRTRKNYLA | SEQ ID NO:59 |
| | CDR-L2 | WASTRES | SEQ ID NO:60 |
| | CDR-L3 | KQSYILRT | SEQ ID NO:61 |
| 2 | CDR-H1 | NYYIH | SEQ ID NO:62 |
| | CDR-H2 | WINLGDVNTKFNEKFKD | SEQ ID NO:63 |
| | CDR-H3 | DGYSFYYFDF | SEQ ID NO:64 |
| | CDR-L1 | KASQSLFNSRTRKNYLA | SEQ ID NO:65 |
| | CDR-L2 | WASTRES | SEQ ID NO:66 |
| | CDR-L3 | IQSHTLRT | SEQ ID NO:67 |

The invention also provides novel antibodies capable of binding human BCMA, wherein the antigen-binding domain of the antibody comprises a set of six CDRs, i.e., CDR-H1, CDR-H2, CDR-H3, CDR-L1, CDR-L2, and CDR-L3, selected from the group of CDR sets defined below:

| CDR Set No. | CDR | CDR Amino Acid Sequence | SEQ ID NO: |
|---|---|---|---|
| 3 | CDR-H1 | NYGLN | SEQ ID NO:68 |
| | CDR-H2 | WINTYSGHPTYVDDFKG | SEQ ID NO:69 |
| | CDR-H3 | EKDDGYRLGLDY | SEQ ID NO:70 |
| | CDR-L1 | SASSSVSYMY | SEQ ID NO:71 |
| | CDR-L2 | DTSNLVS | SEQ ID NO:72 |
| | CDR-L3 | LQYSGYPYT | SEQ ID NO:73 |
| 4 | CDR-H1 | NFWMH | SEQ ID NO:74 |
| | CDR-H2 | AFYPGNDDTYYNQKFK | SEQ ID NO:75 |
| | CDR-H3 | SGYYGSSDAMDY | SEQ ID NO:76 |
| | CDR-L1 | GASENIYGALN | SEQ ID NO:77 |
| | CDR-L2 | GATNLAD | SEQ ID NO:78 |
| | CDR-L3 | QSVLTTPWT | SEQ ID NO:79 |

In one embodiment, a binding protein according to the invention is a bispecific, multivalent immunoglobulin binding protein comprising two or more antigen binding sites, wherein at least one antigen binding site comprises a CDR set selected from CDR Sets 1 and 2 above and at least one antigen binding site comprises a CDR set selected from CDR Sets 3 and 4 above.

In an embodiment, an anti-CD3 antibody according to the invention comprises VH and VL domains, wherein the two variable domains comprise amino acid sequences selected from the following VH/VL pairs:

| VH/VL Pair | VH/VL Pair |
|---|---|
| SEQ ID NO:6/SEQ ID NO:7 | SEQ ID NO:19/SEQ ID NO:20 |
| SEQ ID NO:8/SEQ ID NO:9 | SEQ ID NO:11/SEQ ID NO:21 |
| SEQ ID NO:11/SEQ ID NO:20 | SEQ ID NO:12/SEQ ID NO:21 |
| SEQ ID NO:12/SEQ ID NO:20 | SEQ ID NO:13/SEQ ID NO:21 |
| SEQ ID NO:13/SEQ ID NO:20 | SEQ ID NO:14/SEQ ID NO:21 |
| SEQ ID NO:14/SEQ ID NO:20 | SEQ ID NO:15/SEQ ID NO:21 |
| SEQ ID NO:15/SEQ ID NO:20 | SEQ ID NO:16/SEQ ID NO:21 |
| SEQ ID NO:16/SEQ ID NO:20 | SEQ ID NO:17/SEQ ID NO:21 |
| SEQ ID NO:17/SEQ ID NO:20 | SEQ ID NO:18/SEQ ID NO:21 |
| SEQ ID NO:18/SEQ ID NO:20 | SEQ ID NO:19/SEQ ID NO:21 |
| SEQ ID NO:11/SEQ ID NO:25 | SEQ ID NO:15/SEQ ID NO:25 |
| SEQ ID NO:18/SEQ ID NO:25 | SEQ ID NO:12/SEQ ID NO:25. |
| SEQ ID NO:17/SEQ ID NO:25 |  |

In a further embodiment, an anti-BCMA antibody according to the invention comprises VH and VL domains, wherein the two variable domains comprise amino acid sequences selected from the following VH/VL pairs:

| VH/VL Pair | VH/VL Pair |
|---|---|
| SEQ ID NO:29/SEQ ID NO:30 | SEQ ID NO:36/SEQ ID NO:40 |
| SEQ ID NO:31/SEQ ID NO:32 | SEQ ID NO:36/SEQ ID NO:41 |
| SEQ ID NO:34/SEQ ID NO:38 | SEQ ID NO:36/SEQ ID NO:42 |
| SEQ ID NO:35/SEQ ID NO:38 | SEQ ID NO:36/SEQ ID NO:43 |
| SEQ ID NO:36/SEQ ID NO:38 | SEQ ID NO:36/SEQ ID NO:44 |
| SEQ ID NO:37/SEQ ID NO:38 | SEQ ID NO:34/SEQ ID NO:45 |
| SEQ ID NO:34/SEQ ID NO:39 | SEQ ID NO:34/SEQ ID NO:46 |
| SEQ ID NO:35/SEQ ID NO:39 | SEQ ID NO:34/SEQ ID NO:47 |
| SEQ ID NO:36/SEQ ID NO:39 | SEQ ID NO:34/SEQ ID NO:48 |
| SEQ ID NO:37/SEQ ID NO:39 | SEQ ID NO:34/SEQ ID NO:49 |

In another embodiment, an anti-CD3 antibody or an anti-BCMA antibody may be used to make derivative binding proteins recognizing the same target antigen by techniques well established in the field. Such a derivative may be, e.g., a single-chain antibody (scFv), a Fab fragment (Fab), an Fab' fragment, an F(ab')$_2$, an Fv, and a disulfide linked Fv.

A Fab fragment of an immunoglobulin is composed of two components that covalently associate to form an antibody binding site. The two components are each a variable domain-constant domain chain (VH-CH1 or VL-CL), and therefore each V-C chain of a Fab may be described as one "half" of a Fab binding unit.

In another aspect of the invention, an antibody or bispecific binding protein described herein is capable of modulating a biological function of CD3, BCMA, or both. In another aspect, an anti-CD3 antibody described herein is capable of inhibiting CD3 signaling. In another aspect, an anti-BCMA antibody described herein is capable of inhibiting BCMA interaction with its ligands APRIL and/or BAFF, and optionally an anti-BCMA antibody according to the invention is capable of inhibiting BCMA-mediated cellular signaling pathways.

In an embodiment, an anti-CD3 antibody described herein or an antigen-binding fragment thereof has an on rate constant ($k_{on}$) to human CD3 of at least $1 \times 10^5$ M$^{-1}$s$^{-1}$, for instance, at least $3.3 \times 10^5$ M$^{-1}$s$^{-1}$ or more, as measured by surface plasmon resonance or biolayer interferometry.

In another embodiment, an anti-CD3 antibody described herein or antigen-binding fragment thereof has an off rate constant ($k_{off}$) to human CD3 of less than $5 \times 10^{-3}$ as measured by surface plasmon resonance or biolayer interferometry.

In another embodiment, an anti-CD3 antibody described herein or antigen-binding fragment thereof has a dissociation constant ($K_D$) to human CD3 of less than $2 \times 10^{-8}$ M, for instance, less than $1.5 \times 10^{-8}$ M.

In an embodiment, an anti-BCMA antibody described herein or an antigen-binding fragment thereof has an on rate constant ($k_{on}$) to human BCMA of at least $4 \times 10^4 M^{-1}s^{-1}$, for instance, at least $1 \times 10^5$ $M^{-1}s^{-1}$, at least $2 \times 10^5$ $M^{-1}s^{-1}$ or more, as measured by surface plasmon resonance or biolayer interferometry.

In another embodiment, an anti-BCMA antibody described herein or antigen-binding fragment thereof has an off rate constant ($k_{off}$) to human BCMA of less than $5 \times 10^{-3}s^{-1}$, less than $1 \times 10^{-3}s^{-1}$, less than $5 \times 10^{-4}s^{-1}$, less than $2 \times 10^{-5}s^{-1}$, or less than $1 \times 10^{-5}s^{-1}$, as measured by surface plasmon resonance or biolayer interferometry.

In another embodiment, an anti-BCMA antibody described herein or antigen-binding fragment thereof has a dissociation constant ($K_D$) to BCMA of less than $2 \times 10^{-8}$ M, less than $1 \times 10^{-9}$ M, or less than $5 \times 10^{-10}$ M.

In an embodiment, a bispecific BCMA/CD3 FIT-Ig binding protein capable of binding CD3 and BCMA according to this invention has an on rate constant ($k_{on}$) to human CD3 of at least $1 \times 10^5$ $M^{-1}s^{-1}$, for instance, at least $2 \times 10^5$ $M^{-1}s^{-1}$, or at least $3 \times 10^5$ $M^{-1}s^{-1}$, or more, and the same binding protein has an on rate constant ($k_{on}$) to human BCMA of at least $5 \times 10^4 M^{-1}s^{-1}$, for instance, at least $6 \times 10^4 M^{-1}s^{-1}$, or at least $8 \times 10^4$ $M^{-1}s^{-1}$, or more, as measured by surface plasmon resonance or biolayer interferometry. In further embodiments, a bispecific BCMA/CD3 FIT-Ig binding protein capable of binding CD3 and BCMA as described herein will have an on rate constant ($k_{on}$) to human CD3 that is no more than a 10-fold decrease from the $k_{on}$ for CD3 of the parental anti-CD3 antibody, and is no more than a 10-fold decrease from the $k_{on}$ for BCMA of the parental anti-BCMA antibody from which the anti-CD3 and anti-BCMA specificities, respectively, of the FIT-Ig binding protein were derived. In other words, the FIT-Ig binding protein will retain an on rate constant for each antigen (CD3 or BCMA) that is higher than, the same as, or no more than one order of magnitude less than the on rate constant ($k_{on}$) exhibited by the parental antibodies reactive with the respective CD3 or BCMA antigens. As disclosed herein, a BCMA/CD3 FIT-Ig binding protein for antigen may show improvement in $k_{on}$ for one or both antigens in comparison to the $k_{on}$ for the respective antigens exhibited by the parental antibodies, or the $k_{on}$ for one or both antigens may be essentially the same as exhibited by the parental antibodies, respectively, or, if there is a decrease in $k_{on}$ for one or both antigens shown by the FIT-Ig binding protein in comparison to a parental antibody, then that decrease is no more than a 10-fold decrease. For instance, a decrease in $k_{on}$ for a particular antigen in the FIT-Ig in comparison to the $k_{on}$ for that antigen of a parental antibody is less than 50%, less than a 25% decrease. Such high retained $k_{on}$ values in the bispecific FIT-Ig in comparison to the $k_{on}$s of the parental antibodies is a surprising achievement in the field.

In an embodiment, a bispecific FIT-Ig binding protein capable of binding CD3 and BCMA according to this invention has an off rate constant ($k_{off}$) to human CD3 of less than $1 \times 10^{-2}s^{-1}$, less than $8 \times 10^{-3}s^{-1}$, less than $7 \times 10^{-3}s^{-1}$, for instance, less than $6 \times 10^{-3}s^{-1}$, and the same binding protein has an off rate constant ($k_{off}$) to human BCMA of less than $5 \times 10^{-5}s^{-1}$, less than $4 \times 10^{-5}s^{-1}$, less than $3 \times 10^{-5}s^{-1}$, or less than $5 \times 10^{-6}s^{-1}$, as measured by surface plasmon resonance or biolayer interferometry.

In another embodiment, a bispecific BCMA/CD3 FIT-Ig binding protein capable of binding CD3 and BCMA according to this invention has a dissociation constant ($K_D$) to CD3 of less than $5 \times 10^{-8}$ M, less than $3 \times 10^{-8}$ M, less than $2 \times 10^{-8}$ M, or less than $1.75 \times 10^{-8}$ M, and the same binding protein has a dissociation constant ($K_D$) for human BCMA of less than $1 \times 10^{-9}$ M, less than $6 \times 10^{-10}$ M, less than $3 \times 10^{-10}$ M, less than $1 \times 10^{-10}$ M, less than $8 \times 10^{-11}$ M, or less than $6 \times 10^{-11}$ M. In further embodiments, a bispecific FIT-Ig binding protein capable of binding CD3 and BCMA as described herein will have a dissociation constant ($K_D$) to human CD3 that is no more than 10-fold different from the $K_D$ for CD3 of the parental anti-CD3 antibody, and is no more than 10-fold different from the $K_D$ for BCMA of the parental anti-BCMA antibody from which the anti-CD3 and anti-BCMA specificities, respectively, of the FIT-Ig binding protein were derived. In other words, the FIT-Ig binding protein will retain the binding affinity of the parental antibodies for each antigen (CD3 or BCMA) as indicated by a dissociation constant ($K_D$) that is within one order of magnitude of the $K_D$ exhibited by the parental antibodies reactive with the CD3 or BCMA antigens, respectively.

As disclosed herein, a BCMA/CD3 FIT-Ig binding protein may show improvement in $K_D$ (i.e., has a lower $K_D$ value; more tightly binds) for one or both antigens in comparison to the $K_D$ for the respective antigens exhibited by the parental antibodies, or the $K_D$ for one or both antigens may be essentially the same as exhibited by the parental antibodies, respectively, or the $K_D$ for one or both antigens shown by the FIT-Ig binding protein may show a decrease (i.e., have a greater $K_D$ value, binds less tightly) in comparison to the $K_D$ of a parental antibody, but if there is a difference in $K_D$ between FIT-Ig binding protein and parental antibody, then that difference is no more than a 10-fold difference. For instance,, a BCMA/CD3 FIT-Ig binding protein shows a lower $K_D$ (binds more tightly) for one or both antigens in comparison to the $K_D$ for the respective antigens exhibited by the one or both parental antibodies. Retention of the binding affinity of the parental anti-CD3 and anti-BCMA antibodies $\pm$ 10-fold change in $K_D$ is a surprising achievement in the field.

The invention also provides pharmaceutical compositions comprising at least one anti-CD3 antibody or antigen-binding fragment thereof as described herein and a pharmaceutically acceptable carrier. The invention also provides pharmaceutical compositions comprising at least one anti-BCMA antibody or antigen-binding fragments thereof and a pharmaceutically acceptable carrier. The invention also provides pharmaceutical compositions comprising a combination of anti-CD3 and anti-BCMA antibodies as described herein, or antigen-binding fragment(s) thereof, and a pharmaceutically acceptable carrier. The invention also provides bispecific, multivalent immunoglobulin binding proteins reactive with both CD3 and BCMA, which binding proteins incorporate VH/VL binding sites from anti-CD3 and anti-BCMA antibodies described herein. In particular, the invention provides pharmaceutical compositions comprising at least one FIT-Ig binding protein or at least one MAT-Fab binding protein capable of binding CD3 and BCMA and a pharmaceutically acceptable carrier. Pharmaceutical compositions of the invention may further comprise at least one additional active ingredient. In an embodiment, such an additional ingredient includes, but is not limited to, a therapeutic agent, an imaging agent, a cytotoxic agent, an angiogenesis inhibitor, a kinase inhibitor, a co-stimulation molecule blocker, an adhesion molecule blocker, an antibody of different specificity or functional fragment thereof, a detectable label or reporter; an agonist or antagonist for particular cytokine(s), a narcotic, a non-steroid anti-inflammatory drug (NSAID), an analgesic, an anesthetic, a sedative, a local anesthetic, a neuromuscular blocker, an antimicrobial agent, a corticosteroid, an anabolic steroid, an erythropoietin, an immunogen, an immunosuppressive agent, a growth hormone, a hormone replacement drug, a radiopharmaceutical, an antidepressant, an antipsychotic, a stimulant (e.g., an amphetamine, caffeine, etc.), a beta agonist, an inhaled steroid, an epinephrine or analog, a cytokine.

In another embodiment, a pharmaceutical composition further comprises at least one additional therapeutic agent for treating a disorder in which CD3-mediated and/or BCMA-mediated signaling activity is detrimental.

In a further embodiment, the invention provides isolated nucleic acids encoding one or more amino acid sequences of an anti-CD3 antibody of the invention or an antigen-binding fragment thereof; isolated nucleic acids encoding one or more amino acid sequences of an anti-BCMA antibody of the invention or an antigen-binding fragment thereof; and isolated nucleic acids encoding one or more amino acid sequences of a bispecific Fabs-in-Tandem immunoglobulin (FIT-Ig) binding protein capable of binding both CD3 and BCMA. Such nucleic acids may be inserted into a vector for carrying out various genetic analyses or for expressing, characterizing, or improving one or more properties of an antibody or binding protein described herein. A vector may comprise a one or more nucleic acid molecules encoding one or more amino acid sequences of an antibody or binding protein described herein in which the one or more nucleic acid molecules is operably linked to appropriate transcriptional and/or translational sequences that permit expression of the antibody or binding protein in a particular host cell carrying the vector. Examples of vectors for cloning or expressing nucleic acids encoding amino acid sequences of binding proteins described herein include, but are not limited to, pcDNA, pTT, pTT3, pEFBOS, pBV, pJV, and pBJ, and derivatives thereof.

The invention also provides a host cell comprising a vector comprising a nucleic acid encoding one or more amino acid sequences of an antibody or binding protein described herein. Host cells useful in the invention may be prokaryotic or eukaryotic. An exemplary prokaryotic host cell is *Escherichia coli.* Eukaryotic cells useful as host cells in the invention include protist cells, animal cells, plant cells, and fungal cells. An exemplary fungal cell is a yeast cell, including *Saccharomyces cerevisiae.* An exemplary animal cell useful as a host cell according to the invention includes, but is not limited to, a mammalian cell, an avian cell, and an insect cell. Exemplary mammalian cells include, but are not limited to, CHO cells, HEK cells, and COS cells. An insect cell useful as a host cell according to the invention is an insect Sf9 cell.

In another aspect, the invention provides a method of producing anti-CD3 antibody or a functional fragment thereof comprising culturing a host cell comprising an expression vector encoding the antibody or functional fragment in culture medium under conditions sufficient to cause expression by the host cell of the antibody or fragment capable of binding CD3. In another aspect, the invention provides a method of producing anti-BCMA antibody or a functional fragment thereof comprising culturing a host cell comprising an expression vector encoding the antibody or functional fragment in culture medium under conditions sufficient to cause expression by the host cell of the antibody or fragment capable of binding BCMA. In another aspect, the invention provides a method of producing a bispecific, multivalent binding protein capable of binding CD3 and BCMA, specifically a FIT-Ig binding protein, comprising culturing a host cell comprising an expression vector encoding the FIT-Ig binding protein in culture medium under conditions sufficient to cause expression by the host cell of the binding protein capable of binding CD3 and BCMA. The proteins so produced can be isolated and used in various compositions and methods described herein.

In one embodiment, the present invention provides methods for treating cancer in a subject in need thereof, the method comprising administering to the subject an anti-CD3 antibody or CD3-binding fragment thereof as described herein, wherein the antibody or binding fragment is capable of binding CD3 and inhibiting CD3-mediated signaling in a cell expressing CD3. In another embodiment, the present invention provides methods for treating cancer in a subject in need thereof, the method comprising administering to the subject an anti-BCMA antibody or BCMA binding fragment thereof as described herein, wherein the antibody or binding fragment is capable of binding BCMA and inhibiting BCMA-mediated signaling in a cell expressing BCMA. In another embodiment, the present invention provides methods for treating cancer in a subject in need thereof, the method comprising administering to the subject a bispecific FIT-Ig binding protein capable of binding both CD3 and BCMA as described herein, wherein the binding protein is capable of binding CD3 and BCMA and of inhibiting CD3-mediated signaling in a cell expressing CD3 and of inhibiting BCMA-mediated signaling in a cell expressing BCMA.

In another embodiment, the present invention provides methods for treating an autoimmune disease or a cancer in a subject in need thereof, wherein the binding protein is capable of binding CD3 and BCMA, and wherein the autoimmune disease or cancer is an autoimmune disease or cancer typically responsive to immunotherapy. In another embodiment, the cancer is a cancer that has not been associated with immunotherapy. In another embodiment, the cancer is a cancer that is a refractory or a recurring malignancy. In another embodiment, the binding protein inhibits the growth or survival of tumor cells. In another embodiment, the cancer is selected from the group consisting of melanoma (e.g., metastatic malignant melanoma), renal cancer (e.g. clear cell carcinoma), prostate cancer (e.g. hormone refractory prostate adenocarcinoma), pancreatic adenocarcinoma, breast cancer, colon cancer, lung cancer (e.g. non-small cell lung cancer), esophageal cancer, squamous cell carcinoma of the head and neck, liver cancer, ovarian cancer, cervical cancer, thyroid cancer, glioblastoma, glioma, leukemia, lymphoma, and other neoplastic malignancies.

Methods of treatment described herein may further comprise administering to a subject in need thereof, of an immunostimulatory adjuvant, such as a CpG oligodeoxynucleotide (CpG ODN) comprising a full or partial phosphodiester or phosphorothioate backbone. For example, in a method of treatment of the invention, an immunostimulatory adjuvant may be incorporated into a composition comprising an antibody or FIT-Ig binding protein of the invention, and the composition administered to a subject in need of treatment. In another embodiment, a method of treatment of the invention may comprise a step of administering to a subject in need of treatment an antibody or FIT-Ig binding protein described herein and a separate step of administering an immunostimulatory adjuvant to the subject before, concurrently, or after the step of administering to the subject an antibody or FIT-Ig binding protein of the invention.

Brief Description of the Drawings

Figure 1 is a collection of plots showing binding of test antibodies against a human CD3$\varepsilon$/$\gamma$ heterodimer-Fc fusion protein target immobilized on microplates. The binding activities of newly isolated mAbCD3-001 and mAbCD3-002 are compared against a reference anti-CD3 monoclonal antibody ("control $\alpha$-CD3 mAb") and an irrelevant murine antibody ("mIgG") as a negative control.

Figure 2 is a collection of plots showing binding of test antibodies against a cynomolgus monkey CD3$\varepsilon$/$\gamma$ heterodimer-Fc fusion protein target immobilized on microplates. The binding activities of newly isolated mAbCD3-001 and mAbCD3-002 are compared against a reference anti-CD3 monoclonal antibody ("control $\alpha$-CD3 mAb") and an irrelevant murine antibody ("mIgG") as a negative control.

Figure 3 is a bar graph showing that *in vitro* cultured human T cells were stimulated to proliferation by anti-CD3 antibodies mAbCD3-001 (this invention) and OKT3 (positive control).

Figure 4 is a bar graph showing that *in vitro* cultured human T cells were stimulated to secrete interferon gamma (IFN-g) by anti-CD3 antibodies mAbCD3-001 (this invention) and OKT3 (positive control).

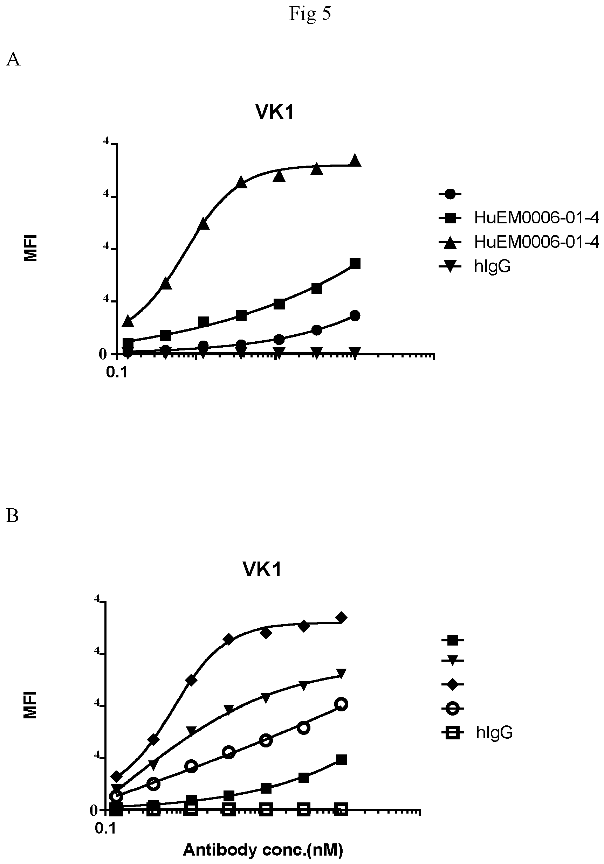

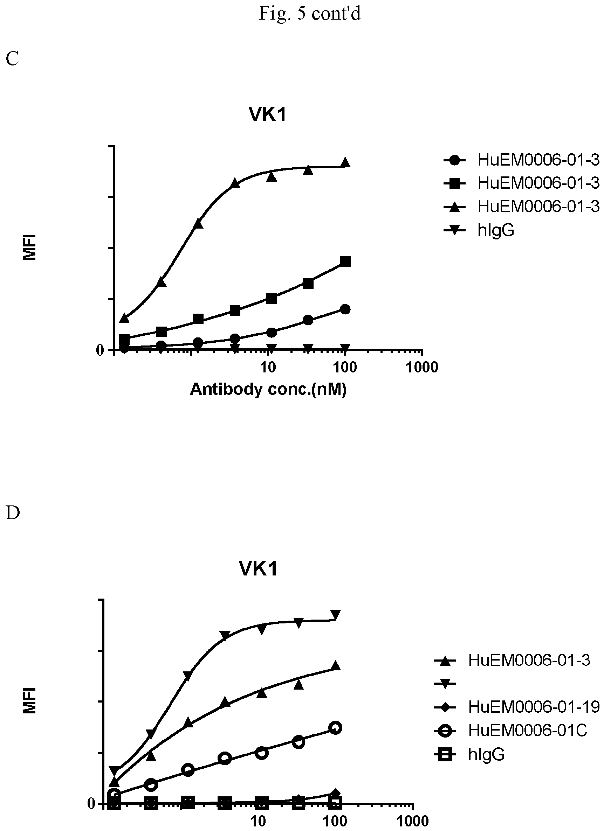

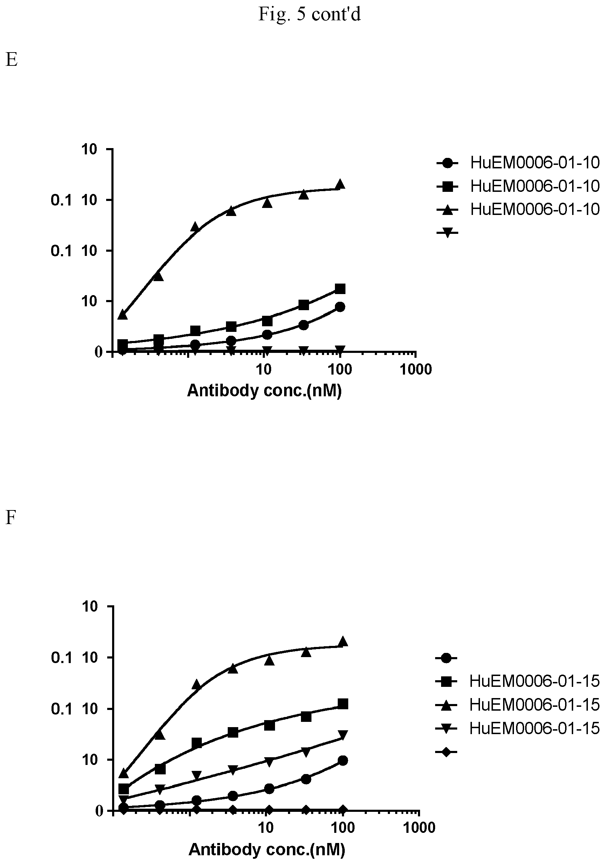

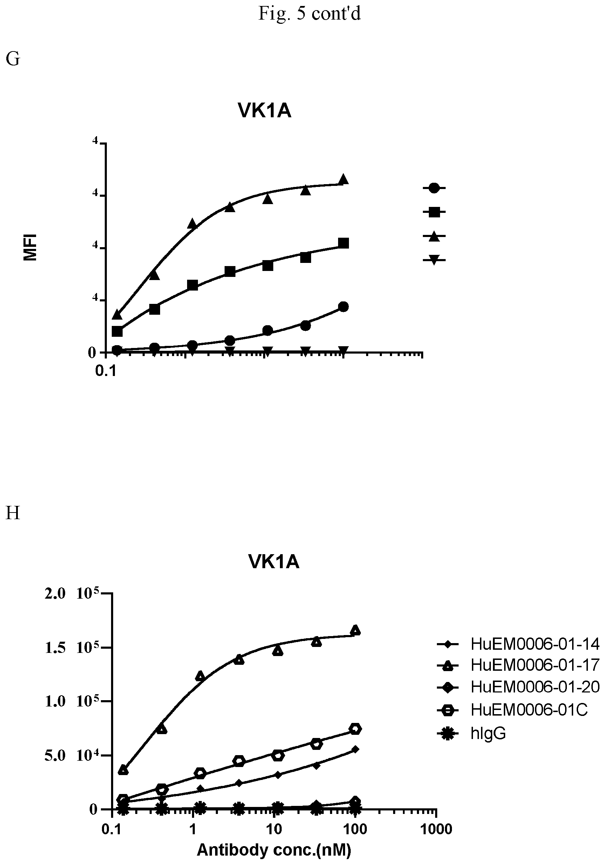

Figures 5A-5H are fluorescence plots comparing the binding activity of humanized anti-CD3 antibody constructs utilizing different humanized VH variants of mAbCD3-001 and one of two VK variants (EM0006-01VK. 1 or EM0006-01VK. 1A, see Table 2). The plots show that the VH variants were the key to CD3 binding activity, and that varying the VL had little effect on binding to Jurkat cells. Grouping several plots on graph panels 5A-5H allowed identification of humanized antibodies of very high affinity, such as HuEM0006-01-8 and HuEM0006-01-17, by contrasting with intermediate and low affinity binders.

Figure 6 is a graph showing the ability of various anti-BCMA antibodies to inhibit BCMA ligand BAFF-induced NF-$\kappa$B phosphorylation in the BCMA-expressing tumor cell line, NCI-H929. An anti-BAFF mAb and an irrelevant anti-RAC1 murine IgG were used as positive and negative controls, respectively. The novel anti-BCMA antibodies mAbBCMA-002 and mAbBCMA-003 described herein compared favorably with reference antibodies (labeled TAB1 and TAB2 in Fig. 6) described in the patent literature. See Example 3.3, *infra,* for details.

Figure 7 is a graph showing the ability of various anti-BCMA antibodies to inhibit BCMA ligand BAFF-induced NF-$\kappa$B phosphorylation in a HEK293 transfected BCMA-expressing cell line, HEK293F-BCMA-NF-kB-luc. Anti-BCMA reference antibodies TAB1 and TAB2 were used as positive controls for comparison. An irrelevant anti-Ro1 murine IgG was used as control.

Figure 8 is a graph showing the ability of anti-BCMA antibodies to inhibit BCMA ligand APRIL (TNFSF13) induced NF-kB-luciferase signal in BCMA-transfected HEK293 cells.

Figure 9 is a graph showing the binding of BCMA/CD3 bispecifc FIT-Ig Fab fragments to BCMA-expressing NCI-H929 cells. The three FIT-Fabs tested have the same BCMA binding domain. See Examples 4.1, 4.2, and 4.3.

Figure 10 is a graph showing the binding of BCMA/CD3 FIT-Ig binding proteins to CHO cells (CHOK1/CD3/TCR) transfected to express the human T cell receptor complex (see Example 1.1). The three FIT-Ig binding proteins tested have different CD3 binding sites, from three different humanized parental anti-CD3 antibodies. See Example 4.3.

Figure 11 is a graph showing the ability of BCMA/CD3 bispecific FIT-Igs and a BCMA/CD3 FIT-Fab to redirect activation of Jurkat-NFAT cells co-cultured with NCI-H929 cells. Monospecific anti-CD3 IgG (HuEM1006-01-24) and its Fab fragment (HuEM1006-01-24-Fab) were tested for comparison, and an irrelevant human IgG was used as a negative control.

Figure 12 is a graph showing the ability of BCMA/CD3 bispecific FIT-Fab binding proteins to redirect activation of Jurkat-NFAT cells co-cultured with NCI-H929. A combination of anti-BCMA and anti-CD3 monoclonal antibodies and an irrelevant Fab (FIT1002-5a-Fab) were used as controls.

Figure 13 is a graph showing the ability of various BCMA/CD3 bispecific FIT-Fabs to redirect T cell cytotoxicity to NCI-H929 cells. An irrelevant FIT-Fab (FIT1002-5a-Fab), a combination of an anti-CD3 Fab and an anti-BCMA mAb (combo), a reference anti-BCMA mAb (TAB1) alone, an anti-CD3 Fab alone (HuEM1006-01-24-Fab), and an irrelevant human IgG were used as controls.

Figure 14 is a graph showing that humanized BCMA/CD3 FIT-Igs and a BCMA/CD3 FIT-Fab demonstrate limited non-target redirected activation of Jurkat-NFAT cells when the assay is performed without BCMA-expressing NCI-H929 target cells. Anti-CD3 IgG (HuEM1006-01-24), its Fab fragment (HuEM1006-01-24-Fab), an irrelevant FIT-Ig (FIT1002-5a), and an irrelevant human IgG (hIgG) were used as controls.

Figure 15 is a graph showing that the humanized BCMA/CD3 FIT-Igs according to the invention were able to redirect T cell cytotoxicity to NCI-H929 tumor cells. A mouse-human chimeric FIT-Ig (FIT1006-4b) and an irrelevant FIT-Ig (FIT1002-5a) were used as controls.

Figure 16 is a graph showing binding activity of two BCMA/CD3 humanized bispecific FIT-Ig binding proteins described above to BCMA-expressing NCI-H929 cells. An irrelevant human IgG antibody (hIgG) was used as a control.

Figure 17 is a graph showing binding activity of two BCMA/CD3 humanized bispecific FIT-Ig binding proteins described above to CD3-expressing Jurkat cells. An irrelevant human IgG antibody (hIgG) was used as a control.

Figure 18 and 19 demonstrate the binding activity to BCMA-expressing (Fig. 18) and CD3-expressing (Fig. 19) target cells, confirm that both targets bispecific constructs of two alternative configurations.

Figure 20 demonstrates inhibition of tumor growth in human PBMC engrafted NPSG mice achieved by treatment with BCMA × CD3 FIT-Ig.

Figure 21 demonstrates B cell depletion induced by treatment with BCMA × CD3 FIT-Ig.

Figure 22 demonstrates transient loss of circulating T cells in FIT-Ig treated cynomolgus monkeys.

Detailed Description of the Invention

This invention pertains to novel anti-CD3 antibodies, novel anti-BCMA antibodies, antigen-binding portions thereof, and multivalent, bispecific binding proteins such as Fabs-in-Tandem immunoglobulins (FIT-Igs) and monovalent asymmetric tandem Fab bispecific antibody" or "MAT-Fab bispecific antibody" or, simply, a "MAT-Fab antibody". Various aspects of the invention relate to anti-CD3 and anti-BCMA antibodies and antibody fragments, FIT-Ig binding proteins, and MAT-Fab binding proteins binding to human CD3 and human BCMA, and pharmaceutical compositions thereof, as well as nucleic acids, recombinant expression vectors and host cells for making such antibodies, functional antibody fragments, and binding proteins. Methods of using the antibodies, functional antibody fragments, and bispecific binding proteins of the invention to detect human CD3, human BCMA, or both; to inhibit human CD3 and/or human BCMA activity, either *in vitro* or *in vivo;* and to treat diseases, especially cancer, that are mediated by CD3 and/or BCMA binding to their ligands, i.e., T cell receptor and A proliferation-inducing ligand (APRIL), respectively, are also encompassed by the invention.

Unless otherwise defined herein, scientific and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition. Further, unless otherwise required by context, singular terms shall include pluralities and plural terms shall include the singular. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise.

Generally, nomenclatures used in connection with, and techniques of, cell and tissue culture, molecular biology, immunology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated. Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

That the present invention may be more readily understood, select terms are defined below.

The term "polypeptide" refers to any polymeric chain of amino acids. The terms "peptide" and "protein" are used interchangeably with the term polypeptide and also refer to a polymeric chain of amino acids. The term "polypeptide" encompasses native or artificial proteins, protein fragments and polypeptide analogs of a protein amino acid sequence. The term "polypeptide" encompasses fragments and variants (including fragments of variants) thereof, unless otherwise contradicted by context. For an antigenic polypeptide, a fragment of polypeptide optionally contains at least one contiguous or nonlinear epitope of polypeptide. The precise boundaries of the at least one epitope fragment can be confirmed using ordinary skill in the art. The fragment comprises at least about 5 contiguous amino acids, such as at least about 10 contiguous amino acids, at least about 15 contiguous amino acids, or at least about 20 contiguous amino acids. A variant of a polypeptide is as described herein.

The term "isolated protein" or "isolated polypeptide" is a protein or polypeptide that by virtue of its origin or source of derivation is not associated with naturally associated components that accompany it in its native state, is substantially free of other proteins from the same species, is expressed by a cell from a different species, or does not occur in nature. Thus, a polypeptide that is chemically synthesized or synthesized in a cellular system different from the cell from which it naturally originates will be "isolated" from its naturally associated components. A protein may also be rendered substantially free of naturally associated components by isolation, using protein purification techniques well known in the art.

The term "recovering" refers to the process of rendering a chemical species such as a polypeptide substantially free of naturally associated components by isolation, e.g., using protein purification techniques well known in the art.

The term "biological activity" refers to all inherent biological properties of the anti-CD3 or anti-BCMA antibodies described herein. Biological properties of CD3 antibodies include, but are not limited to, binding to CD3 protein; biological properties of anti-BCMA antibodies include, but are not limited to, binding to, e.g., A proliferation-inducing ligand (APRIL), and/or B cell activation factor (BAFF) proteins.

The term "specific binding" or "specifically binding" in reference to the interaction of an antibody, a binding protein, or a peptide with a second chemical species, means that the interaction is dependent upon the presence of a particular structure (e.g., an antigenic determinant or epitope) on the second chemical species. For example, an antibody recognizes and binds to a specific protein structure rather than to proteins generally. If an antibody is specific for epitope "A", the presence of a molecule containing epitope A (or free, unlabeled A), in a reaction containing labeled "A" and the antibody, will reduce the amount of labeled A bound to the antibody.

The term "antibody" broadly refers to any immunoglobulin (Ig) molecule comprised of four polypeptide chains, two heavy (H) chains and two light (L) chains, or any functional fragment, mutant, variant, or derivation thereof, which retains the essential epitope binding features of an Ig molecule. Such mutant, variant, or derivative antibody formats are known in the art. Nonlimiting embodiments of which are discussed below.

In a full-length antibody, each heavy chain is comprised of a heavy chain variable region (abbreviated herein as VH) and a heavy chain constant region. The heavy chain constant region is comprised of three domains: CH1, CH2, and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDRs), interspersed with regions that are more conserved, termed framework regions (FRs). Each VH and VL is comprised of three CDRs and four FRs, arranged from amino-terminus to carboxy-terminus in the following order: FR1, CDR1, FR2, CDR2, FR3, CDR3, FR4. First, second and third CDRs of a VH domain are commonly enumerated as CDR-H1, CDR-H2, and CDR-H3; likewise, first, second and third CDRs of a VL domain are commonly enumerated as CDR-L1, CDR-L2, and CDR-L3. Immunoglobulin molecules can be of any type (e.g., IgG, IgE, IgM, IgD, IgA and IgY), class (e.g., IgG1, IgG2, IgG3, IgG4, IgA1 and IgA2) or subclass.

The term "Fc region" is used to define the C-terminal region of an immunoglobulin heavy chain, which may be generated by papain digestion of an intact antibody. The Fc region may be a native sequence Fc region or a variant Fc region. The Fc region of an immunoglobulin generally comprises two constant domains, i.e., a CH2 domain and a CH3 domain, and optionally comprises a CH4 domain, for example, as in the case of the Fc regions of IgM and IgE antibodies. The Fc region of IgG, IgA, and IgD antibodies comprises a hinge region, a CH2 domain, and a CH3 domain. In contrast, the Fc region of IgM and IgE antibodies lacks a hinge region but comprises a CH2 domain, a CH3 domain and a CH4 domain. Variant Fc regions having replacements of amino acid residues in the Fc portion to alter antibody effector function are known in the art (see, e.g., Winter *et al.*, US Patent Nos. 5,648,260 and 5,624,821). The Fc portion of an antibody mediates several important effector functions, for example, cytokine induction, ADCC, phagocytosis, complement dependent cytotoxicity (CDC), and half-life/clearance rate of antibody and antigen-antibody complexes. In some cases these effector functions are desirable for therapeutic antibody but in other cases might be unnecessary or even deleterious, depending on the therapeutic objectives. Certain human IgG isotypes, particularly IgG1 and IgG3, mediate ADCC and CDC via binding to FcyRs and complement C1q, respectively. In still another embodiment at least one amino acid residue is replaced in the constant region of the antibody, for example the Fc region of the antibody, such that effector functions of the antibody are altered. The dimerization of two identical heavy chains of an immunoglobulin is mediated by the dimerization of CH3 domains and is stabilized by the disulfide bonds within the hinge region that connects CH1 constant domains to the Fc constant domains (e.g., CH2 and CH3). The anti-inflammatory activity of IgG is completely dependent on sialylation of the N-linked glycan of the IgG Fc fragment. The precise glycan requirements for anti-inflammatory activity have been determined, such that an appropriate IgG1 Fc fragment can be created, thereby generating a fully recombinant, sialylated IgG1 Fc with greatly enhanced potency (see, Anthony et al., Science, 320:373-376 (2008)).

The terms "antigen-binding portion" and "antigen-binding fragment" or "functional fragment" of an antibody are used interchangeably and refer to one or more fragments of an antibody that retain the ability to specifically bind to an antigen, i.e., the same antigen (e.g., CD3, BCMA) as the full-length antibody from which the portion or fragment is derived. It has been shown that the antigen-binding function of an antibody can be performed by fragments of a full-length antibody. Such antibody embodiments may also be bispecific, dual specific, or multispecific formats; specifically binding to two or more different antigens (e.g., CD3 and a different antigen, such as BCMA). Examples of binding fragments encompassed within the term "antigen-binding portion" of an antibody include (i) a Fab fragment, a monovalent fragment consisting of the VL, VH, CL, and CH1 domains; (ii) a F(ab')$_2$ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., Nature, 341: 544-546 (1989); PCT Publication No. WO 90/05144), which comprises a single variable domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see, for example, Bird et al., Science, 242: 423-426 (1988); and Huston et al., Proc. Natl. Acad. Sci. USA, 85: 5879-5883 (1988)). Such single chain antibodies are also intended to be encompassed within the term "antigen-binding portion" of an antibody and equivalent terms given above. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see, for example, Holliger et al., Proc. Natl. Acad. Sci. USA, 90: 6444-6448 (1993). Such antibody binding portions are known in the art (Kontermann and Dübel eds., Antibody Engineering (Springer-Verlag, New York, 2001), p. 790 (ISBN 3-540-41354-5)). In addition, single chain antibodies also include "linear antibodies" comprising a pair of tandem Fv segments (VH-CH1-VH-CH1) which, together with complementary light chain polypeptides, form a pair of antigen binding regions (Zapata et al., Protein Eng., 8(10): 1057-1062 (1995); and US Patent No. 5,641,870)).

An immunoglobulin constant (C) domain refers to a heavy (CH) or light (CL) chain constant domain. Murine and human IgG heavy chain and light chain constant domain amino acid sequences are known in the art.

The term "monoclonal antibody" or "mAb" refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic determinant (epitope). Furthermore, in contrast to polyclonal antibody preparations that typically include different antibodies directed against different determinants (epitopes), each mAb is directed against a single determinant on the antigen. The modifier "monoclonal" is not to be construed as requiring production of the antibody by any particular method.

The term "human antibody" includes antibodies having variable and constant regions derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo), for example in the CDRs and in particular CDR3. However, the term "human antibody" does not include antibodies in which CDR sequences derived from the germline of another mammalian species, such as a mouse, have been grafted onto human framework sequences.

The term "recombinant human antibody" includes all human antibodies that are prepared, expressed, created or isolated by recombinant means, such as antibodies expressed using a recombinant expression vector transfected into a host cell, antibodies isolated from a recombinant, combinatorial human antibody library (Hoogenboom, H.R., Trends Biotechnol., 15: 62-70 (1997); Azzazy and Highsmith, Clin. Biochem., 35: 425-445 (2002); Gavilondo and Larrick, BioTechniques, 29: 128-145 (2000); Hoogenboom and Chames, Immunol. Today, 21: 371-378 (2000)), antibodies isolated from an animal (e.g., a mouse) that is transgenic for human immunoglobulin genes (see, e.g., Taylor et al., Nucl.

Acids Res., 20: 6287-6295 (1992); Kellermann and Green, Curr. Opin. Biotechnol., 13: 593-597 (2002); Little et al., Immunol. Today, 21: 364-370 (2000)); or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

The term "chimeric antibody" refers to antibodies that comprise heavy and light chain variable region sequences from one species and constant region sequences from another species, such as antibodies having murine heavy and light chain variable regions linked to human constant regions.

The term "CDR-grafted antibody" refers to antibodies that comprise heavy and light chain variable region sequences from one species but in which the sequences of one or more of the CDR regions of VH and/or VL are replaced with CDR sequences of another species, such as antibodies having human heavy and light chain variable regions in which one or more of the human CDRs has been replaced with murine CDR sequences.

The term "humanized antibody" refers to antibodies that comprise heavy and light chain variable region sequences from a non-human species (e.g., a mouse) but in which at least a portion of the VH and/or VL sequence has been altered to be more "human-like", i.e., more similar to human germline variable sequences. One type of humanized antibody is a CDR-grafted antibody, in which CDR sequences from a non-human species (e.g., mouse) are introduced into human VH and VL framework sequences. A humanized antibody is an antibody or a variant, derivative, analog or fragment thereof which immunospecifically binds to an antigen of interest and which comprises framework regions and constant regions having substantially the amino acid sequence of a human antibody but complementarity determining regions (CDRs) having substantially the amino acid sequence of a non-human antibody. As used herein, the term "substantially" in the context of a CDR refers to a CDR having an amino acid sequence at least 80%, at least 85%, at least 90%, at least 95%, at least 98% or at least 99% identical to the amino acid sequence of a non-human antibody CDR. A humanized antibody comprises substantially all of at least one, and typically two, variable domains (Fab, Fab', $F(ab')_2$, Fv) in which all or substantially all of the CDR regions correspond to those of a non-human immunoglobulin (i.e., donor antibody) and all or substantially all of the framework regions are those of a human immunoglobulin consensus sequence. In an embodiment, a humanized antibody also comprises at least a portion of an immunoglobulin constant region (Fc), typically that of a human immunoglobulin. In some embodiments, a humanized antibody contains both the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific embodiments, a humanized antibody only contains a humanized variable domain of a light chain and/or humanized heavy chain.

A humanized antibody may be selected from any class of immunoglobulins, including IgM, IgG, IgD, IgA and IgE, and any isotype, including without limitation IgG1, IgG2, IgG3, and IgG4. The humanized antibody may comprise sequences from more than one class or isotype, and particular constant domains may be selected to optimize desired effector functions using techniques well known in the art.

The framework and CDR regions of a humanized antibody need not correspond precisely to the parental sequences, e.g., the donor antibody CDR or the acceptor framework may be mutagenized by substitution, insertion and/or deletion of at least one amino acid residue so that the CDR or framework residue at that site does not correspond to either the donor antibody or the consensus framework. In an exemplary embodiment, such mutations, however, will not be extensive. Usually, at least 80%, for instance, at least 85%, at least 90%, or at least 95% of the humanized antibody residues will correspond to those of the parental FR and CDR sequences. Back mutation at a particular framework position to restore the same amino acid that appears at that position in the donor antibody is often utilized to preserve a particular loop structure or to correctly orient the CDR sequences for contact with target antigen.

The term "CDR" refers to the complementarity determining regions within antibody variable domain sequences. There are three CDRs in each of the variable regions of the heavy chain and the light chain, which are designated CDR-H1, CDR-H2, CDR-H3, CDR-L1, CDR-L2, and CDR-L3. The term "CDR set" as used herein refers to a group of three CDRs that occur in a single variable region capable of binding the antigen. The exact boundaries of these CDRs have been defined differently according to different systems. The system described by Kabat (Kabat et al., Sequences of Proteins of Immunological Interest (National Institutes of Health, Bethesda, Maryland (1987) and (1991)) not only provides an unambiguous residue numbering system applicable to any variable region of an antibody, but also provides precise residue boundaries defining the three CDRs.

The term "Kabat numbering", which is recognized in the art, refers to a system of numbering amino acid residues which are more variable (i.e., hypervariable) than other amino acid residues in the heavy and light chain variable regions of an antibody or an antigen-binding portion thereof. See, Kabat et al., Ann. NY Acad. Sci., 190: 382-391 (1971); and Kabat et al., Sequences of Proteins of Immunological Interest, Fifth Edition, U.S. Department of Health and Human Services, NIH Publication No. 91-3242 (1991).

The growth and analysis of extensive public databases of amino acid sequences of variable heavy and light regions over the past twenty years have led to the understanding of the typical boundaries between framework regions (FRs) and CDR sequences within variable region sequences and have enabled persons skilled in the art to accurately determine the CDRs according to Kabat numbering, Chothia numbering, or other systems. See, e.g., Martin, "Protein Sequence and Structure Analysis of Antibody Variable Domains," In Kontermann and Dübel, eds., Antibody Engineering (Springer-Verlag, Berlin, 2001), chapter 31, pages 432-433.

The term "multivalent binding protein" denotes a binding protein comprising two or more antigen binding sites. A multivalent binding protein is preferably engineered to have three or more antigen binding sites, and is generally not a naturally occurring antibody. The term "bispecific binding protein" refers to a binding protein capable of binding two targets of different specificity. "Fabs-in-Tandem immunoglobulin" (FIT-Ig) binding proteins of the invention comprise two or more antigen binding sites and are typically tetravalent binding proteins. A FIT-Ig may be monospecific, i.e., capable of binding one antigen, or multispecific, i.e., capable of binding two or more antigens. An exemplary FIT-Ig according to this invention binds both CD3 and BCMA and, therefore, is bispecific. A FIT-Ig binding protein comprising two long (heavy) V-C-V-C-Fc chain polypeptides and four short (light) V-C chain polypeptides forms a hexamer exhibiting four Fab antigen binding sites (VH-CH1 paired with VL-CL, sometimes notated VH-CH1::VL-CL). Each half of a FIT-Ig comprises a heavy chain polypeptide and two light chain polypeptides, and complementary immunoglobulin pairing of the VH-CH1 and VL-CL elements of the three chains results in two Fab-structured antigen binding sites, arranged in tandem. In the present invention, it is preferred that the immunoglobulin domains comprising the Fab elements are directly fused in the heavy chain polypeptide, without the use of interdomain linkers. That is, the N-terminal V-C element of the long (heavy) polypeptide chains is directly fused at its C-terminus to the N-terminus of another V-C element, which in turn is linked to a C-terminal Fc region. In bispecific FIT-Ig binding proteins, the tandem Fab elements will be reactive with different antigens. Each Fab antigen binding site comprises a heavy chain variable domain and a light chain variable domain with a total of six CDRs per antigen binding site. In some embodiment, the multivalent binding protein of this invention is a FIT-Ig Fab fragment (i.e., FIT-Fab), which is virtually a FIT-Ig without the C-terminal Fc region. Such FIT-Fab can be obtained by removal of the C-terminal Fc region from an existing FIT-Ig, or produced by any of a number of techniques known in the art, for example, expression from host cells comprising expression vector(s) encoding the corresponding peptide chains.

A description of the design, expression, and characterization of FIT-Ig molecules is provided in PCT Publication WO 2015/103072. An example of such FIT-Ig molecules comprises a heavy chain and two different light chains. The heavy chain comprises the structural formula $VL_A$-CL-$VH_B$-CH1-Fc where CL is directly fused to $VH_B$ or $VH_B$-CH1-$VL_A$-CL-Fc where CH1 is directly fused to $VL_A$, wherein $VL_A$ is a variable light domain from a parental antibody that binds antigen A, $VH_B$ is a variable heavy domain from a parental antibody that binds antigen B, CL is a light chain constant domain, CH1 is a heavy chain constant domain, and Fc is an immunoglobulin Fc region (e.g., the C-terminal hinge-CH2-CH3 portion of a heavy chain of an IgG1 antibody). The two light polypeptide chains of the FIT-Ig have the formulas $VH_A$-CH1 and $VL_B$-CL, respectively. In bispecific FIT-Ig embodiments, antigen A and antigen B are different antigens, or different epitopes of the same antigen. In the present invention, one of A and B is CD3 and the other is BCMA.

As used herein, the term "fused directly" or "directly fused", when referring to the linear connection of two domains in a polypeptide structure, means that the domains are joined directly by a peptide bond, without the use of an artificial polypeptide linker or connector.

The term "activity" includes properties such as the ability to bind a target antigen with specificity, the affinity of an antibody for an antigen, the ability to neutralize the biological activity of a target antigen, the ability to inhibit interaction of a target antigen with its natural receptor(s), and the like. Exemplary antibodies and binding proteins of the present invention have the ability to inhibit CD3 binding to its ligand, the ability to inhibit BCMA binding to its ligand, or both in the case of bispecific binding proteins described herein.

The term "$k_{on}$" (also "Kon", "kon"), as used herein, is intended to refer to the on rate constant for association of a binding protein (e.g., an antibody) to an antigen to form an association complex, e.g., antibody/antigen complex, as is known in the art. The "$k_{on}$" also is known by the terms "association rate constant", or "ka", as used interchangeably herein. This value indicates the binding rate of an antibody to its target antigen or the rate of complex formation between an antibody and antigen as is shown by the equation below:

Antibody ("Ab") + Antigen ("Ag")→Ab-Ag.

The term "$k_{off}$" (also "Koff", "koff"), as used herein, is intended to refer to the off rate constant for dissociation, or "dissociation rate constant", of a binding protein (e.g., an antibody) from an association complex (e.g., an antibody/antigen complex) as is known in the art. This value indicates the dissociation rate of an antibody from its target antigen or separation of Ab-Ag complex over time into free antibody and antigen as shown by the equation below:

$$Ab + Ag \leftarrow Ab\text{-}Ag.$$

The term "$K_D$" (also "$K_d$"), as used herein, is intended to refer to the "equilibrium dissociation constant" and refers to the value obtained in a titration measurement at equilibrium, or by dividing the dissociation rate constant ($k_{off}$) by the association rate constant ($k_{on}$). The association rate constant ($k_{on}$), the dissociation rate constant ($k_{off}$), and the equilibrium dissociation constant ($K_D$) are used to represent the binding affinity of an antibody to an antigen. Methods for determining association and dissociation rate constants are well known in the art. Using fluorescence-based techniques offers high sensitivity and the ability to examine samples in physiological buffers at equilibrium. Other experimental approaches and instruments such as a BIAcore® (biomolecular interaction analysis) assay can be used (e.g., instrument available from BIAcore International AB, a GE Healthcare company, Uppsala, Sweden). Biolayer interferometry (BLI) using, e.g., the Octet® RED96 system (Pall Fort6Bio LLC), is another affinity assay technique. Additionally, a KinExA® (Kinetic Exclusion Assay) assay, available from Sapidyne Instruments (Boise, Idaho) can also be used.

The term "isolated nucleic acid" shall mean a polynucleotide (e.g., of genomic, cDNA, or synthetic origin, or some combination thereof) that, by human intervention, is not associated with all or a portion of the polynucleotides with which it is found in nature; is operably linked to a polynucleotide that it is not linked to in nature; or does not occur in nature as part of a larger sequence.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence. "Operably linked" sequences include both expression control sequences that are contiguous with the gene of interest and expression control sequences that act *in trans* or at a distance to control the gene of interest. The term "expression control sequence" as used herein refers to polynucleotide sequences that are necessary to effect the expression and processing of coding sequences to which they are ligated. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance protein stability; and when desired, sequences that enhance protein secretion. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include components whose presence is essential for expression and processing, and can also include additional components whose presence is advantageous, for example, leader or signal sequences and fusion partner sequences.

"Transformation", as defined herein, refers to any process by which exogenous DNA enters a host cell. Transformation may occur under natural or artificial conditions using various methods well known in the art. Transformation may rely on any known method for the insertion of foreign nucleic acid sequences into a prokaryotic or eukaryotic host cell. The method is selected based on the host cell being transformed and may include, but is not limited to, transfection, viral infection, electroporation, lipofection, and particle bombardment. Such "transformed" cells include stably transformed cells in which the inserted DNA is capable of replication either as an autonomously replicating plasmid or as part of the host chromosome. They also include cells which transiently express the inserted DNA or RNA for limited periods of time.

The term "recombinant host cell" (or simply "host cell"), is intended to refer to a cell into which exogenous DNA has been introduced. In an embodiment, the host cell comprises two or more (e.g., multiple) nucleic acids encoding antibodies, such as the host cells described in US Patent No. 7,262,028, for example. Such terms are intended to refer not only to the particular subject cell, but also to the progeny of such a cell. Because certain modifications may occur in succeeding generations due to either mutation or environmental influences, such progeny may not, in fact, be identical to the parent cell, but are still included within the scope of the term "host cell" as used herein. In an embodiment, host cells include prokaryotic and eukaryotic cells selected from any of the Kingdoms of life. In another embodiment, eukaryotic cells include protist, fungal, plant and animal cells. In another embodiment, host cells include but are not limited to the prokaryotic cell line *Escherichia coli;* mammalian cell lines CHO, HEK 293, COS, NS0, SP2 and PER.C6; the insect cell line Sf9; and the fungal cell *Saccharomyces cerevisiae.*

Standard techniques may be used for recombinant DNA, oligonucleotide synthesis, and tissue culture and transformation (e.g., electroporation, lipofection). Enzymatic reactions and purification techniques may be performed according to manufacturer's specifications or as commonly accomplished in the art or as described herein. The foregoing techniques and procedures may be generally performed according to conventional methods well known in the art and as described in various general and more specific references that are cited and discussed throughout the present specification. See e.g., Sambrook et al., Molecular Cloning: A Laboratory Manual, 2nd ed. (Cold Spring Harbor Laboratory Press, Cold Spring Harbor, N.Y., 1989).

The term "agonist", as used herein, refers to a modulator that, when contacted with a molecule of interest, causes an increase in the magnitude of a certain activity or function of the molecule compared to the magnitude of the activity or function observed in the absence of the agonist. The terms "antagonist" and "inhibitor", as used herein, refer to a modulator that, when contacted with a molecule of interest causes a decrease in the magnitude of a certain activity or function of the molecule compared to the magnitude of the activity or function observed in the absence of the antagonist. Particular antagonists of interest include those that block or reduce the biological or immunological activity of human CD3 and human BCMA.

As used herein, the term "effective amount" refers to the amount of a therapy that is sufficient to reduce or ameliorate the severity and/or duration of a disorder or one or more symptoms thereof; prevent the advancement of a disorder; cause regression of a disorder; prevent the recurrence, development, or progression of one or more symptoms associated with a disorder; detect a disorder; or enhance or improve the prophylactic or therapeutic effect(s) of another therapy (e.g., prophylactic or therapeutic agent).

Production of Anti-CD3 and Anti-BCMA Antibodies

Anti-CD3 and anti-BCMA antibodies of the present invention may be produced by any of a number of techniques known in the art. For example, expression from host cells, wherein expression vector(s) encoding the heavy and light chains is (are) transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection, and the like. Although it is possible to express the antibodies of the invention in either prokaryotic or eukaryotic host cells, expression of antibodies in eukaryotic cells is preferable, and for instance, in mammalian host cells, because such eukaryotic cells (and in particular mammalian cells) are more likely than prokaryotic cells to assemble and secrete a properly folded and immunologically active antibody.

Exemplary mammalian host cells for expressing the recombinant antibodies of the invention include Chinese Hamster Ovary (CHO cells) (including dhfr⁻ CHO cells, described in Urlaub and Chasin, Proc. Natl. Acad. Sci. USA, 77: 4216-4220 (1980), used with a DHFR selectable marker, e.g., as described in Kaufman and Sharp, J. Mol. Biol., 159: 601-621 (1982)), NS0 myeloma cells, COS cells, and SP2 cells. When recombinant expression vectors encoding antibody genes are introduced into mammalian host cells, the antibodies are produced by culturing the host cells for a period of time sufficient to allow for expression of the antibody in the host cells or, secretion of the antibody into the culture medium in which the host cells are grown. Antibodies can be recovered from the culture medium using standard protein purification methods.

Host cells can also be used to produce functional antibody fragments, such as Fab fragments or scFv molecules. It will be understood that variations on the above procedure are within the scope of the present invention. For example, it may be desirable to transfect a host cell with DNA encoding functional fragments of either the light chain and/or the heavy chain of an antibody of this invention. Recombinant DNA technology may also be used to remove some, or all, of the DNA encoding either or both of the light and heavy chains that is not necessary for binding to the antigens of interest. The molecules expressed from such truncated DNA molecules are also encompassed by the antibodies of the invention. In addition, bifunctional antibodies may be produced in which one heavy and one light chain are an antibody of the invention and the other heavy and light chain are specific for an antigen other than the antigens of interest by crosslinking an antibody of the invention to a second antibody by standard chemical crosslinking methods.

In an exemplary system for recombinant expression of an antibody, or antigen-binding portion thereof, of the invention, a recombinant expression vector encoding both the antibody heavy chain and the antibody light chain is introduced into dhfr⁻ CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transfected host cells are cultured to allow for expression of the antibody heavy and light chains and intact antibody is recovered from the culture medium. Standard molecular biology techniques are used to prepare the recombinant expression vector, transfect the host cells, select for transfectants, culture the host cells and recover the antibody from the culture medium. Still further the invention provides a method of making a recombinant anti-CD3 or anti-BCMA antibody of the invention by culturing a transfected host cell of the invention in a suitable culture medium until a recombinant antibody of the invention is produced. The method can further comprise isolating the recombinant antibody from the culture medium.

Production of Bispecific FIT-Igs Binding CD3 and BCMA

This invention provides Fabs-in-Tandem immunoglobulin binding proteins (FIT-Igs) that bind to both CD3 and BCMA. An exemplary embodiment of such FIT-Ig molecules comprises (1) a heavy polypeptide chain that comprises either the structural formula (i) $VL_A$-CL-$VH_B$-CH1-Fc wherein CL is directly fused to $VH_B$, or the structural formula (ii) $VH_B$-CH1-$VL_A$-CL-Fc wherein CH1 is directly fused to $VL_A$; (2) a light polypeptide chain of the formula $VH_A$-CH1; and (3) another light polypeptide chain of the formula $VL_B$-CL,
wherein VL is a light chain variable domain, CL is a light chain constant domain, VH is a heavy chain variable domain, CH1 is a heavy chain constant domain, Fc is an immunoglobulin Fc region, A is an epitope of CD3 or BCMA and B is an epitope of CD3 or BCMA, with the proviso that A and B are different. In accordance with the present invention, such FIT-Ig binding proteins bind to both CD3 and BCMA.

When expressed recombinantly in a suitable host cell, the three chains of a FIT-Ig typically associate, in the same manner as a natural immunoglobulin, into a six-chain, multivalent, monomeric protein wherein two of such heavy chains (1), two of such light chains (2), and two of such light chains (3), associate to form a six-chain binding protein monomer exhibiting four functional Fab antigen binding sites. Such a FIT-Ig binding protein comprises two identical subunits, wherein each subunit comprises one heavy chain (1), one light chain (2), and one light chain (3) that together form a pair of Fab binding sites arranged in tandem. Pairing of the Fc regions of two such heavy chain subunits yields a six-chain, bispecific, FIT-Ig binding protein of the invention having a total of four functional Fab binding units.

It is possible to use a peptide linker on the heavy chain to separate the tandemly connected Fab moieties, however for bispecific FIT-Igs according to the invention the omission of such linker sequences is preferred. Whereas in multivalent engineered immunoglobulin formats having tandem binding sites, it was commonly understood in the field that the adjacent binding sites would interfere with each other unless a flexible linker was used to separate the binding sites spatially. It has been discovered for the BCMA/CD3 FIT-Igs of the present invention, however, that the arrangement of the immunoglobulin domains according to the chain formulas given above results in polypeptide chains that are well-expressed in transfected mammalian cells, assemble appropriately, and are secreted as bispecific, multivalent immunoglobulin-like binding proteins that bind the target antigens CD3 and BCMA. Despite the absence of any linker sequences between the Fab binding sites, the BCMA/CD3 FIT-Igs of the invention retain the binding affinities for the target antigens, exhibiting comparable binding affinities to the parental mAbs. Moreover, omission of synthetic linker sequences from the binding proteins can avoid the creation of antigenic sites recognizable by mammalian immune systems, and in this way the elimination of linkers decreases possible immunogenicity of the FIT-Igs and leads to a half-life in circulation that is like a natural antibody, that is, the FIT-Ig is not rapidly cleared through immune opsonization and capture in the liver.

Each variable domain (VH or VL) in a FIT-Ig may be obtained from one or more "parental" monoclonal antibodies that bind one of the target antigens, i.e., CD3 or BCMA. FIT-Ig binding proteins are advantageously produced using variable domain sequences of anti-CD3 and anti-BCMA monoclonal antibodies as disclosed herein. For instance, the parental antibodies are humanized antibodies. Variable domains may also be prepared or improved using affinity maturation techniques.

An aspect of the present invention pertains to selecting parental antibodies with at least one or more properties desired in the FIT-Ig molecule. In an embodiment, the antibody properties are selected from the group consisting of antigen specificity, affinity to antigen, potency, biological function, epitope recognition, stability, solubility, production efficiency, lack of immunogenicity, pharmacokinetics, bioavailability, tissue cross-reactivity, and orthologous antigen binding. CD3 and BCMA are both cell surface proteins, and interaction with their respective ligands touches off intracellular signaling pathways; accordingly, optimal bispecific BCMA/CD3 FIT-Igs and FIT-Fabs of the invention will be able to inhibit or block CD3-mediated and/or BCMA-mediated signaling.

Antibodies, functional fragments thereof, and binding proteins according to the invention may be purified (for an intended use) by using one or more of a variety of methods and materials available in the art for purifying antibodies and binding proteins. Such methods and materials include, but are not limited to, affinity chromatography (e.g., using resins, particles, or membranes conjugated to Protein A, Protein G, Protein L, or a specific ligand of the antibody, functional fragment thereof, or binding protein), ion exchange chromatography (for example, using ion exchange particles or membranes), hydrophobic interaction chromatography ("HIC"; for example, using hydrophobic particles or membranes), ultrafiltration, nanofiltration, diafiltration, size exclusion chromatography ("SEC"), low pH treatment (to inactivate contaminating viruses), and combinations thereof, to obtain an acceptable purity for an intended use. A non-limiting example of a low pH treatment to inactivate contaminating viruses comprises reducing the pH of a solution or suspension comprising an antibody, functional fragment thereof, or binding protein of the invention to pH 3.5 with 0.5 M phosphoric acid, at 18°C - 25°C, for 60 to 70 minutes.

Uses of Antibodies and Binding Proteins of the Invention

Given their ability to bind to human CD3 and/or BCMA, the antibodies described herein, functional fragments thereof, and bispecific multivalent binding proteins described herein can be used to detect CD3 or BCMA, or both, e.g., in a biological sample containing cells that express one or both of those target antigens. The antibodies, functional fragments, and binding proteins of the invention can be used in a conventional immunoassay, such as an enzyme linked immunosorbent assay (ELISA), a radioimmunoassay (RIA), or tissue immunohistochemistry. The invention provides a method for detecting CD3 or BCMA in a biological sample comprising contacting a biological sample with an antibody, antigen-binding portion thereof, or binding protein of the invention and detecting whether binding to a target antigen occurs, thereby detecting the presence or absence of the target in the biological sample. The antibody, functional fragment, or binding protein may be directly or indirectly labeled with a detectable substance to facilitate detection of the bound or unbound antibody/fragment/binding protein. Suitable detectable substances include various enzymes, prosthetic groups, fluorescent materials, luminescent materials and radioactive materials. Examples of suitable enzymes include horseradish peroxidase, alkaline phosphatase, $\beta$-galactosidase, or acetylcholinesterase. Examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; and examples of suitable radioactive material include $^{3}$H, $^{14}$C, $^{35}$S, $^{90}$Y, $^{99}$Tc, $^{111}$In, $^{125}$I, $^{131}$I, $^{177}$Lu, $^{166}$Ho, or $^{153}$Sm.

The antibodies, functional fragments thereof, and binding proteins of the invention preferably are capable of neutralizing human CD3 and/or human BCMA activity both *in vitro* and *in vivo*. Accordingly, the antibodies, functional fragments thereof, and binding proteins of the invention can be used to inhibit human CD3 and/or human BCMA activity, e.g., inhibit cell signaling mediated by CD3/T cell interaction and/or BCMA/B cell interaction in a cell culture containing CD3-expressing and/or BCMA-expressing cells, in human subjects, or in other mammalian subjects having CD3 or BCMA with which an antibody, functional fragment thereof, or binding protein of the invention cross-reacts.

In another embodiment, the invention provides a method for treating a subject suffering from a disease or disorder in which CD3 and/or BCMA activity is detrimental, such method comprising administering to the subject an antibody or binding protein of the invention in an effective amount, such that activity mediated by CD3 binding and/or BCMA binding in the subject is reduced.

As used herein, the term "a disorder in which CD3 and/or BCMA activity is detrimental" is intended to include diseases and other disorders in which the interaction of CD3 with a CD3 ligand or the interaction of BCMA with a BCMA ligand in a subject suffering from the disorder is either responsible for the pathophysiology of the disorder or is a factor that contributes to a worsening of the disorder. Accordingly, a disorder in which CD3 and/or BCMA activity is detrimental is a disorder in which inhibition of CD3 and/or BCMA activity is expected to alleviate the symptoms and/or progression of the disorder.

In another embodiment, the present invention provides methods for treating an autoimmune disease or a cancer in a subject in need thereof, comprising administering to the subject an antibody, functional fragment thereof, or a binding protein described herein that is capable of binding CD3, BCMA, or both CD3 and BCMA, and wherein the autoimmune disease or cancer is a disease that is responsive to immunotherapy. In another embodiment, a method of the invention is used for treating an autoimmune disease or cancer that has not been associated with immunotherapy. In another embodiment, a method of the invention is used for treating a cancer that is a refractory or a recurring malignancy. In another embodiment, a CD3 or BCMA antibody, functional fragment thereof, or a BCMA/CD3 bispecific binding protein of the invention is used in a method that inhibits the growth or survival of tumor cells.

In another embodiment, the invention provides a method for treating cancer in a subject comprising the step of administering to the subject an antibody to CD3 or BCMA described herein, a functional fragment thereof, or a BCMA/CD3 bispecific binding protein described herein, e.g., such as a Fabs-in-tandem immunoglobulin (FIT-Ig) binding protein, or a MAT-Fab binding protein wherein the cancer is selected from any of a group consisting of: a melanoma (e.g., metastatic malignant melanoma), a renal cancer (e.g., clear cell carcinoma), a prostate cancer (e.g. hormone refractory prostate adenocarcinoma), a pancreatic adenocarcinoma, a breast cancer, a colon cancer, a lung cancer (e.g. non-small cell lung cancer), an esophageal cancer, a squamous cell carcinoma of the head and neck, a liver cancer, an ovarian cancer, a cervical cancer, a thyroid cancer, a glioblastoma, a glioma, a leukemia, a lymphoma, a primary bone cancer (e.g., osteosarcoma, Ewing sarcoma, malignant fibrous histiocytoma, and chondrosarcoma), a metastatic cancer, and other neoplastic malignancies.

The invention also provides pharmaceutical compositions comprising an antibody, or antigen-binding portion thereof, or a bispecific multivalent binding protein of the invention (i.e., the primary active ingredient), or a bispecific monovalent binding protein of the invention and a pharmaceutically acceptable carrier. The pharmaceutical compositions comprising proteins of the invention are for use in, but not limited to, diagnosing, detecting, or monitoring a disorder; treating, managing, or ameliorating a disorder or one or more symptoms thereof; and/or research. In a specific embodiment, a composition comprises one or more antibodies or binding proteins of the invention. In another embodiment, the pharmaceutical composition comprises one or more antibodies or binding proteins of the invention and one or more prophylactic or therapeutic agents other than antibodies or binding proteins of the invention for treating a disorder in which CD3 and/or BCMA activity is detrimental. In an embodiment, the prophylactic or therapeutic agents are known to be useful for or have been or currently are being used in the prevention, treatment, management, or amelioration of a disorder or one or more symptoms thereof. In accordance with these embodiments, the composition may further comprise a carrier, diluent. or excipient. An excipient is generally any compound or combination of compounds that provides a desired feature to a composition other than that of the primary active ingredient (i.e., other than an antibody, functional portion thereof, or binding protein of the invention).

The antibodies (including functional fragments thereof) and binding proteins of the invention can be incorporated into pharmaceutical compositions suitable for administration to a subject. Typically, the pharmaceutical composition comprises an antibody or binding protein of the invention and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols (such as, mannitol or sorbitol), or sodium chloride in the composition. Pharmaceutically acceptable carriers may further comprise minor amounts of auxiliary substances such as wetting or emulsifying agents, preservatives, or buffers, which enhance the shelf life or effectiveness of the antibody or binding protein present in the composition.

A pharmaceutical composition of the invention is formulated to be compatible with its intended route of administration. Examples of routes of administration include, but are not limited to, parenteral (e.g., intravenous, intradermal, subcutaneous, intramuscular), oral, intranasal (e.g., inhalation), transdermal (e.g., topical), intratumoral, transmucosal, and rectal administration. In a specific embodiment, the composition is formulated in accordance with routine procedures as a pharmaceutical composition adapted for intravenous, subcutaneous, intramuscular, oral, intranasal, or topical administration to human beings. Typically, compositions for intravenous administration are solutions in sterile isotonic aqueous buffer. Where necessary, the composition may also include a solubilizing agent and a local anesthetic, such as lidocaine (xylocaine, lignocaine), to ease pain at the site of the injection.

The method of the invention may comprise administration of a composition formulated for parenteral administration by injection (e.g., by bolus injection or continuous infusion). Formulations for injection may be presented in unit dosage form (e.g., in ampoules or in multidose containers) with an added preservative. The compositions may take such forms as suspensions, solutions or emulsions in oily or aqueous vehicles, and may contain formulatory agents such as suspending, stabilizing and/or dispersing agents. Alternatively, the primary active ingredient may be in powder form for constitution with a suitable vehicle (e.g., sterile pyrogen-free water) before use.

The methods of the invention may additionally comprise administration of compositions formulated as depot preparations. Such long acting formulations may be administered by implantation (e.g., subcutaneously or intramuscularly) or by intramuscular injection. Thus, for example, the compositions may be formulated with suitable polymeric or hydrophobic materials (e.g., as an emulsion in an acceptable oil) or ion exchange resins, or as sparingly soluble derivatives (e.g., as a sparingly soluble salt).

An antibody, functional fragment thereof, or binding protein of the invention also can be administered with one or more additional therapeutic agents useful in the treatment of various diseases. Antibodies, functional fragments thereof, and binding proteins described herein can be used alone or in combination with an additional agent, e.g., a therapeutic agent, said additional agent being selected by the skilled artisan for its intended purpose. For example, the additional agent can be a therapeutic agent art-recognized as being useful to treat the disease or condition being treated by the antibody or binding protein of the present invention. The additional agent also can be an agent that imparts a beneficial attribute to the therapeutic composition, e.g., an agent that affects the viscosity of the composition.

Having now described the present invention in detail, the same will be more clearly understood by reference to the following examples, which are included for purposes of illustration only and are not intended to be limiting of the invention.

EXAMPLES

Example 1: Generation of Anti-human CD3 Monoclonal Antibodies

Anti-human CD3 monoclonal antibodies were generated as follows:

Example 1.1: Immunization with Human CD3 Antigen

Anti-CD3 antibodies were obtained by immunizing groups often Balb/c and SJL/J mice (Shanghai Laboratory Animal Center) with an alternative immunization strategies. Four different CD3 immunogens were used, which included 2 peptides (CD3ε fragments: LSLKEFSELEQSGYYVC (SEQ ID NO:2) and QDGNEEMGGITQTPYK (SEQ ID NO:3), a recombinant huCD3εγ/Fc fusion protein (a fusion protein heterodimer: first chain, QDGNEEMGGITQTPYKVSISGTTVILTCPQYPGSEILWQHNDKNIGGDEDDKNIGSDEDH LSLKEFSELEQSGYYVCYPRGSKPEDANFYLYLRARVCENCMEMDGSSGSSDKTHTCPP CPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREP QVYTLPPCRDELTKNQVSLWCLVKGFYPSDIAVEWESNGQPENNYKTTPPVLDSDGSFFL YSKLTVDKSRWQQGNVFSCSVMHEALHNHYTQKSLSLSPGK (SEQ ID NO:4) and second chain, QSIKGNHLVKVYDYQEDGSVLLTCDAEA-KNITWFKDGKMIGFLTEDKKKWNLGSNAKD PRGMYQCKGSQNKSKPLQVYYRMCQNCIELNAATISGSSGSSDKTH-TCPPCPAPELLGGP SVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVCTLPPSRDE LTKNQVSLSCAVKGFYPSDIAVEWESNGQPENNYKTTPPVLDSDGSFFLVSKLTVDKSRW QQGNVFSCSVMHEALH-NHYTQKSLSLSPGK (SEQ ID NO:5)), and a CHO cell line (CHOK1/CD3/TCR) transfected with full-length human CD3γ chain, CD3ε chain, CD3δ chain,CD3ζ chain (zeta-chain), TCRα chain, and TCRβ chain, to express the human T cell receptor complex.

Example 1.2: Generation of Hybridomas

Mice were immunized at 2-week intervals with one of the immunogens (some groups being boosted with a different immunogen than used in the initial immunization) and monitored for serum titer once a week after the second injection. After 4 to 6 immunizations, splenocytes were harvested and fused with mouse myeloma cells to form hybridoma cell lines. Supernatants of hybridoma cells were then screened against the huCD3/Fc dimer target and counter-selected with an irrelevant protein/Fc dimer to identify cell lines that produce CD3-specific mouse antibodies. Positive hybridomas were tested in a cell binding assay against Jurkat cell and TCR complex-transfected CHO cell targets to confirm cell surface binding of antibodies. Finally, confirmed cell binders were then tested for cynomolgus cross-reactivity by ELISA with a cynoCD3εγ/Fc fusion protein as a target, and a Jurkat-NFAT-Luciferase reporter cell line was used to characterize anti-CD3 agonistic activity. Only two of the hybridomas tested in this manner were producing monoclonal antibodies that tested positively in all the assays. These were designated mAbCD3-001 and mAbCD3-002.

Example 1.3: Heavy and light chain variable region sequences

To amplify heavy and light chain variable regions, total RNA of each hybridoma clone was isolated from more than $5\times10^6$ cells with TRIzol™ RNA extraction reagent (Invitrogen, Cat. #15596018). cDNA was synthesized using an Invitrogen™ SuperScript™ III First-Strand Synthesis SuperMix kit (ThermoFisher Scientific Cat. #18080) following manufacturer's instructions, and the cDNAs encoding the variable regions for light and heavy mouse immunoglobulin chains were amplified using a MilliporeSigma™ Novagen™ Mouse Ig-Primer Set (Fisher Scientific Cat. #698313). PCR products were analyzed by electrophoresis on a 1.2% agarose gel with SYBR™ Safe DNA gel stain (ThermoFisher Cat. #S33102). DNA fragments with correct size were purified using a NucleoSpin® Gel and PCR Clean-up kit (Macherey-Nagel Cat. #740609) according to manufacturer's instructions and were subcloned into pMD18-T vector individually. Fifteen colonies from each transformation were selected and sequences of insert fragments were analyzed by DNA sequencing. Sequences were confirmed if at least 8 colony fragments matched consensus sequences for VH and VL. The protein sequences of murine monoclonal antibody variable regions were analyzed by sequence homology alignment and listed in Table 1. Complementarity determining regions (CDRs) are underlined based on Kabat numbering.

Table 1. Variable region sequences of 2 murine anti-CD3 antibodies

| antibody | domain | SEQ ID NO. | amino acid sequence 123456789012345678901234567890 |
|---|---|---|---|
| mAbCD3-001 | VH | 6 | QVQLQQSGPDLVKPGASVKISCKASGFSFT<u>NYYVH</u>WMKQR PGQGLEWIG<u>WISPGSDNTKYNEKFKG</u>KATLTADTSSTTAY MQLSSLTSEDSAVYYCAR<u>DDYGNYYFDY</u>WGRGSTLTVSS |
| | VL | 7 | DIVMSQSPSSLAVSAGEKVTMTCKSS<u>QSLLNSRTRKNYLA</u> WYQQKPGQSPKLLIY<u>WASTRES</u>GVPDRFTGSGSGTDFTLT ISSVQAEDLAVYYC<u>KQSYILRT</u>FGGGTKLEIK |
| mAbCD3-002 | VH | 8 | QVQLQQSGPELVKPGASVRISCKASGYTFT<u>NYYIH</u>WVKQR PGQGLEWIG<u>WINLGDVNTKFNEKFKD</u>KATLTADKSSTTAY MQLSSLTSEDSAVYFCAR<u>DGYSFYYFDF</u>WGQGTTLTVSS |
| | VL | 9 | DIVMSQSPSSLAVSVGEKVTMSC<u>KASQSLFNSRTRKNYLA</u> WYQQKPGQSPKLLIY<u>WASTRES</u>GVPDRFTGSGSGTDFIFT ISSVQAEDLAIYYC<u>IQSHTLRT</u>FGGGTKLEIK |

Example 1.4: Anti-CD3 antibodies bind both human and cynomolgus CD3

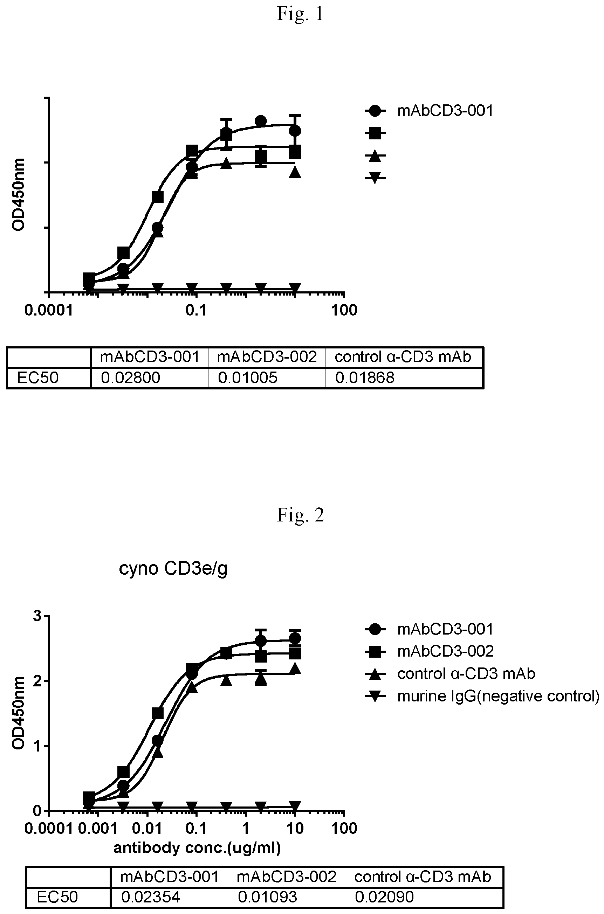

The binding properties of the isolated murine anti-CD3 antibodies were measured with ELISA as follows: Heterodimeric CD3εγ/Fc fusion protein was coated at 1 μg/mL on 96-well plates at 4° C overnight. Plates were washed once with washing buffer (PBS containing 0.05% Tween 20) and blocked with ELISA blocking buffer (1% BSA in PBS containing 0.05% Tween 20) at room temperature for 2 hours. Anti-CD3 antibodies were then added and incubated at 37° C for 1 hour. Plates were washed three times with washing buffer. HRP labeled antimouse IgG secondary antibody (Sigma, Cat. #A0168) was added and the plates were incubated at 37° C for 30 minutes then washed 5 times in washing buffer. 100 μl of tetramethylbenzidine (TMB) chromogenic solution was added to each well. Following color development, the reaction was stopped with 1 Normal HCl and absorbance at 450 nm was measured on a Varioskan™ LUX microplate reader (ThermoFisher Scientific). Binding signals were plotted against antibody concentration with GraphPad Prism 5.0 software and EC50s were calculated accordingly. As shown in Figure 1 and Figure 2, mAbCD3-002 showed the highest binding activity, while mAbCD3-001 had similar binding EC50 to a reference anti-CD3ε antibody expressed using variable domain sequences reported in US Patent No.8,236,308).

Example 1.5: Anti-CD3 antibodies activate human T cells *in vitro*

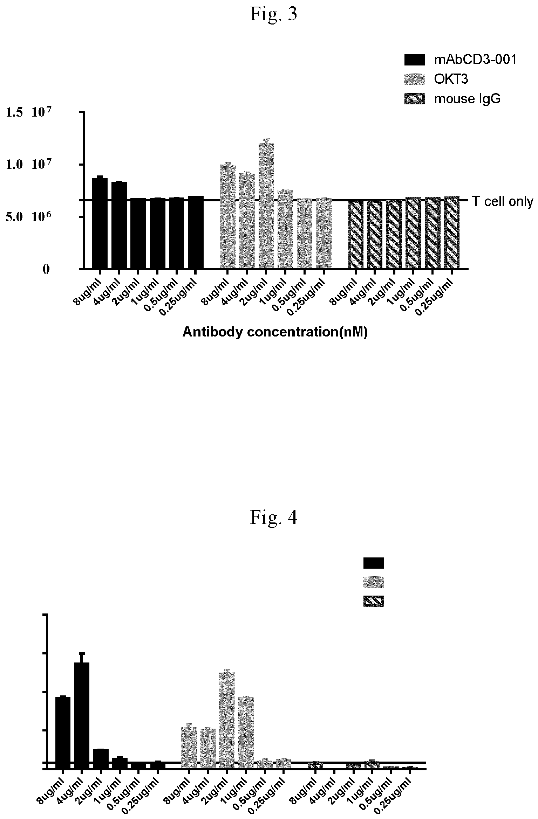

Peripheral Blood Mononuclear Cells (PBMCs) were obtained from healthy donors by using Lymphoprep™ mononuclear cell isolation medium (STEMCELL Technologies, Cat. #07851) and T cells were isolated from PBMCs with a CD3-negative T cell selection kit (EasySep™ STEMCELL Technologies, Cat. #17951). Proliferation (CTG) and cytokine (IFN-γ) production data were acquired using the following protocol: 100 μl of test antibody (mAbCD3-001, OKT3, or negative control IgG) were coated on a high-binding 96-well plate (Nunc™, Cat. #3361) at 4° C overnight followed by DPBS washing. Commercially available OKT3 antibody was used as a positive anti-CD3 control and an irrelevant mouse IgG was used as negative control. For each well, $1 \times 10^5$ T cells in 200 μl culture medium (RPMI1640 + 10% FBS + 1% Penicillin-Streptomycin solution + 1% GlutaMAX™ supplement) were seeded and incubated at 37° C for 96 hrs. Proliferation was measured by using an ATP catalyzed quantification kit (CellTiter-Glo™, Promega). IFN-γ was measured by a LANCE® (Lanthanide Chelate Excite) TR-FRET assay kit (PerkinElmer, Cat. #TRF1217M). Data were analyzed with GraphPad Prism 5.0 software. The results are shown in Figures 3 and 4. The cell proliferation and IFN-γ production data indicated that mAbCD3-001 activates human T cells *in vitro.*

Example 2: Humanization of anti-CD3 antibodies

Example 2.1: Humanization of mAbCD3-001

The anti-CD3 mAbCD3-001 variable region genes were employed to create a humanized mAb. In the first step of this process, the amino acid sequences of the VH and VK of mAbCD3-001 (see Table 1, *supra*) were compared against the available database of human Ig V-gene sequences in order to find the overall best-matching human germline Ig V-gene sequences. Additionally, the framework 4 of VH or VL was compared against the J-region database to find the human framework having the highest homology to the murine VH and VL regions, respectively. For the light chain, the closest human V-gene match was the B3 gene (V-base database), and for the heavy chain the closest human match was the VH1-2 gene. Humanized variable domain sequences were then designed where the CDR-L1, CDR-L2 and CDR-L3 of the mAbCD3-001 light chain were grafted onto framework sequences of the B3 gene, with JK4 framework 4 sequence after CDR-L3; and the CDR-H1, CDR-H2, and CDR-H3 sequences of the mAbCD3-001 VH were grafted onto framework sequences of the VH1-2, with JH6 framework 4 sequence after CDR-H3. A 3D Fv model of mAbCD3-001 was then generated and analyzed to determine if there were any framework residues that have a less than 4Å distance to the CDR residues and that were most likely critical to support loop structures or the VH/VL interface. These residues in humanized sequences should be back-mutated to mouse residues at the same position to retain affinity/activity. Q1E mutation was always included to eliminate N-terminal pyroglutamate formation if applicable. In the case of the heavy chain, potential mutations of Y27F, T28S, V37M, M48I, V67A, M69L and R71A (Kabat numbering) were identified. In the case of the light chain, T5S and N22T were identified for back mutation. Via the importance tier of each back mutation, as determined by its interaction with CDRs, the most important back mutations were introduced in the humanized VH sequence in order of priority, followed by other back mutations in successive designs. Also, the VK CDR-L1 sequence had a NS pattern that was a potential deamidation site. To remove this asparagine deamidation liability, NS was mutated to QS, NT or NA in the humanized kappa chain. The humanized VH and VL constructs are shown in Table 2 (below). (Back mutated framework amino acid residues are indicated with double underscore; murine CDRs from the original parental antibody are underlined.)

Table 2: Humanized VH/VL design of anti-CD3 antibody mAbCD3-001

| humanized VH and VL domains | SEQ ID NO. | amino acid sequence 1234567890123456789012345678901234567890 |
|---|---|---|
| EM0006-01VH.1 | 10 | EVQLVQSGAEVKKPGASVKVSCKASGYTFTNYYVHWVRQA PGQGLEWMGWISPGSDNTKYNEKFKGRVTMTRDTSISTAY MELSRLRSDDTAVYYCARDDYGNYYFDYWGQGTTVTVSS |
| EM0006-01VH.1A | 11 | EVQLVQSGAEVKKPGASVKVSCKASGYTFTNYYVHWVRQA PGQGLEWMGWISPGSDNTKYNEKFKGRVTLTADTSISTAY MELSRLRSDDTAVYYCARDDYGNYYFDYWGQGTTVTVSS |
| EM0006-01VH.1B | 12 | EVQLVQSGAEVKKPGASVKVSCKASGYTFTNYYVHWVRQA PGQGLEWIGWISPGSDNTKYNEKFKGRVTLTADTSISTAY MELSRLRSDDTAVYYCARDDYGNYYFDYWGQGTTVTVSS |
| EM0006-01VH.1C | 13 | EVQLVQSGAEVKKPGASVKVSCKASGYTFTNYYVHWVRQA PGQGLEWIGWISPGSDNTKYNEKFKGKATLTADTSISTAY MELSRLRSDDTAVYYCARDDYGNYYFDYWGQGTTVTVSS |
| EM0006-01VH.1D | 14 | EVQLVQSGAEVKKPGASVKVSCKASGYTFTNYYVHWMRQA PGQGLEWIGWISPGSDNTKYNEKFKGKATLTADTSISTAY MELSRLRSDDTAVYYCARDDYGNYYFDYWGQGTTVTVSS |
| EM0006-01VH.1E | 15 | EVQLVQSGAEVKKPGASVKVSCKASGYTFTNYYVHWMRQA PGQGLEWIGWISPGSDNTKYNEKFKGRVTLTADTSISTAY MELSRLRSDDTAVYYCARDDYGNYYFDYWGQGTTVTVSS |
| EM0006-01VH.1F | 16 | EVQLVQSGAEVKKPGASVKVSCKASGFSFTNYYVHWMRQA PGQGLEWIGWISPGSDNTKYNEKFKGKATLTADTSISTAY MELSRLRSDDTAVYYCARDDYGNYYFDYWGQGTTVTVSS |

(continued)

| humanized VH and VL domains | SEQ ID NO. | amino acid sequence 123456789012345678901234567890 1234567890 |
|---|---|---|
| EM0006-01VH.1G | 17 | EVQLVQSGAEVKKPGASVKVSCKASGFSFTNYYVHWMRQA PGQGLEWIGWISPGSDNTKYNEKFKGRVTLTADTSISTAY MELSRLRSDDTAVYYCARDDYGNYYFDYWGQGTTVTVSS |
| EM0006-01VH.1H | 18 | EVQLVQSGAEVKKPGASVKVSCKASGFSFTNYYVHWMRQA PGQGLEWMGWISPGSDNTKYNEKFKGRVTMTRDTSISTAY MELSRLRSDDTAVYYCARDDYGNYYFDYWGQGTTVTVSS |
| EM0006-01VH.1I | 19 | EVQLVQSGAEVKKPGASVKVSCKASGFSFTNYYVHWMKQA PGQGLEWIGWISPGSDNTKYNEKFKGKATLTADTSITTAY MELSRLRSDDTAVYYCARDDYGNYYFDYWGQGTTVTVSS |
| EM0006-01VK.1 | 20 | DIVMTQSPDSLAVSLGERATINCKSSQSLLNSRTRKNYLA WYQQKPGQPPKLLIYWASTRESGVPDRFSGSGSGTDFTLT ISSLQAEDVAVYYCKQSYILRTFGGGTKVEIK |
| EM0006-01VK.1A | 21 | DIVMSQSPDSLAVSLGERATITCKSSQSLLNSRTRKNYLA WYQQKPGQPPKLLIYWASTRESGVPDRFSGSGSGTDFTLT ISSLQAEDVAVYYCKQSYILRTFGGGTKVEIK |
| EM0006-01VK.1(NS-QS) | 22 | DIVMTQSPDSLAVSLGERATINCKSSQSLLQSRTRKNYLA WYQQKPGQPPKLLIYWASTRESGVPDRFSGSGSGTDFTLT ISSLQAEDVAVYYCKQSYILRTFGGGTKVEIK |
| EM0006-01VK.1A(NS-QS) | 23 | DIVMSQSPDSLAVSLGERATITCKSSQSLLQSRTRKNYLA WYQQKPGQPPKLLIYWASTRESGVPDRFSGSGSGTDFTLT ISSLQAEDVAVYYCKQSYILRTFGGGTKVEIK |
| EM0006-01VK.1(NS-NT) | 24 | DIVMTQSPDSLAVSLGERATINCKSSQSLLNTRTRKNYLA WYQQKPGQPPKLLIYWASTRESGVPDRFSGSGSGTDFTLT ISSLQAEDVAVYYCKQSYILRTFGGGTKVEIK |
| EM0006-01VK.1(NS-NA) | 25 | DIVMTQSPDSLAVSLGERATINCKSSQSLLNARTRKNYLA WYQQKPGQPPKLLIYWASTRESGVPDRFSGSGSGTDFTLT ISSLQAEDVAVYYCKQSYILRTFGGGTKVEIK |
| Note: EM0006-01VH.1 or EM0006-01VK.1 means CDR-grafted VH/VL without back mutations. | | |

Humanized VH and VK genes back-translated from corresponding amino acid sequence designs were synthesized *de novo* and then cloned into vectors containing the human IgG1 and human kappa constant domains (SEQ ID NO:26 and SEQ ID NO:27, respectively).

Table 3: Human Constant Region Sequences Used in Antibody Humanization

| Constant Region | SEQ ID NO. | Amino Acid Sequences 12345678901234567890123456789012345678901234567890 |
|---|---|---|
| CH1-hinge-CH2-CH3 human constant IgG1 mutant | 26 | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK |
| CLκ human constant kappa | 27 | RTVAAPSVFIFPPSDEQLKSGTASVVCLLNNFYPREAKVQ WKVDNALQSGNSQESVTEQDSKDSTYSLSSTLTLSKADYE KHKVYACEVTHQGLSSPVTKSFNRGEC |

The pairing of the human VH and the human VK created 27 humanized antibodies, designated HuEM0006-01-1 to HuEM0006-01-27 (Table 3). Chimeric antibody (HuEM0006-01c) with parental mouse VH/VL and human constant sequences was also produced and used as a positive control for humanized antibody ranking. All recombinant humanized mAbs were expressed in HEK293 cells transiently and purified by Protein A chromatography.

Table 4: Production List of Humanized Anti-CD3 Antibodies Derived from mAbCD3-001

| Antibody Identifier | VH Region in Heavy Chain | VL Region in Light κ Chain |
|---|---|---|
| HuEM0006-01-1 | EM0006-01VH.1A | EM0006-01VK.1 |
| HuEM0006-01-2 | EM0006-01VH.1B | EM0006-01VK.1 |
| HuEM0006-01-3 | EM0006-01VH.1C | EM0006-01VK.1 |
| HuEM0006-01-4 | EM0006-01VH.1D | EM0006-01VK.1 |
| HuEM0006-01-5 | EM0006-01VH.1E | EM0006-01VK.1 |
| HuEM0006-01-6 | EM0006-01VH.1F | EM0006-01VK.1 |
| HuEM0006-01-7 | EM0006-01VH.1G | EM0006-01VK.1 |
| HuEM0006-01-8 | EM0006-01VH.1H | EM0006-01VK.1 |
| HuEM0006-01-9 | EM0006-01VH.1I | EM0006-01VK.1 |
| HuEM0006-01-10 | EM0006-01VH.1A | EM0006-01VK.1A |
| HuEM0006-01-11 | EM0006-01VH.1B | EM0006-01VK.1A |
| HuEM0006-01-12 | EM0006-01VH.1C | EM0006-01VK.1A |
| HuEM0006-01-13 | EM0006-01VH.1D | EM0006-01VK.1A |
| HuEM0006-01-14 | EM0006-01VH.1E | EM0006-01VK.1A |
| HuEM0006-01-15 | EM0006-01VH.1F | EM0006-01VK.1A |
| HuEM0006-01-16 | EM0006-01VH.1G | EM0006-01VK.1A |
| HuEM0006-01-17 | EM0006-01VH.1H | EM0006-01VK.1A |
| HuEM0006-01-18 | EM0006-01VH.1I | EM0006-01VK.1A |
| HuEM0006-01-19 | EM0006-01VH* | EM0006-01VK.1 (NS-QS) |

(continued)

| Antibody Identifier | VH Region in Heavy Chain | VL Region in Light κ Chain |
|---|---|---|
| HuEM0006-01-20 | EM0006-01VH | EM0006-01VK.1A (NS-QS) |
| HuEM0006-01-21 | EM0006-01VH | EM0006-01VK.1 (NS-NT) |
| HuEM0006-01-22 | EM0006-01VH | EM0006-01VK.1 (NS-NA) |
| HuEM0006-01-23 | EM0006-01VH.1A | EM0006-01VK.1 (NS-NA) |
| HuEM0006-01-24 | EM0006-01VH.1H | EM0006-01VK.1 (NS-NA) |
| HuEM0006-01-25 | EM0006-01VH.1E | EM0006-01VK.1 (NS-NA) |
| HuEM0006-01-26 | EM0006-01VH.1B | EM0006-01VK.1 (NS-NA) |
| HuEM0006-01-27 | EM0006-01VH.1G | EM0006-01VK.1 (NS-NA) |
| HuEM0006-01c | EM0006-01VH* | EM0006-01VK |
| * EM0006-01VH includes the murine variable heavy region of mAbCD3-001; EM0006-01VK includes the murine variable light region of mAbCD3-001. | | |

Example 2.2: Humanized anti-CD3 antibodies showed different CD3 binding activity

Different humanized VH and VK combinations led to humanized anti-CD3 variants with different CD3 binding affinities. The binding activity of the humanized variants of mAbCD3-001 were tested via flow cytometry with the human CD3-expressing Jurkat T cell line. $5 \times 10^5$ Jurkat cells in FACS buffer were seeded into each well of a 96-well plate. Cells were centrifuged at 400g for 5 minutes and supernatants were discarded. For each well, 100 μl of serially diluted antibodies were then added and mixed with the cells. After 40 minutes incubation at 4° C, plates were washed several times to remove excess antibodies. Secondary fluorochrome-conjugated antibody (Alexa Fluor® 647 goat anti-human IgG1 H&L; Jackson ImmunoResearch, Cat. #109-606-170) was then added and incubated with cells at room temperature for 20 minutes. After another round of centrifugation and a washing step, cells were resuspended in FACS buffer for reading on a CytoFLEX Flow Cytometer (Beckman Coulter). Median Fluorescence Intensity (MFI) readouts were plotted against antibody concentration and analyzed with GraphPad Prism 5.0 software.

As shown in Figure 5A-5H, humanized anti-CD3 antibodies showed a wide range of affinities to the cell surface CD3 target on Jurkat cells. VH variants evidently were the key elements for this CD3 binding modulation, as varying the kappa chain (i.e., between EM0006-01VK.1 and EM0006-01VK. 1A) did not appear to have a significant impact on binding. Some humanized antibodies, notably HuEM0006-01-08 and HuEM0006-01-17 having the VH variant EM006-01VH. 1H (see Table 4 and SEQ ID NO: 18), even showed much higher CD3 binding than chimeric control antibody HuEM0006-01c, having the parental mouse VH region EM0006-01VH (see Table 4).

Example 3: Generation of Anti-BCMA Monoclonal Antibodies

Anti-BCMA antibodies were obtained by immunizing Balb/c or SJL mice with a recombinant BCMA extracellular domain/Fc dimer formed by homodimerization of human BCMA(ECD) fused to a human Fc region:

MLQMAGQCSQNEYFDSLLHACIPCQLRCSSNTPPLTCQRYCNASVTNSVKGTNAIEGRMD

PKSCDKTHTCPPCPAPELLGGPSVFLFPPKPKDTLMISRTPEVTCVVVDVSHEDPEVKFN

WYVDGVEVHNAKTKPREEQYNSTYRVVSVLTVLHQDWLNGKEYKCKVSNKALPAPIEKTI

SKAKGQPREPQVYTLPPSREEMTKNQVSLTCLVKGFYPSDIAVEWESNGQPENNYKTTPP

VLDSDGSFFLYSKLTVDKSRWQQGNVFSCSVMHEALHNHYTQKSLSLSPGK (SEQ ID NO:28).

Mice were immunized at 2-week intervals and monitored for serum titer once a week after the second injection.

Example 3.1: Generation of Hybridomas

After 4 to 6 immunizations splenocytes were harvested and fused with mouse myeloma cells to form hybridoma cell lines. Fusion products were plated in selection media containing hypoxanthine-aminopterin-thymidine (HAT) in 96-well plates at a density of $1 \times 10^5$ spleen cells per well. Seven to ten days post-fusion, macroscopic hybridoma colonies were observed. Supernatants of hybridoma cells were then screened and selected to identify cell lines producing BCMA-specific mouse antibodies. Five anti-BCMA antibodies were selected and sequenced.

Example 3.2: Heavy and light chain variable region sequences

To amplify heavy and light chain variable region, total RNA of each hybridoma clone was isolated from more than $5 \times 10^6$ cells with TRIzol™ RNA extraction reagent (Invitrogen, Cat. #15596018). cDNA was synthesized using an Invitrogen™ SuperScript™ III First-Strand Synthesis SuperMix kit (ThermoFisher Scientific Cat. #18080) following manufacturer's instructions, and the cDNAs encoding the variable regions for light and heavy mouse immunoglobulin chains were amplified using a MilliporeSigma™ Novagen™ Mouse Ig-Primer Set (Fisher Scientific Cat. #698313). PCR products were analyzed by electrophoresis on a 1.2% agarose gel with SYBR™ Safe DNA gel stain (ThermoFisher Cat. #S33102). DNA fragments with correct size were purified using a NucleoSpin® Gel and PCR Clean-up kit (Macherey-Nagel, Cat. #740609) according to manufacturer's instructions and were subcloned into pMD18-T vector individually. Fifteen colonies from each transformation were selected and sequences of insert fragments were analyzed by DNA sequencing. Sequences were confirmed if at least 8 colony fragments matched consensus sequences for VH and VL. The protein sequences of murine mAb variable regions were analyzed by sequence homology alignment.

Example 3.3: Binding kinetics of anti-BCMA antibodies by Surface Plasmon Resonance (SPR)

Binding affinities and kinetic constants of anti-BCMA antibodies were determined by surface plasmon resonance at 25° C using a Biacore T200 instrument (GE Healthcare) using standard procedures. Briefly, goat anti-mouse IgG Fc antibody was directly immobilized across a biosensor chip, and antibody samples were injected over reaction matrices at a flow rate of 5 µl/min. Mouse anti-BCMA IgG test antibody was injected over the immobilized surface and captured by the immobilized anti-Fc antibodies. Human and cynomolgus BCMA(ECD)/Fc target polypeptides were then injected over the captured mouse anti-BCMA IgG surface. The association and dissociation rate constants, $k_{on}$ ($M^{-1}s^{-1}$) and $k_{off}$ ($s^{-1}$), respectively, were determined with a continuous flow rate of 30 µl/min. Rate constants were derived by making kinetic binding measurements at five different concentrations of target BCMA(ECD) polypeptide. The equilibrium dissociation constant $K_D$ (M) of the reaction between antibodies and related target proteins was then calculated from the kinetic rate constants using the formula $K_D = k_{off}/k_{on}$. Kinetic constants were determined by processing and fitting the data to a 1:1 binding model using Biacore analysis software. Results are shown in Table 7.

Table 7: Affinity Measurements for Anti-BCMA Monoclonal Antibodies

| Antigen Capture Target | Antibody | $k_{on}$ (1/Ms) | $k_{off}$ (1/s) | $K_D$ (M) |
|---|---|---|---|---|
| Human BCMA(ECD) | TAb1 | $6.03 \times 10^4$ | $1.98 \times 10^{-5}$ | $3.28 \times 10^{-10}$ |
| | TAb2 | $3.03 \times 10^5$ | 0.00209 | $6.89 \times 10^{-9}$ |
| | mAbBCMA-001 | $2.23 \times 10^5$ | $2.00 \times 10^{-4}$ | $8.96 \times 10^{-10}$ |
| | mAbBCMA-002 | $2.12 \times 10^5$ | 0.00292 | $1.38 \times 10^{-8}$ |
| | mAbBCMA-003 | $4.54 \times 10^4$ | $1.85 \times 10^{-5}$ | $4.08 \times 10^{-10}$ |
| | mAbBCMA-004 | $6.63 \times 10^5$ | 0.00842 | $1.27 \times 10^{-8}$ |
| | mAbBCMA-005 | $8.36 \times 10^4$ | $7.46 \times 10^{-5}$ | $8.92 \times 10^{-10}$ |

(continued)

| Antigen Capture Target | Antibody | $k_{on}$ (1/Ms) | $k_{off}$ (1/s) | $K_D$ (M) |
|---|---|---|---|---|
| Cyno BCMA(ECD) | TAb1 | $8.64 \times 10^4$ | $1.77 \times 10^{-5}$ | $2.05 \times 10^{-10}$ |
| | TAb2 | $1.03 \times 10^5$ | 0.00252 | $2.44 \times 10^{-8}$ |
| | mAbBCMA-001 | N/A | N/A | N/A |
| | mAbBCMA-002 | $4.03 \times 10^4$ | $3.04 \times 10^{-4}$ | $7.55 \times 10^{-9}$ |
| | mAbBCMA-003 | $6.06 \times 10^4$ | $2.36 \times 10^{-4}$ | $3.90 \times 10^{-9}$ |
| | mAbBCMA-004 | N/A | N/A | N/A |
| | mAbBCMA-005 | N/A | N/A | N/A |

N/A = no measurable binding signal detected
TAb1 is a reference anti-BCMA antibody, which is clone CA8 from Int'l Publication No. WO 2012/163805
TAb2 is a reference anti-BCMA antibody, which is clone 83A10 from Int'l Publication No. WO 2014/122143

The two anti-BCMA antibodies showing high affinity for both human and cynomolgus BCMA targets were further developed and analyzed. The variable domain sequences for these selected anti-BCMA monoclonals, mAbBCMA-002 and mAbBCMA-003, are set out in Table 8, below. Complementarity determining regions (CDRs) are underlined based on Kabat numbering.

Table 8: Variable Region Sequences of anti-BCMA Antibodies

| Antibody | domain | SEQ ID NO. | amino acid sequence 12345678901234567890123456789012345678901234567890 |
|---|---|---|---|
| mAbBCMA-002 | VH | 29 | QIQLVQSGPELKKPGETVKISCKASGYSFTNYGLNWVKQA PGKGLKWMGWINTYSGHPTYVDDFKGRFAFSLETSASTAY LQINKLKNEDTSTYFCVREKDDGYRLGLDYWGQGTSVTVS S |
| | VL | 30 | QIVLTQSPAIMSASPGEKVTMTCSASSSVSYMYWYQQRPG SSPRLWIYDTSNLVSGVPARFSGSRSGTSYSLTISGMEAE DAATYYCLQYSGYPYTFGGGTKLEIK |
| mAbBCMA-003 | VH | 31 | EVQLQQSGTVLARPGASVRMSCKASGYIFPNFWMHWVKQR PGQGLDWIGAFYPGNDDTYYNQKFKGKAKLTAVTSASTAY MELSSLTSEDSAVYYCARSGYYGSSDAMDYWGQGTSVTVS S |
| | VL | 32 | DIQMTQSPASLSASVGETVTVTCGASENIYGALNWYQRKQ GKSPQLLIYGATNLADGISSRFSGSGSGRQFSLKISSLLP DDVATYFCQSVLTTPWTFGGGTKLEIK |

Example 3.4: Anti-BCMA antibodies showed different BCMA blocking activities

The ability of monoclonal anti-BMCA antibodies to block NFκB phosphorylation stimulated by the BCMA ligand BAFF in the human myeloma cell line NCI-H929 was assessed by using a Phospho-NFκB (Ser536) cellular assay kit (Cisbio; Cat. #64 NFBPEG). NCI-H929 human myeloma cells were starved in assay medium (RPMI1640, 0.1% BSA) at 37° C overnight. Cells were washed, resuspended, and seeded into 384-well microplates (PerkinElmer, Cat. #6008280) at $2 \times 10^5$ cells per well. Antibodies were then added into the wells and incubated with cells for about 10 minutes at 37° C. Anti-BAFF antibody (R&D Systems, Cat. #BAF124) was used as a positive control, and an irrelevant anti-RAC1 monoclonal antibody was used as a negative control. Reference anti-BCMA antibodies TAb1 and TAb2 (clone CA8 from Int'l Publication No. WO 2012/163805 and clone 83A10 from Int'l Publication No. WO 2014/122143, respectively) were tested for comparison.

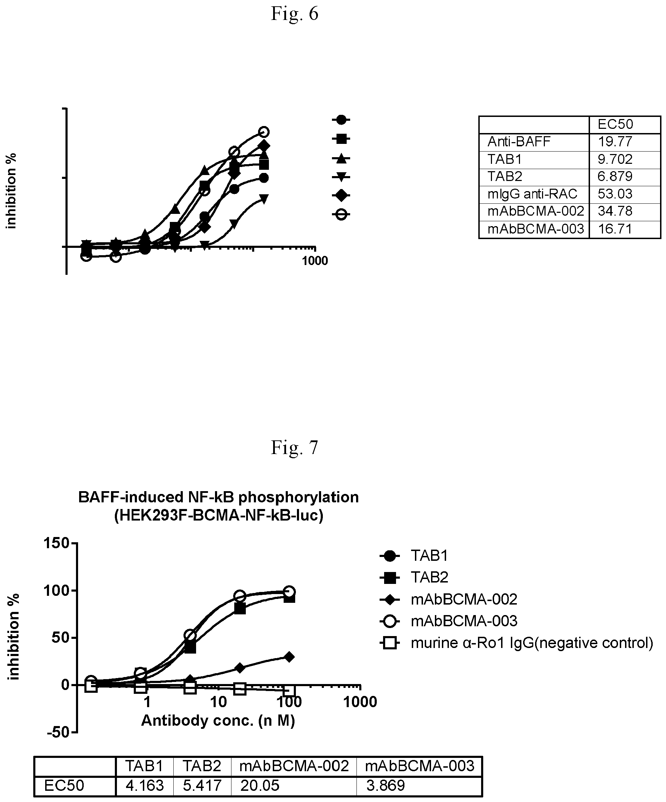

Recombinant BAFF was then added to each well at a concentration of 5 μg/ml and incubated for 30 minutes. Cells were lysed by adding kit lysis buffer and incubated for at least 30 minutes at room temperature with shaking. Cell lysates were then transferred to a 384-well small volume microplate (PerkinElmer, Cat. #6008280). Assay kit reagents were prepared and added into wells following the manufacturer's instructions. After a final incubation at room temperature for 4 hours, plates were read for fluorescence emission at wavelength of 665 nm and 620 nm. Inhibition percentage was calculated and plotted against antibody concentration with GraphPad Prism 5.0 software. As shown in Figure 6, the selected anti-BCMA antibodies isolated as described above, mAbBCMA-002 and mAbBCMA-003 showed superior inhibition activity to BAFF-induced NF-κB phosphorylation than the negative control (anti-RAC).

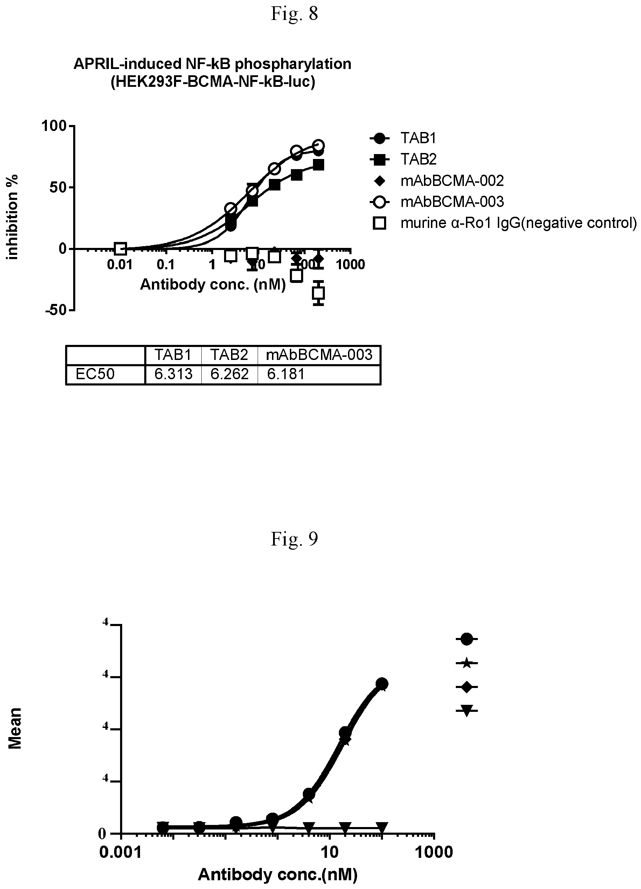

Another reporter gene-based luminescence assay system was also used to characterize antibody blocking of ligand binding activity to BCMA. A stable HEK293F cell line transfected to express BCMA and emitting a luciferase signal when NF-κB phosphorylation is induced (HEK293F-BCMA-NF-kB-luc clone 1H2) was established in-house and used for this luminescence assay. Cells were harvested, washed and re-suspended in assay medium (RPMI1640 with 10% FBS). Cells were then seeded into 96-well microplates (Costar, Cat. #3903) at $5 \times 10^4$ cells per well and incubated with test antibodies: mAbBCMA-002, mAbBCMA-003, TAb1 (anti-BCMA), TAb2 (anti-BCMA), or irrelevant murine IgG. A BCMA ligand, either BAFF or APRIL (TNFSF13, CD286), was added and co-incubated with antibody solutions for 10 minutes. One-Glo™ Luciferase Assay System (Promega, Cat. #E6130) reagents were prepared and added to wells following the manufacturer's instructions. Plates were read for luminescence signals with Varioskan™ LUX microplate reader (Thermo Scientific). Inhibition percentage was calculated and plotted against antibody concentration with Graph-Pad Prism 5.0 software. As shown in Figure 7 (BAFF blocking) and Figure 8 (APRIL blocking), mAbBCMA-002 showed no activity or weak blocking activity while mAbBCMA-003 showed strong NF-κB signal pathway blocking activity similar to the positive reference antibodies TAb1 and TAb2.

The binding domains of mAbBCMA-002 and mAbBCMA-003 were next used to generate bispecific BCMA/CD3 FIT-Ig binding proteins.

Example 4: Production of BCMA/CD3 Fabs-in-Tandem Immunoglobulins (FIT-Igs)

Example 4.1: Generation of BCMA/CD3 FIT-Ig binding proteins (FIT-Igs)

Bispecific Fabs-in-Tandem Immunoglobulin (FIT-Ig) binding proteins recognizing both human CD3 and human BCMA were constructed. The FIT-Ig constructs were engineered to omit the use of synthetic linker sequences between immunoglobulin domains, following the general procedures described in international publication WO 2015/103072.

The DNA constructs used to generate FIT-Ig antibodies capable of binding CD3 and BCMA encoded the variable domains of parental anti-CD3 and anti-BCMA monoclonal antibodies (mAbs). Each FIT-Ig binding protein consisted of three polypeptide chains having the following structures:

Chain 1 (long chain): $VL_{CD3}$-CL-$VH_{BCMA}$-CH1-hinge-CH2-CH3;
Chain 2 (first short chain): $VH_{CD3}$-CH1;
Chain 3 (second short chain): $VL_{BCMA}$-CL;

wherein $VL_{BCMA}$ is the light chain variable domain of a monoclonal antibody recognizing BCMA, $VH_{CD3}$ is the heavy chain variable domain of a monoclonal antibody recognizing CD3, $VL_{CD3}$ is the light chain variable domain of a monoclonal antibody recognizing CD3, $VH_{BCMA}$ is the heavy chain variable domain of a monoclonal antibody recognizing BCMA, each CL is a light chain constant domain, each CH1 is a first heavy chain constant domain, and hinge-CH2-CH3 is an antibody C-terminal Fc region.

To construct the long chain vector, cDNA encoding the $VL_{CD3}$-CL-$VH_{BCMA}$ segment was synthesized *de novo* and inserted into the multiple cloning site (MCS) of a vector including coding sequences for human CH1-hinge-CH2-CH3. In the resulting vector, the MCS sequence was eliminated during homologous recombination to ensure that all the domain fragments were in the correct reading frame. Similarly, to construct the first and second short chains, $VH_{CD3}$ and $VL_{BCMA}$ structural genes were *de novo* synthesized and inserted into the MCS of the appropriate vectors including coding segments for human CH1 and CL domains, respectively.

Three plasmids were mixed at a ratio of 1:2:1.5, then co-transfected into HEK293 cells. After 7 days expression, cell culture supernatants were collected and purified by Protein A chromatography. The concentration of purified FIT-Ig protein was measured by A280, and homogeneity was analyzed by size exclusion chromatography (SEC).

To test the binding characteristics of the new murine anti-BCMA antibodies, mAbBCMA-002 and mAbBCMA-003, in a bispecific FIT-Ig format, the $VH_{BCMA}$ and $VL_{BCMA}$ domains used in the Chain 1 and Chain 3 polypeptides (*supra*) were the VH and VL domains set forth in Table 8 (i.e., either SEQ ID NO:29 or SEQ ID NO:31 for VH, and either SEQ ID NO:30 or SEQ ID NO:32 for VL). For the $VH_{CD3}$ and $VL_{CD3}$ domains in Chain 1 and Chain 2 (*supra*), the parental anti-CD3 antibody was one of three selected humanized anti-CD3 antibodies: HuEM0006-01-24 antibody (VH = SEQ ID NO:18; VL = SEQ ID NO:25), HuEM0006-01-25 antibody (VH = SEQ ID NO:15; VL = SEQ ID NO:25), or HuEM0006-01-26 antibody (VH = SEQ ID NO:12; VL = SEQ ID NO:25). For the constant domains CH1-hinge-CH2-CH3 and CL in the FIT-Ig structure, human sequences were used, i.e., SEQ ID NO:26 and SEQ ID NO:27. Utilizing cDNAs encoding the foregoing polypeptide domains, FIT-Ig expression vectors were constructed and used to transfect HEK293 cells. Cultures of each no-linker FIT-Ig contract were grown and FIT-Igs purified as described above. The six FIT-Ig binding proteins were given the designations shown in Table 9 below:

Table 9: Production list for huCD3/chimeric BCMA FIT-Ig binding proteins

| FIT-Ig Antibody ID | variable domains | outer Fab binding site (CD3) | inner Fab binding site (BCMA) |
|---|---|---|---|
| FIT1006-3a | VH | SEQ ID NO:18 | SEQ ID NO:29 |
| | VL | SEQ ID NO:25 | SEQ ID NO:30 |
| FIT1006-5a | VH | SEQ ID NO:15 | SEQ ID NO:29 |
| | VL | SEQ ID NO:25 | SEQ ID NO:30 |
| FIT1006-7a | VH | SEQ ID NO:12 | SEQ ID NO:29 |
| | VL | SEQ ID NO:25 | SEQ ID NO:30 |
| FIT1006-4a | VH | SEQ ID NO:18 | SEQ ID NO:31 |
| | VL | SEQ ID NO:25 | SEQ ID NO:32 |
| FIT1006-6a | VH | SEQ ID NO:15 | SEQ ID NO:31 |
| | VL | SEQ ID NO:25 | SEQ ID NO:32 |
| FIT1006-8a | VH | SEQ ID NO:12 | SEQ ID NO:31 |
| | VL | SEQ ID NO:25 | SEQ ID NO:32 |

Example 4.2: Generation of BCMA/CD3 FIT-Ig Fab fragment binding proteins (FIT-Fabs)

Full-length FIT-Ig protein was digested and purified with a Pierce™ Fab Preparation Kit (ThermoFisher Scientific, Cat. #44985). In this process, the Fc domain of the FIT-Ig protein was removed by enzymatic cleavage using immobilized papain on agarose beads. The FIT-Ig Fab fragment (FIT-Fab) was then purified from the flow-through from Protein A chromatography. The concentration of purified FIT-Fab protein was measured by A280, and homogeneity was analyzed by size exclusion chromatography (SEC).

Example 4.3: FIT-Ig bispecific antibodies showed binding to both CD3 and BCMA targets

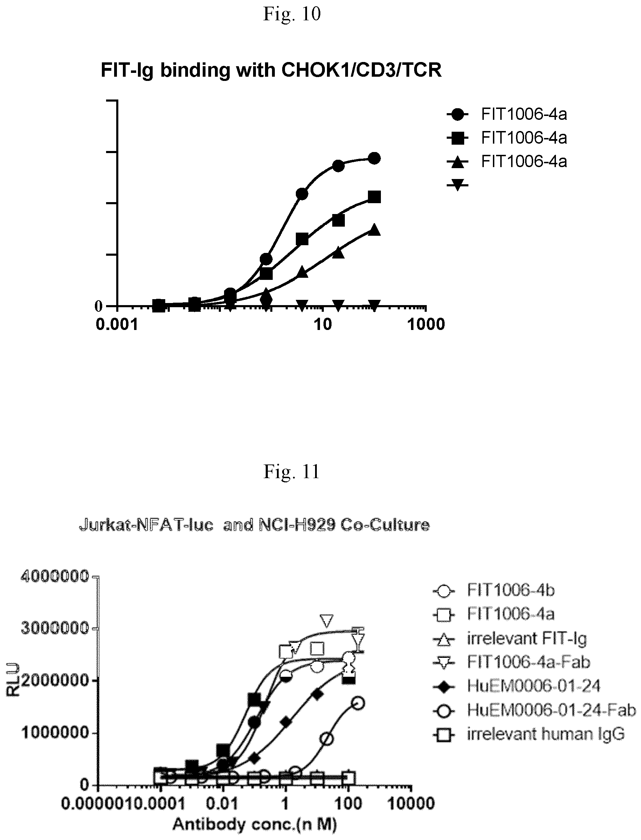

Binding activity of the chimeric bispecific BCMA/CD3 FIT-Ig antibodies was tested via flow cytometry with a human CD3/TCR complex transfected CHO cell line (CHOK1/CD3/TCR cells) and BCMA-expressing NCI-H929 cells. Briefly, $5 \times 10^5$ cells in FACS buffer were seeded into 96 well plates. Cells were centrifuged at $400 \times$g for 5 minutes and supernatants were discarded. For each well, 100 $\mu$l serially diluted FIT-Ig or FIT-Fab antibodies were then added and mixed with the cells. Following incubation for 40 minutes at 4° C, plates were washed several times to remove excess antibodies. Secondary antibody (goat anti-huIgG kappa chain specific) was then added and incubated with cells at room temperature for 20 minutes. After another round of centrifugation and washing, cells were resuspended with FACS buffer for reading on a CytoFLEX Flow Cytometer. Results were analyzed and plotted with GraphPad Prism 5.0 software. The results are shown in Figures 9 and 10.

As shown in Fig. 9, binding activity to BCMA with Fab fragments of bispecific chimeric BCMA/CD3 FIT-Fab antibodies showed exactly the same binding curve when they consist of the same BCMA binding domains. As shown in Fig. 10, chimeric BCMA/CD3 FIT-Ig binding proteins maintained similar binding activity curves to the parental monoclonal humanized CD3 antibodies (*Cf.* Figure 5B, 5C).

Example 4.4: Chimeric FIT-Fab and FIT-Ig showed redirected CD3 activation

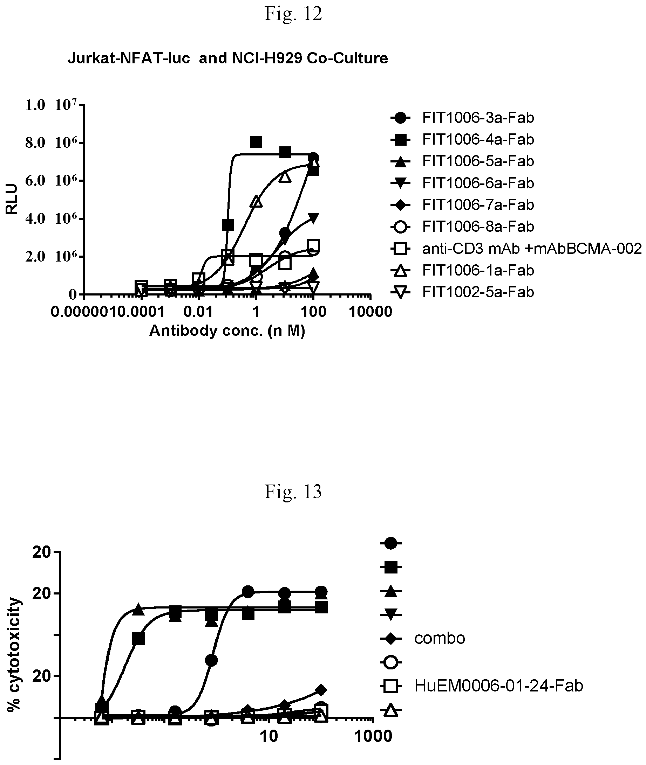

To measure redirected CD3 activation by BCMA/CD3 bispecific FIT-Ig and FIT-Fab antibodies, a co-cultured reporter gene assay was used. Jurkat-NFAT-luc cells will trigger downstream luciferase signal once cell surface CD3 is activated. NCI-H929 cells were used as the BCMA-expressing target cell, which can crosslink CD3/TCR complex on T cells via bispecific BCMA/CD3 antibodies upon BCMA binding. Jurkat-NFAT-luc and NCI-H929 cells were washed and resuspended in assay medium (RPMI1640 with 10% FBS) separately. Both cell types were seeded into 96 well plates (Costar #3903) at $1\times10^5$ cells per well in a ratio of 1:1. FIT-Ig or FIT-Fab antibodies were added and mixed with the cells and incubated for 4 hours at 37° C. At the end of incubation, ONE-Glo™ luminescence assay kit (Promega, Cat. #E6130) reagents were prepared and added into wells according the manufacturer's instructions. Plates were read for luminescence signals with Varioskan™ LUX microplate reader (ThermoFisher Scientific). The results are shown in Figures 11 and 12.

Referring to Figure 11, FIT-Ig concentration causing T cell activation is plotted for the FIT-Ig binding proteins prepared as described in Example 4.1, utilizing two of the highest affinity anti-BCMA and anti-CD3 antibodies, namely, mAbBCMA-003 and HuEM0006-01-24. In the figure, FIT1006-4a is a FIT-Ig having an outer CD3-binding Fab binding site and an inner BCMA-binding Fab binding site (*supra*; see Table 9); FIT1006-4b is a FIT-Ig constructed using the same amino acid sequences but having the position of the binding domains reversed, that is, having an outer BCMA-binding Fab binding site and an inner CD3-binding Fab binding site; and FIT1006-4a-Fab which was made from FIT1006-4a by papain digestion (see Example 4.2).

The performance of these binding proteins is compared against two negative controls: (i) a FIT-Ig having binding sites reactive with two irrelevant antigen targets ("FIT1002-5a"), and (ii) a humanized IgG monoclonal antibody reactive with an irrelevant antigen ("hIgG"). Also, two anti-CD3 binding proteins, namely, a humanized anti-CD3 monoclonal antibody (HuEM0006-01-24) and a Fab fragment made therefrom (HuEM0006-01-24-Fab), were also tested. It can be seen that all of the bispecific BCMA/CD3 binding proteins led to increased T cell activation in the presence of BCMA-expressing target cells in comparison to monospecific anti-CD3 binding proteins having no BCMA binding activity.

Referring to Figure 12, the concentration of various bispecific BCMA/CD3 FIT-Fab binding proteins causing T cell activation in the presence of BCMA-expressing target cells is plotted for FIT-Fabs prepared from FIT-Ig binding proteins described in Table 9, above (designated FIT1006-3a-Fab, FIT1006-4a-Fab, FIT1006-5a-Fab, FIT1006-6a-Fab, FIT1006-7a-Fab, FIT1006-8a-Fab). The performance of these FIT-Fabs was compared against a combination of a reference anti-CD3 Fab and mAbBCMA-002, a reference FIT-Fab antibody designated FIT1006-1a-Fab utilizing anti-CD3 and anti-BCMA binding regions disclosed in WO 2016/020332, and a negative control FIT-Fab designated FIT1002-5a-Fab prepared using parental antibody binding sites directed at two irrelevant antigen targets.

These results demonstrated that the BCMA/CD3 bispecific antibodies can activate CD3 by crosslinking upon binding to BCMA on the surface of tumor cells. Not only FIT-Ig binding proteins (Fig. 11) but also FIT-Fab binding proteins (Fig. 12) showed redirected activation in this assay. Additionally, FIT1006-4a-Fab showed a surprisingly steep activation curve at low concentration.

Example 4.5: Chimeric BCMA/CD3 FIT-Fabs showed redirected T cell cytotoxicity

The tumor cell killing potency of the BCMA/CD3 bispecific binding proteins was measured in a redirected T cell cytotoxicity assay using the human myeloma cell line NCI-H929 as target cells and human T cells as effector cells. Briefly, cells were harvested, washed, and resuspended with assay medium (RPMI1640 with 10% FBS). NCI-H929 cells were seeded into flat-bottom 96 well plates (Corning, Cat. #3599) at $5\times10^4$ cells per well. T cells were purified from human PBMC with a commercial PBMC isolation kit (EasySep™, Stemcell Technologies, Cat. #17951) and were added to the wells at $2\times10^5$ cells per well. Test antibodies were added and incubated with the cells mixture for 48 hours at 37° C. Lactate dehydrogenase (LDH) release was measured with a CytoTox 96® cytotoxicity assay kit (Promega, Cat. #G1780). OD490 readouts were obtained following the manufacturer's instructions. Target cells NCI-H929 max lysis (100%) minus minimal lysis (0%) was presented as the normalization denominator. The percentage of LDH release was plotted against the concentrations of bispecific antibodies. As shown in Figure 13, bispecific FIT-Fabs having anti-BCMA and anti-CD3 specificity demonstrated redirected T cell cytotoxicity to NCI-H929 tumor cells, while monospecific humanized anti-CD3 Fab and a combination of anti-CD3 Fab (Fab fragment of HuEM0006-01-24) and anti-BCMAmAb (TAB1) showed no cytotoxic activity.

Example 4.5: Chimeric FIT-Ig showed limited non-target redirected CD3 activation in vitro

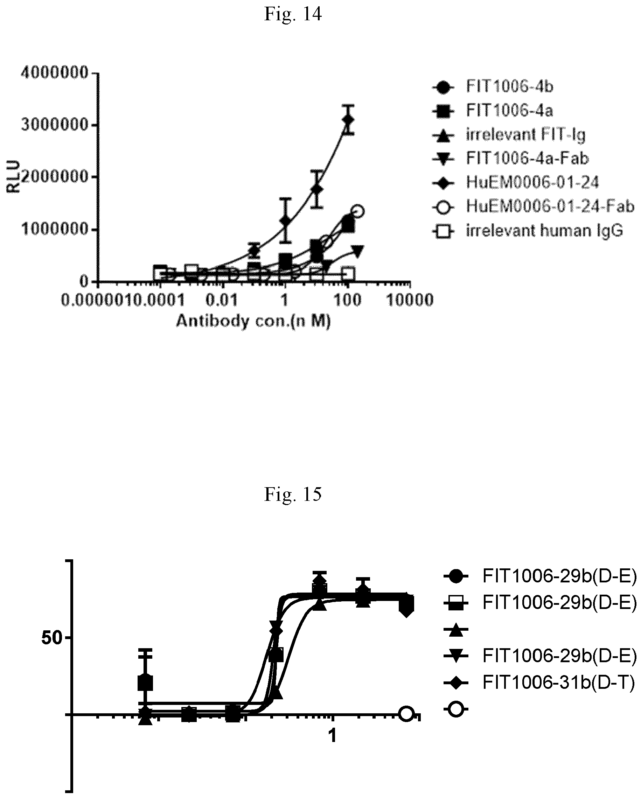

Non-target redirected CD3 activation was tested using a Jurkat-NFAT-luc based reporter gene assay in the absence of target cells. Jurkat-NFAT-luc cells were harvested, washed and resuspended in assay medium (RPMI1640 with 10% FBS) and seeded into 96 well plates (Costar #3903) at $1\times10^5$ cells per well. Test antibodies were added and mixed with the cells and incubated for 4 hours at 37° C. Following incubation, ONE-Glo™ luminescence assay kit (Promega, Cat. #E6130) reagents were prepared and added into wells following the manufacturer's instructions. Plates were read for luminescence signals with Varioskan™ Lux plate reader. The results are shown in Figure 14.

This assay was similar to the test conducted in Example 4.4, above, except in the absence of cells expressing a co-target for the bispecific binding proteins, in this case BCMA. The results show that bispecific BCMA/CD3 FIT-Ig antibodies (FIT1006-4a and FIT1006-4b) and the BCMA/CD3 FIT-Fab designated FIT1006-4a-Fab, all having the same CD3 binding domain as the humanized anti-CD3 monoclonal antibody HuEM0006-01-24, showed much less non-target redirected activation than the anti-CD3 antibody alone, in the absence of BCMA-expressing target cells (*Cf.* Fig. 11).

Example 5: Production of humanized bispecific BCMA/CD3 FIT-Ig binding proteins

The anti-BCMA monoclonal mAbBCMA-003 showed higher BCMA binding affinity and better redirected cell killing when used in a BCMA/CD3 FIT-Ig and FIT-Fab format. Accordingly mAbBCMA-003 was selected for humanization and subsequent use in constructing humanized bispecific binding proteins.

Example 5.1: Humanization of Anti-BCMA Antibody mAbBCMA-003

The mAbBCMA-003 variable region genes were employed to create humanized mAbs. In the first step of this process, the amino acid sequences of the VH and VK of mAbBCMA-003 (see Table 8, *supra*) were compared against the available database of human Ig V-gene sequences in order to find the overall best-matching human germline Ig V-gene sequences. Additionally, the framework 4 of VH or VL was compared against the J-region database to find the human framework having the highest homology to the murine VH and VL regions, respectively. For the light chain, the closest human V-gene match was the VK1-39(02) gene, and for the heavy chain the closest human match was the VH1-03 gene. Humanized variable domain sequences were then designed where the CDR-L1, CDR-L2 and CDR-L3 of the mAbBCMA-003 light chain were grafted onto framework sequences of the VK1-39(02) gene, with JK2 framework 4 sequence after CDR-L3; and the CDR-H1, CDR-H2, and CDR-H3 sequences of the mAbBCMA-003 VH were grafted onto framework sequences of the VH1-03 gene, with JH6 framework 4 sequence after CDR-H3. A 3D Fv model of mAbBCMA-003 was then generated and analyzed to determine if there were any framework residues that have a less than 4Å distance to the CDR residues and that were most likely critical to support loop structures or the VH/VL interface. These residues in humanized sequences should be back-mutated to mouse residues at the same position to retain affinity/activity. Q1E mutation was always included to eliminate N-terminal pyroglutamate formation if applicable. In the case of the heavy chain, potential mutations of P30T, I48M, K66R, A67V, L69I, and A71R (Kabat numbering) were identified as desirable back mutations. In the case of the light chain, V58I and R69T were identified as desirable back mutations. Via the importance tier of each back mutation, as determined by its interaction with CDRs, the most important back mutations were introduced in the humanized VH sequence in order of priority, followed by other back mutations in successive designs. In addition, a DG dipeptide occurring at the C-terminal end of CDR-L2 presented a potential aspartic acid isomerization site, and this was eliminated in light chain variants by the following alternative substitutions: D56A, D56E, D56S, D56T, or G57A. The humanized VH and VL design constructs are shown in Table 10 (below). (Back mutated framework amino acid residues are indicated with double underscore; murine CDRs from the original parental antibody are underlined.)

Table 10: Humanized VH/VL design of anti-BCMA mAbBCMA-003

| Humanized VH/VL | SEQ ID NO. | Amino acid sequences<br>12345678901234567890123456789012345678790 |
|---|---|---|
| mAbBCMA-003 VH.1 | 33 | EVQLVQSGAEVKKPGASVKVSCKASGYTFTNFWMHWVRQA PGQRLEWMGAFYPGNDDTYYNQKFKGRVTITRDTSASTAY MELSSLRSEDMAVYYCARSGYYGSSDAMDYWGQGTTVTVS S |
| mAbBCMA-003 VH.1A | 34 | EVQLVQSGAEVKKPGASVKVSCKASGYTFTNFWMHWVRQA PGQRLEWMGAFYPGNDDTYYNQKFKGRVTITADTSASTAY MELSSLRSEDMAVYYCARSGYYGSSDAMDYWGQGTTVTVS S |

(continued)

| Humanized VH/VL | SEQ ID NO. | Amino acid sequences<br>123456789012345678901234567890 |
|---|---|---|
| mAbBCMA-003 VH.1B | 35 | EVQLVQSGAEVKKPGASVKVSCKASGYTFTNFWMHWVRQA<br>PGQRLEWIGAFYPGNDDTYYNQKFKGKATLTADTSASTAY<br>MELSSLRSEDMAVYYCARSGYYGSSDAMDYWGQGTTVTVS<br>S |
| mAbBCMA-003 VH.1C | 36 | EVQLVQSGAEVKKPGASVKVSCKASGYTFPNFWMHWVRQA<br>PGQRLEWIGAFYPGNDDTYYNQKFKGKATLTADTSASTAY<br>MELSSLRSEDMAVYYCARSGYYGSSDAMDYWGQGTTVTVS<br>S |
| mAbBCMA-003 VH.1D | 37 | EVQLVQSGAEVKKPGASVKVSCKASGYTFPNFWMHWVRQA<br>PGQRLEWMGAFYPGNDDTYYNQKFKGRVTITRDTSASTAY<br>MELSSLRSEDMAVYYCARSGYYGSSDAMDYWGQGTTVTVS<br>S |
| mAbBCMA-003 VK.1 | 38 | DIQMTQSPSSLSASVGDRVTITCGASENIYGALNWYQQKP<br>GKAPKLLIYGATNLADGVPSRFSGSGSGTDFTLTISSLQP<br>EDFATYYCQSVLTTPWTFGQGTKLEIK |
| mAbBCMA-003 VK.1A | 39 | DIQMTQSPSSLSASVGDRVTITCGASENIYGALNWYQQKP<br>GKAPKLLIYGATNLADGIPSRFSGSGSGRDFTLTISSLQP<br>EDFATYYCQSVLTTPWTFGQGTKLEIK |
| mAbBCMA-003 VK.1 (D-A) | 40 | DIQMTQSPSSLSASVGDRVTITCGASENIYGALNWYQQKP<br>GKAPKLLIYGATNLAAGVPSRFSGSGSGTDFTLTISSLQP<br>EDFATYYCQSVLTTPWTFGQGTKLEIK |
| mAbBCMA-003 VK.1 (D-E) | 41 | DIQMTQSPSSLSASVGDRVTITCGASENIYGALNWYQQKP<br>GKAPKLLIYGATNLAEGVPSRFSGSGSGTDFTLTISSLQP<br>EDFATYYCQSVLTTPWTFGQGTKLEIK |
| mAbBCMA-003 VK.1 (D-S) | 42 | DIQMTQSPSSLSASVGDRVTITCGASENIYGALNWYQQKP<br>GKAPKLLIYGATNLASGVPSRFSGSGSGTDFTLTISSLQP<br>EDFATYYCQSVLTTPWTFGQGTKLEIK |
| mAbBCMA-003 VK.1 (D-T) | 43 | DIQMTQSPSSLSASVGDRVTITCGASENIYGALNWYQQKP<br>GKAPKLLIYGATNLATGVPSRFSGSGSGTDFTLTISSLQP<br>EDFATYYCQSVLTTPWTFGQGTKLEIK |
| mAbBCMA-003 VK.1 (G-A) | 44 | DIQMTQSPSSLSASVGDRVTITCGASENIYGALNWYQQKP<br>GKAPKLLIYGATNLADAVPSRFSGSGSGTDFTLTISSLQP<br>EDFATYYCQSVLTTPWTFGQGTKLEIK |

(continued)

| Humanized VH/VL | SEQ ID NO. | Amino acid sequences<br>123456789012345678901234567890 |
|---|---|---|
| mAbBCMA-003 VK.1A (D-A) | 45 | DIQMTQSPSSLSASVGDRVTITCGASENIYGALNWYQQKP<br>GKAPKLLIYGATNLAAGIPSRFSGSGSGRDFTLTISSLQP<br>EDFATYYCQSVLTTPWTFGQGTKLEIK |
| mAbBCMA-003 VK.1A (D-E) | 46 | DIQMTQSPSSLSASVGDRVTITCGASENIYGALNWYQQKP<br>GKAPKLLIYGATNLAEGIPSRFSGSGSGRDFTLTISSLQP<br>EDFATYYCQSVLTTPWTFGQGTKLEIK |
| mAbBCMA-003 VK.1A (D-S) | 47 | DIQMTQSPSSLSASVGDRVTITCGASENIYGALNWYQQKP<br>GKAPKLLIYGATNLASGIPSRFSGSGSGRDFTLTISSLQP<br>EDFATYYCQSVLTTPWTFGQGTKLEIK |
| mAbBCMA-003 VK.1A (D-T) | 48 | DIQMTQSPSSLSASVGDRVTITCGASENIYGALNWYQQKP<br>GKAPKLLIYGATNLATGIPSRFSGSGSGRDFTLTISSLQP<br>EDFATYYCQSVLTTPWTFGQGTKLEIK |
| mAbBCMA-003 VK.1A (G-A) | 49 | DIQMTQSPSSLSASVGDRVTITCGASENIYGALNWYQQKP<br>GKAPKLLIYGATNLADAIPSRFSGSGSGRDFTLTISSLQP<br>EDFATYYCQSVLTTPWTFGQGTKLEIK |

The humanized anti-BCMA VH and VL genes were produced synthetically and then respectively cloned into FIT-Ig vectors as described in Example 4.1, which also contained the VH and VL genes from anti-CD3 monoclonal HuEM0006-01-24. The pairing of the humanized VH and the humanized VL created the humanized BCMA/CD3 FIT-Ig binding proteins listed in Table 11 below. A chimeric antibody with parental mouse VH/VL of mAbBCMA-003 and human constant sequences was also produced as a positive control for humanized binding protein ranking. All recombinant FIT-Igs were expressed and purified as described in Example 4.1.

Table 11: Production List of Humanized BCMA/CD3 FIT-Igs

| FIT-Ig Identifier | Outer Fab Binding Site | | Inner Fab Binding Site | |
|---|---|---|---|---|
| | VL$_{BCMA}$ | VH$_{BCMA}$ | VL$_{CD3}$ | VH$_{CD3}$ |
| FIT1006-27b | SEQ ID NO:38 | SEQ ID NO:34 | SEQ ID NO:25 | SEQ ID NO:18 |
| FIT1006-28b | SEQ ID NO:38 | SEQ ID NO:35 | SEQ ID NO:25 | SEQ ID NO:18 |
| FIT1006-29b | SEQ ID NO:38 | SEQ ID NO:36 | SEQ ID NO:25 | SEQ ID NO:18 |
| FIT1006-30b | SEQ ID NO:38 | SEQ ID NO:37 | SEQ ID NO:25 | SEQ ID NO:18 |
| FIT1006-31b | SEQ ID NO:39 | SEQ ID NO:34 | SEQ ID NO:25 | SEQ ID NO:18 |
| FIT1006-32b | SEQ ID NO:39 | SEQ ID NO:35 | SEQ ID NO:25 | SEQ ID NO:18 |
| FIT1006-33b | SEQ ID NO:39 | SEQ ID NO:36 | SEQ ID NO:25 | SEQ ID NO:18 |
| FIT1006-34b | SEQ ID NO:39 | SEQ ID NO:37 | SEQ ID NO:25 | SEQ ID NO:18 |
| FIT1006-29b(D-A) | SEQ ID NO:45 | SEQ ID NO:36 | SEQ ID NO:25 | SEQ ID NO:18 |
| FIT1006-29b(D-E) | SEQ ID NO:46 | SEQ ID NO:36 | SEQ ID NO:25 | SEQ ID NO:18 |
| FIT1006-29b(D-S) | SEQ ID NO:47 | SEQ ID NO:36 | SEQ ID NO:25 | SEQ ID NO:18 |
| FIT1006-29b(D-T) | SEQ ID NO:48 | SEQ ID NO:36 | SEQ ID NO:25 | SEQ ID NO:18 |

(continued)

| FIT-Ig Identifier | Outer Fab Binding Site | | Inner Fab Binding Site | |
| --- | --- | --- | --- | --- |
| | $VL_{BCMA}$ | $VH_{BCMA}$ | $VL_{CD3}$ | $VH_{CD3}$ |
| FIT1006-29b(G-A) | SEQ ID NO:49 | SEQ ID NO:36 | SEQ ID NO:25 | SEQ ID NO:18 |
| FIT1006-31b(D-A) | SEQ ID NO:45 | SEQ ID NO:34 | SEQ ID NO:25 | SEQ ID NO:18 |
| FIT1006-31b(D-E) | SEQ ID NO:46 | SEQ ID NO:34 | SEQ ID NO:25 | SEQ ID NO:18 |
| FIT1006-31b(D-S) | SEQ ID NO:47 | SEQ ID NO:34 | SEQ ID NO:25 | SEQ ID NO:18 |
| FIT1006-31b(D-T) | SEQ ID NO:48 | SEQ ID NO:34 | SEQ ID NO:25 | SEQ ID NO:18 |
| FIT1006-31b(G-A) | SEQ ID NO:49 | SEQ ID NO:34 | SEQ ID NO:25 | SEQ ID NO:18 |
| FIT1006-35b(D-T) | SEQ ID NO:48 | SEQ ID NO:34 | SEQ ID NO:25 | SEQ ID NO: 17 |

Bispecific FIT-Ig and FIT-Fab binding proteins having the ability to bind both BCMA and CD3 antigens were constructed in the same manner as in Examples 4.1 and 4.2, *supra,* using cDNAs encoding the humanized variable domains listed in Table 11 above and human constant region sequences as shown in Table 3 (SEQ ID NO:26 and SEQ ID NO:27). No linkers between immunoglobulin domains were used, therefore the complete sequences for the FIT-Ig binding proteins can be derived from the sequence information in Tables 11 and 3. For example, the amino acid sequences for the three polypeptide chains of three exemplary FIT-Ig binding proteins disclosed in Table 11 are set forth in Tables 12, 13, and 14 below, for FIT-Igs FIT1006-29b(D-A), FIT1006-31b(D-T), and FIT1006-35b(D-T). These FIT-Igs have the BCMA binding site at the N-terminal position of the assembled chains, and the CD3 binding site is situated internally in the FIT-Ig structure adjacent (N-terminal) to the Fc region but C-terminal to the BCMA binding site. In other words, the domain configuration of the component polypeptide chains is:

Chain 1 (long chain): $VL_{BCMA}$-CL-$VH_{CD3}$-CH1-hinge-CH2-CH3;
Chain 2 (first short chain): $VH_{BCMA}$-CH1;
Chain 3 (second short chain): $VL_{CD3}$-CL;

wherein $VL_{BCMA}$ is the light chain variable domain of a humanized monoclonal antibody recognizing BCMA, $VB_{CD3}$ is the heavy chain variable domain of a humanized monoclonal antibody recognizing CD3, $VL_{CD3}$ is the light chain variable domain of a humanized monoclonal antibody recognizing CD3, $VH_{BCMA}$ is the heavy chain variable domain of a humanized monoclonal antibody recognizing BCMA, each CL is a light chain constant domain (SEQ ID NO:27), each CH1 is a first heavy chain constant domain, and CH1-hinge-CH2-CH3 the C-terminal heavy chain constant region from CH1 through the terminus of the Fc region (see SEQ ID NO:26).

Table 12: Amino Acid Sequences of FIT-Ig FIT1006-29b(D-A) Component Chains

| Polypeptide | SEQ ID NO: | Amino Acid Sequence<br>123456789012345678901234567890 |
|---|---|---|
| FIT1006-29b(D-A) Chain #1 | 50 | DIQMTQSPSSLSASVGDRVTITCGASENIYGALNWYQQKP<br>GKAPKLLIYGATNLAAGVPSRFSGSGSGTDFTLTISSLQP<br>EDFATYYCQSVLTTPWTFGQGTKLEIKRTVAAPSVFIFPP<br>SDEQLKSGTASVVCLLNNFYPREAKVQWKVDNALQSGNSQ<br>ESVTEQDSKDSTYSLSSTLTLSKADYEKHKVYACEVTHQG<br>LSSPVTKSFNRGECEVQLVQSGAEVKKPGASVKVSCKASG<br>FSFTNYYVHWMRQAPGQGLEWMGWISPGSDNTKYNEKFKG<br>RVTMTRDTSISTAYMELSRLRSDDTAVYYCARDDYGNYYF<br>DYWGQGTTVTVSSASTKGPSVFPLAPSSKSTSGGTAALGC<br>LVKDYFPEPVTVSWNSGALTSGVHTFPAVLQSSGLYSLSS<br>VVTVPSSSLGTQTYICNVNHKPSNTKVDKKVEPKSCDKTH<br>TCPPCPAPEAAGGPSVFLFPPKPKDTLMISRTPEVTCVVV<br>DVSHEDPEVKFNWYVDGVEVHNAKTKPREEQYNSTYRVVS<br>VLTVLHQDWLNGKEYKCKVSNKALPAPIEKTISKAKGQPR<br>EPQVYTLPPSREEMTKNQVSLTCLVKGFYPSDIAVEWESN<br>GQPENNYKTTPPVLDSDGSFFLYSKLTVDKSRWQQGNVFS<br>CSVMHEALHNHYTQKSLSLSPGK |
| | | |
| FIT1006-29b(D-A) Chain #2 | 51 | EVQLVQSGAEVKKPGASVKVSCKASGYTFPNFWMHWVRQA<br>PGQRLEWIGAFYPGNDDTYYNQKFKGKATLTADTSASTAY<br>MELSSLRSEDMAVYYCARSGYYGSSDAMDYWGQGTTVTVS<br>SASTKGPSVFPLAPSSKSTSGGTAALGCLVKDYFPEPVTV<br>SWNSGALTSGVHTFPAVLQSSGLYSLSSVVTVPSSSLGTQ<br>TYICNVNHKPSNTKVDKKVEPKSC |
| | | |
| FIT1006-29b(D-A) Chain #3 | 52 | DIVMTQSPDSLAVSLGERATINCKSSQSLLNARTRKNYLA<br>WYQQKPGQPPKLLIYWASTRESGVPDRFSGSGSGTDFTLT<br>ISSLQAEDVAVYYCKQSYILRTFGGGTKVEIKRTVAAPSV<br>FIFPPSDEQLKSGTASVVCLLNNFYPREAKVQWKVDNALQ<br>SGNSQESVTEQDSKDSTYSLSSTLTLSKADYEKHKVYACE<br>VTHQGLSSPVTKSFNRGEC |

Table 13: Amino Acid Sequences of FIT-Ig FIT1006-31b(D-T) Component Chains

| Polypeptide | SEQ ID NO: | Amino Acid Sequence<br>1234567890123456789012345678901234567890 |
|---|---|---|
| FIT1006-31b(D-T) Chain #1 | 53 | DIQMTQSPSSLSASVGDRVTITCGASENIYGALNWYQQKP<br>GKAPKLLIYGATNLATGIPSRFSGSGSGRDFTLTISSLQP<br>EDFATYYCQSVLTTPWTFGQGTKLEIKRTVAAPSVFIFPP<br>SDEQLKSGTASVVCLLNNFYPREAKVQWKVDNALQSGNSQ<br>ESVTEQDSKDSTYSLSSTLTLSKADYEKHKVYACEVTHQG<br>LSSPVTKSFNRGECEVQLVQSGAEVKKPGASVKVSCKASG<br>FSFTNYYVHWMRQAPGQGLEWMGWISPGSDNTKYNEKFKG<br>RVTMTRDTSISTAYMELSRLRSDDTAVYYCARDDYGNYYF<br>DYWGQGTTVTVSSASTKGPSVFPLAPSSKSTSGGTAALGC<br>LVKDYFPEPVTVSWNSGALTSGVHTFPAVLQSSGLYSLSS<br>VVTVPSSSLGTQTYICNVNHKPSNTKVDKKVEPKSCDKTH<br>TCPPCPAPEAAGGPSVFLFPPKPKDTLMISRTPEVTCVVV<br>DVSHEDPEVKFNWYVDGVEVHNAKTKPREEQYNSTYRVVS<br>VLTVLHQDWLNGKEYKCKVSNKALPAPIEKTISKAKGQPR<br>EPQVYTLPPSREEMTKNQVSLTCLVKGFYPSDIAVEWESN<br>GQPENNYKTTPPVLDSDGSFFLYSKLTVDKSRWQQGNVFS<br>CSVMHEALHNHYTQKSLSLSPGK |
| | | |
| FIT1006-31b(D-T) Chain #2 | 54 | EVQLVQSGAEVKKPGASVKVSCKASGYTFTNFWMHWVRQA<br>PGQRLEWMGAFYPGNDDTYYNQKFKGRVTITADTSASTAY<br>MELSSLRSEDMAVYYCARSGYYGSSDAMDYWGQGTTVTVS<br>SASTKGPSVFPLAPSSKSTSGGTAALGCLVKDYFPEPVTV<br>SWNSGALTSGVHTFPAVLQSSGLYSLSSVVTVPSSSLGTQ<br>TYICNVNHKPSNTKVDKKVEPKSC |
| | | |
| FIT1006-31b(D-T) Chain #3 | 55 | DIVMTQSPDSLAVSLGERATINCKSSQSLLNARTRKNYLA<br>WYQQKPGQPPKLLIYWASTRESGVPDRFSGSGSGTDFTLT<br>ISSLQAEDVAVYYCKQSYILRTFGGGTKVEIKRTVAAPSV<br>FIFPPSDEQLKSGTASVVCLLNNFYPREAKVQWKVDNALQ<br>SGNSQESVTEQDSKDSTYSLSSTLTLSKADYEKHKVYACE<br>VTHQGLSSPVTKSFNRGEC |

Table 14: Amino Acid Sequences of FIT-Ig FIT1006-35b(D-T) Component Chains

| Polypeptide | SEQ ID NO: | Amino Acid Sequence 12345678901234567890123456789012345667890 |
|---|---|---|
| FIT1006-35b(D-T) Chain #1 | 80 | DIQMTQSPSSLSASVGDRVTITCGASENIYGALNWYQQKP<br>GKAPKLLIYGATNLATGIPSRFSGSGSGRDFTLTISSLQP<br>EDFATYYCQSVLTTPWTFGQGTKLEIKRTVAAPSVFIFPP<br>SDEQLKSGTASVVCLLNNFYPREAKVQWKVDNALQSGNSQ<br>ESVTEQDSKDSTYSLSSTLTLSKADYEKHKVYACEVTHQG<br>LSSPVTKSFNRGECEVQLVQSGAEVKKPGASVKVSCKASG<br>FSFTNYYVHWMRQAPGQGLEWIGWISPGSDNTKYNEKFKG<br>RVTLTADTSISTAYMELSRLRSDDTAVYYCARDDYGNYYF<br>DYWGQGTTVTVSSASTKGPSVFPLAPSSKSTSGGTAALGC<br>LVKDYFPEPVTVSWNSGALTSGVHTFPAVLQSSGLYSLSS<br>VVTVPSSSLGTQTYICNVNHKPSNTKVDKKVEPKSCDKTH<br>TCPPCPAPEAAGGPSVFLFPPKPKDTLMISRTPEVTCVVV<br>DVSHEDPEVKFNWYVDGVEVHNAKTKPREEQYNSTYRVVS<br>VLTVLHQDWLNGKEYKCKVSNKALPAPIEKTISKAKGQPR<br>EPQVYTLPPSREEMTKNQVSLTCLVKGFYPSDIAVEWESN<br>GQPENNYKTTPPVLDSDGSFFLYSKLTVDKSRWQQGNVFS<br>CSVMHEALHNHYTQKSLSLSPGK |
| FIT1006-35b(D-T) Chain #2 | 81 | EVQLVQSGAEVKKPGASVKVSCKASGYTFTNFWMHWVRQA<br>PGQRLEWMGAFYPGNDDTYYNQKFKGRVTITADTSASTAY<br>MELSSLRSEDMAVYYCARSGYYGSSDAMDYWGQGTTVTVS<br>SASTKGPSVFPLAPSSKSTSGGTAALGCLVKDYFPEPVTV<br>SWNSGALTSGVHTFPAVLQSSGLYSLSSVVTVPSSSLGTQ<br>TYICNVNHKPSNTKVDKKVEPKSC |
| FIT1006-35b(D-T) Chain #3 | 82 | DIVMTQSPDSLAVSLGERATINCKSSQSLLN**A**RTRKNYLA<br>WYQQKPGQPPKLLIYWASTRESGVPDRFSGSGSGTDFTLT<br>ISSLQAEDVAVYYCKQSYILRTFGGGTKVEIKRTVAAPSV<br>FIFPPSDEQLKSGTASVVCLLNNFYPREAKVQWKVDNALQ<br>SGNSQESVTEQDSKDSTYSLSSTLTLSKADYEKHKVYACE<br>VTHQGLSSPVTKSFNRGEC |

Example 5.3: Binding Kinetics of Humanized BCMA/CD3 FIT-Igs

Binding affinities and kinetic constants of BCMA/CD3 bispecific FIT-Ig antibodies was measured by Surface Plasmon Resonance (SPR) at 25° C using a Biacore™ T200 instrument (GE Healthcare) using standard procedures. Results are shown in Table 15.

Briefly, heterodimeric CD3/Fc antigen or BCMA/Fc antigen following a typical amine coupling method was directly immobilized across a biosensor chip, then test antibodies were injected over reaction matrices at a flow rate of 5 $\mu$l/minute and the binding response recorded. The association and dissociation rate constants, $k_{on}$ ($M^{-1}s^{-1}$) and $k_{off}$ ($s^{-1}$) respectively, were determined with a continuous flow rate of 30 $\mu$l/minute. Rate constants were derived by making kinetic binding measurements at five different concentrations of human CD3/Fc protein or human BCMA/Fc protein. The equilibrium dissociation constant $K_D$ (M) of the reaction between antibodies and related target proteins was then calculated from the kinetic rate constants using the formula $K_D = k_{off}/k_{on}$. Affinities for humanized anti-CD3/humanized anti-BCMA FIT-Ig antibodies were measured, as set forth in Table 15, below.

Table 15: Binding Affinities for Humanized BCMA/CD3 FIT-Ig Binding Proteins

| Antigen Target | FIT-Ig | $k_{on}$ (1/Ms) | $k_{off}$ (1/s) | $K_D$ (M) |
|---|---|---|---|---|
| huCD3ε/γ-Fc | FIT1006-29b(D-A) | $3.494 \times 10^5$ | $5.849 \times 10^{-3}$ | $1.674 \times 10^{-8}$ |
| | FIT1006-29b(D-E) | $2.870 \times 10^5$ | $5.711 \times 10^{-3}$ | $1.990 \times 10^{-8}$ |
| | FIT1006-31b(D-E) | $3.369 \times 10^5$ | $5.743 \times 10^{-3}$ | $1.705 \times 10^{-8}$ |
| | FIT1006-31b(D-T) | $3.670 \times 10^5$ | $5.722 \times 10^{-3}$ | $1.559 \times 10^{-8}$ |
| cynoBCMA/Fc | FIT1006-29b(D-A) | $1.919 \times 10^5$ | $2.101 \times 10^{-4}$ | $1.095 \times 10^{-9}$ |
| | FIT1006-29b(D-E) | $1.178 \times 10^5$ | $2.765 \times 10^{-4}$ | $2.346 \times 10^{-9}$ |
| | FIT1006-31b(D-E) | $1.036 \times 10^5$ | $1.665 \times 10^{-4}$ | $1.607 \times 10^{-9}$ |
| | FIT1006-31b(D-T) | $1.881 \times 10^5$ | $1.913 \times 10^{-4}$ | $1.017 \times 10^{-9}$ |
| huBCMA/Fc | FIT1006-29b(D-A) | $8.777 \times 10^4$ | $<5.0 \times 10^{-6}$ | $<5.697 \times 10^{-11}$ |
| | FIT1006-29b(D-E) | $6.424 \times 10^4$ | $3.673 \times 10^{-5}$ | $5.717 \times 10^{-10}$ |
| | FIT1006-31b(D-E) | $6.221 \times 10^4$ | $2.434 \times 10^{-5}$ | $3.913 \times 10^{-10}$ |
| | FIT1006-31b(D-T) | $8.585 \times 10^4$ | $1.688 \times 10^{-5}$ | $1.966 \times 10^{-10}$ |
| cynoCD3ε/γ-Fc | FIT1006-29b(D-A) | $5.407 \times 10^5$ | $6.066 \times 10^{-3}$ | $1.122 \times 10^{-8}$ |
| | FIT1006-29b(D-E) | $5.472 \times 10^5$ | $5.765 \times 10^{-3}$ | $1.054 \times 10^{-8}$ |
| | FIT1006-31b(D-E) | $5.827 \times 10^5$ | $6.321 \times 10^{-3}$ | $1.085 \times 10^{-8}$ |
| | FIT1006-31b(D-T) | $5.459 \times 10^5$ | $6.210 \times 10^{-3}$ | $1.137 \times 10^{-8}$ |

Example 5.4: Humanized bispecific FIT-Igs showed redirected CD3 activation and cytotoxicity

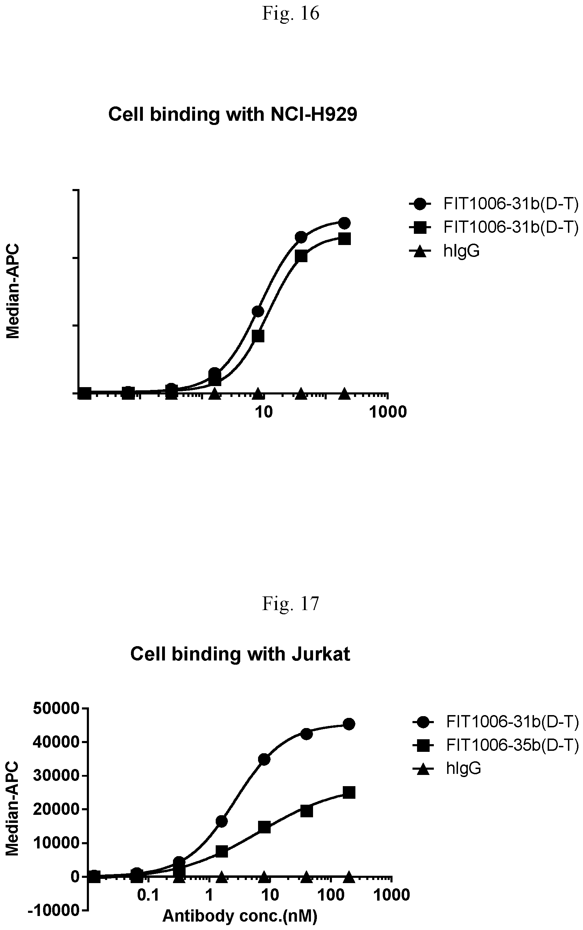

The tumor cell killing potency of BCMA/CD3 humanized bispecific FIT-Ig antibodies was measured in a redirected T cell cytotoxicity assay using human myeloma cell line NCI-H929 as target cells and human T cells as effector cells. Briefly, cells were harvested, washed and resuspended with assay medium (RPMI1640 with 10% FBS). NCI-H929 cells were seeded into flat-bottom 96 wells plate (Corning #3599) at $5 \times 10^4$ cells per well. T cells were purified from human PBMC with a commercial kit (Stemcell #17951) and were added into the same plates at $2 \times 10^5$ cells per well. FIT-Ig binding proteins were then added and incubated with the cell mixture. After 48 hours of incubation at 37° C, LDH release was measured with an assay kit (Promega #G1780). Following the manufacturer's instructions, OD490 readouts were obtained. Target cells NCI-H929 max lysis (100%) minus minimal lysis (0%) was presented as normalization denominator. The percentage of LDH release was plotted against the concentrations of bispecific Abs. In this example, the humanized FIT-Ig showed similar redirected T cell cytotoxicity as the parental chimeric FIT-Ig. The results are shown in Figure 15. The results show that humanized BCMA/CD3 FIT-Igs according to the invention were able to redirect T cell cytotoxicity to NCI-H929 tumor cells in co-culture. Referring to Figures 16 and 17, the binding of two BCMA/CD3 bispecific FIT-Ig binding proteins according to the invention to BCMA-expressing and CD3-expressing target cells is confirmed.

Alternative configurations of two of the exemplary BCMA/CD3 FIT-Ig binding proteins, wherein the outer binding site is the CD3 Fab binding site and the inner binding site is the BCMA Fab binding site of the tandemly arranged Fab regions were prepared, designated FIT1006-31a(D-T) and FIT1006-35a(D-T). The polypeptide chain formulas for these two FIT-Igs were:

Chain 1 (long chain): $VL_{CD3}$-CL-$VH_{BCMA}$-CH1-hinge-CH2-CH3;
Chain 2 (first short chain): $VH_{CD3}$-CH1;
Chain 3 (second short chain): $VL_{BCMA}$-CL;

The amino acid sequences of the polypeptide chains for FIT1006-31a(D-T) and FIT1006-35a(D-T) are given below:

Table 16: Amino Acid Sequences of FIT-Ig FIT1006-31a(D-T) Component Chains

| Polypeptide | SEQ ID NO: | Amino Acid Sequence 1234567890123456789012345678901234567890 |
|---|---|---|
| FIT1006-31a(D-T) Chain #1 | 83 | DIVMTQSPDSLAVSLGERATINCKSSQSLLN**A**RTRKNYLA WYQQKPGQPPKLLIYWASTRESGVPDRFSGSGSGTDFTLT ISSLQAEDVAVYYCKQSYILRTFGGGTKVEIKRTVAAPSV FIFPPSDEQLKSGTASVVCLLNNFYPREAKVQWKVDNALQ SGNSQESVTEQDSKDSTYSLSSTLTLSKADYEKHKVYACE VTHQGLSSPVTKSFNRGECEVQLVQSGAEVKKPGASVKVS CKASGYTFTNFWMHWVRQAPGQRLEWMGAFYPGNDDTYYN QKFKGRVTITADTSASTAYMELSSLRSEDMAVYYCARSGY YGSSDAMDYWGQGTTVTVSSASTKGPSVFPLAPSSKSTSG GTAALGCLVKDYFPEPVTVSWNSGALTSGVHTFPAVLQSS GLYSLSSVVTVPSSSLGTQTYICNVNHKPSNTKVDKKVEP KSCDKTHTCPPCPAPEAAGGPSVFLFPPKPKDTLMISRTP EVTCVVVDVSHEDPEVKFNWYVDGVEVHNAKTKPREEQYN STYRVVSVLTVLHQDWLNGKEYKCKVSNKALPAPIEKTIS KAKGQPREPQVYTLPPSREEMTKNQVSLTCLVKGFYPSDI AVEWESNGQPENNYKTTPPVLDSDGSFFLYSKLTVDKSRW QQGNVFSCSVMHEALHNHYTQKSLSLSPGK |
| FIT1006-31a(D-T) Chain #2 | 84 | EVQLVQSGAEVKKPGASVKVSCKASGFSFTNYYVHWMRQA PGQGLEWMGWISPGSDNTKYNEKFKGRVTMTRDTSISTAY MELSRLRSDDTAVYYCARDDYGNYYFDYWGQGTTVTVSSA STKGPSVFPLAPSSKSTSGGTAALGCLVKDYFPEPVTVSW NSGALTSGVHTFPAVLQSSGLYSLSSVVTVPSSSLGTQTY ICNVNHKPSNTKVDKKVEPKSC |
| FIT1006-31a(D-T) Chain #3 | 85 | DIQMTQSPSSLSASVGDRVTITCGASENIYGALNWYQQKP GKAPKLLIYGATNLATGIPSRFSGSGSGRDFTLTISSLQP EDFATYYCQSVLTTPWTFGQGTKLEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |

Table 17: Amino Acid Sequences of FIT-Ig FIT1006-35a(D-T) Component Chains

| Polypeptide | SEQ ID NO: | Amino Acid Sequence 1234567890123456789012345678901234567890 |
|---|---|---|
| FIT1006-35a(D-T) Chain #1 | 86 | DIVMTQSPDSLAVSLGERATINCKSSQSLLN**A**RTRKNYLA WYQQKPGQPPKLLIYWASTRESGVPDRFSGSGSGTDFTLT ISSLQAEDVAVYYCKQSYILRTFGGGTKVEIKRTVAAPSV FIFPPSDEQLKSGTASVVCLLNNFYPREAKVQWKVDNALQ SGNSQESVTEQDSKDSTYSLSSTLTLSKADYEKHKVYACE VTHQGLSSPVTKSFNRGECEVQLVQSGAEVKKPGASVKVS CKASGYTFTNFWMHWVRQAPGQRLEWMGAFYPGNDDTYYN QKFKGRVTITADTSASTAYMELSSLRSEDMAVYYCARSGY YGSSDAMDYWGQGTTVTVSSASTKGPSVFPLAPSSKSTSG GTAALGCLVKDYFPEPVTVSWNSGALTSGVHTFPAVLQSS GLYSLSSVVTVPSSSLGTQTYICNVNHKPSNTKVDKKVEP KSCDKTHTCPPCPAPEAAGGPSVFLFPPKPKDTLMISRTP EVTCVVVDVSHEDPEVKFNWYVDGVEVHNAKTKPREEQYN STYRVVSVLTVLHQDWLNGKEYKCKVSNKALPAPIEKTIS KAKGQPREPQVYTLPPSREEMTKNQVSLTCLVKGFYPSDI AVEWESNGQPENNYKTTPPVLDSDGSFFLYSKLTVDKSRW QQGNVFSCSVMHEALHNHYTQKSLSLSPGK |
| FIT1006-35a(D-T) Chain #2 | 87 | EVQLVQSGAEVKKPGASVKVSCKASGFSFTNYYVHWMRQA PGQGLEWIGWISPGSDNTKYNEKFKGRVTLTADTSISTAY MELSRLRSDDTAVYYCARDDYGNYYFDYWGQGTTVTVSSA STKGPSVFPLAPSSKSTSGGTAALGCLVKDYFPEPVTVSW NSGALTSGVHTFPAVLQSSGLYSLSSVVTVPSSSLGTQTY ICNVNHKPSNTKVDKKVEPKSC |
| FIT1006-35a(D-T) Chain #3 | 88 | DIQMTQSPSSLSASVGDRVTITCGASENIYGALNWYQQKP GKAPKLLIYGATNLATGIPSRFSGSGSGRDFTLTISSLQP EDFATYYCQSVLTTPWTFGQGTKLEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |

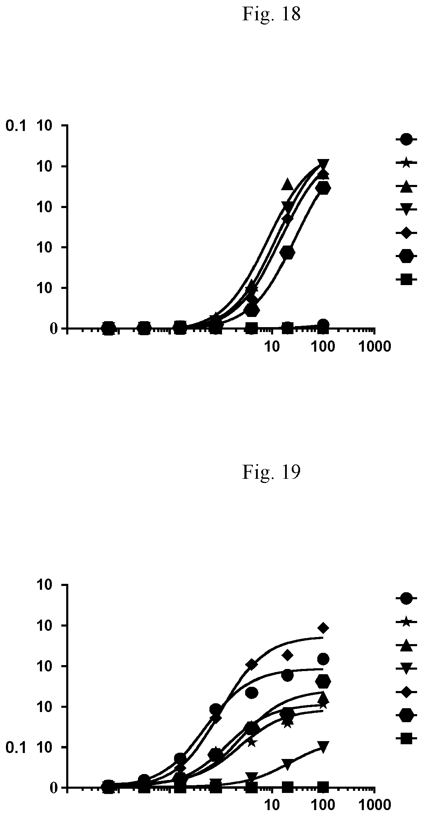

The binding activity to BCMA-expressing and CD3-expressing target cells of the two alternative configurations are shown in Fig. 18 and Fig. 19 respectively. Comparing the two configurations for each target, the corresponding binding domain placed distal to Fc exhibited relatively higher binding activity than the same binding domain placed proximal to Fc, indicating certain impact of configuration on binding activity. Nonetheless, the results confirm both configurations possess the desired target binding activity towards both BCMA and CD3.

Example 6 Treatment with BCMA x CD3 FIT-Ig decreases NCI-H929 tumor volume in human PBMC engrafted NPSG mice

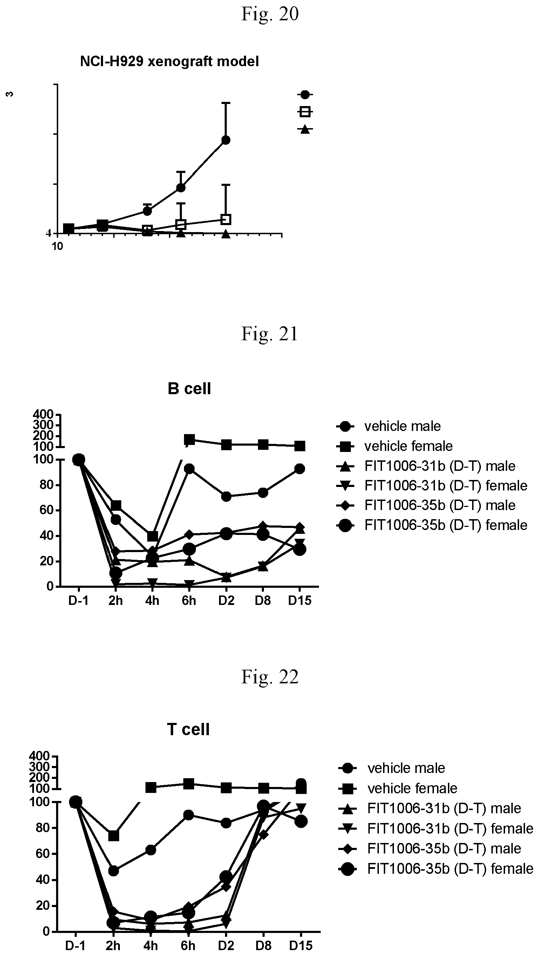

The antitumor efficacy was evaluated in NPSG mice, which is an immunodeficient strain lacking T cells, B cells and natural killer cells. The NCI-H929 cells ($5 \times 10^6$) were injected subcutaneously into the right dorsal flank NPSG mice. In the same day, the mice received a single intravenous dose of $5 \times 10^6$ human PBMC. The animals were randomized based on tumor size (70~140 mm$^3$) on day 11 and treatment was initiated in the same day. Tumor growth was monitored by caliper measurements. The study was terminated on day 25, mice were euthanized when tumors size exceeded 3000 mm$^3$. Mice were treated once a week for 3 weeks (QW x 3) with 6 mg/kg of FIT1006-31b(D-T) or FIT1006-35b(D-T) or vehicle by intraperitoneal (i.p.) injection. As shown in Fig. 20, FIT-Ig treatment group mice showed significant tumor growth inhibition by comparing with vehicle group. Especially for the FIT 1006-35b(D-T) treatment group, tumors were completely eradicated.

Example 7 BCMA x CD3 FIT-Ig depletes B cell populations in cynomolgus monkeys and shows limited cytokine release profile

Cynomolgus monkeys B cells were reported to have higher BCMA expression than human (Seckinger, A. et al., (2017). Target Expression, Generation, Preclinical Activity, and Pharmacokinetics of the BCMA-T Cell Bispecific Antibody EM801 for Multiple Myeloma Treatment. Cancer Cell, 31(3), 396-410). A pilot non-GLP toxicology and pharmacology study was performed in cynomolgus monkeys to evaluate the ability of BCMA x CD3 FIT-Ig to deplete B cell populations in these animals. The study has three groups each consists of 1 male and 1 female monkey with comparable body weight across these groups, Group 1 received vehicle, Group 2 received a single injection of 0.5mg/kg FIT1006-31b(D-T), and Group 3 received a single injection of 0.5mg/kg FIT1006-35b(D-T), all dosed by i.v. injection on Day 1. Blood samples were collected via forelimb or hindlimb subcutaneous vein 2 days before dosing (Day -1, baseline), 2, 4, 6 and 24 hours after dosing on Day 1, and on Day 8 and Day 15. Blood samples were analyzed by FACS for B and T cell markers and relative percentage change of each population was determined by comparing with the baseline level on Day -1. Serum samples were also analyzed for cytokine levels (INFγ, IL-2, IL-6 and TNFα) using a commercial Cytometric Bead Array (CBA) kit.

Figure 21 demonstrates over 50% depletion of circulating B cells resulted from administration of the BCMA x CD3 FIT-Ig from the first post-dosing point (2 hours postdosing) till the last time point (day 15). A transient B cell depletion was also seen in the vehicle group by the second and third time point (2 hours and 4 hours of day 1), which may relate to the blood sampling schedule. But the B cell population of vehicle group exhibited quick recovery reaching plateau by 6 hours post dosing.

As shown in Figure 22, circulating T cell levels in FIT-Ig treatment groups exhibited a transient loss, which was recovered to the level of vehicle group on day 8 and maintained until the end of the experiment. The transient T cell loss was considered due to T cell activation and redistribution upon treatment.

The invention may be embodied in other specific forms without departing from the essential characteristics of the invention described above. The foregoing embodiments are therefore to be considered illustrative rather than limiting of the invention described herein. The scope of the invention is indicated by the appended claims.

Further disclosed are the following embodiments:

1. An anti-CD3 antibody or antigen-binding portion thereof, comprising a set of six CDRs, of CDR-H1, CDR-H2, CDR-H3, CDR-L1, CDR-L2, and CDR-L3, selected from the group of CDR sets as follows:

| CDR Set No. | CDR | CDR Amino Acid Sequence | SEQ ID NO: |
|---|---|---|---|
| 1 | CDR-H1 | NYYVH | SEQ ID NO:56 |
| | CDR-H2 | WISPGSDNTKYNEKFKG | SEQ ID NO:57 |
| | CDR-H3 | DDYGNYYFDY | SEQ ID NO:58 |
| | CDR-L1 | KSSQSLLNSRTRKNYLA | SEQ ID NO:59 |
| | CDR-L2 | WASTRES | SEQ ID NO:60 |
| | CDR-L3 | KQSYILRT | SEQ ID NO:61 |
| 2 | CDR-H1 | NYYIH | SEQ ID NO:62 |
| | CDR-H2 | WINLGDVNTKFNEKFKD | SEQ ID NO:63 |
| | CDR-H3 | DGYSFYYFDF | SEQ ID NO:64 |
| | CDR-L1 | KASQSLFNSRTRKNYLA | SEQ ID NO:65 |
| | CDR-L2 | WASTRES | SEQ ID NO:66 |
| | CDR-L3 | IQSHTLRT | SEQ ID NO:67 |

2. The anti-CD3 antibody or antigen-binding portion thereof according to embodiment 1, comprising VH and VL domains having amino acid sequences selected from the following VH/VL pairs:

| VH/VL Pair | VH/VL Pair |
|---|---|
| SEQ ID NO:6/SEQ ID NO:7 | SEQ ID NO:19/SEQ ID NO:20 |
| SEQ ID NO:8/SEQ ID NO:9 | SEQ ID NO:11/SEQ ID NO:21 |
| SEQ ID NO:11/SEQ ID NO:20 | SEQ ID NO:12/SEQ ID NO:21 |
| SEQ ID NO:12/SEQ ID NO:20 | SEQ ID NO:13/SEQ ID NO:21 |
| SEQ ID NO:13/SEQ ID NO:20 | SEQ ID NO:14/SEQ ID NO:21 |
| SEQ ID NO:14/SEQ ID NO:20 | SEQ ID NO:15/SEQ ID NO:21 |
| SEQ ID NO:15/SEQ ID NO:20 | SEQ ID NO:16/SEQ ID NO:21 |
| SEQ ID NO:16/SEQ ID NO:20 | SEQ ID NO:17/SEQ ID NO:21 |
| SEQ ID NO:17/SEQ ID NO:20 | SEQ ID NO:18/SEQ ID NO:21 |
| SEQ ID NO:18/SEQ ID NO:20 | SEQ ID NO:19/SEQ ID NO:21 |
| SEQ ID NO:11/SEQ ID NO:25 | SEQ ID NO:12/SEQ ID NO:25 |
| SEQ ID NO:15/SEQ ID NO:25 | SEQ ID NO:17/SEQ ID NO:25. |
| SEQ ID NO:18/SEQ ID NO:25 | |

3. A pharmaceutical composition comprising at least one anti-CD3 antibody or antigen-binding fragment thereof according to embodiments 1 or 2, and a pharmaceutically acceptable carrier.

4. Use of the anti-CD3 antibody or antigen-binding portion thereof according to embodiments 1 or 2 for preparation of a medicament for treating a disease or disorder in which CD3-mediated activity and/or BCMA-mediated activity is detrimental.

5. The use according to embodiment 4, wherein said disease is a cancer and optionally selected from: a multiple myeloma, a melanoma (e.g., metastatic malignant melanoma), a renal cancer (e.g., clear cell carcinoma), a prostate cancer (e.g., hormone refractory prostate adenocarcinoma), a pancreatic adenocarcinoma, a breast cancer, a colon cancer, a lung cancer (e.g., non-small cell lung cancer), an esophageal cancer, a squamous cell carcinoma of the head and neck, a liver cancer, an ovarian cancer, a cervical cancer, a thyroid cancer, a glioblastoma, a glioma, a leukemia, a lymphoma, and a primary bone cancer (e.g., osteosarcoma, Ewing sarcoma, malignant fibrous histiocytoma, or chondrosarcoma).

6. A binding protein comprising first, second and third polypeptide chains, or a FIT-Fab fragment thereof, wherein

said first polypeptide chain comprises, from amino to carboxyl terminus, (i) $VL_A$-CL-$VB_B$-CH1-Fc wherein CL is directly fused to $VH_B$, or (ii) $VH_B$-CH1-$VL_A$-CL-$F_C$ wherein CH1 is directly fused to $VL_A$;

said second polypeptide chain comprises, from amino to carboxyl terminus, $VH_A$-CH1; and

said third polypeptide chain comprises, from amino to carboxyl terminus, $VL_B$-CL; wherein VL is a light chain variable domain, CL is a light chain constant domain, VH is a heavy chain variable domain, CH1 is a heavy chain constant domain, Fc is an immunoglobulin Fc region, A is an epitope of CD3 or BCMA and B is an epitope of CD3 or BCMA, with the proviso that A and B are different, said binding protein being capable of binding to both CD3 and BCMA.

7. The binding protein or FIT-Fab fragment according to embodiment 6, wherein the $VL_A$ and $VH_A$ are variable domains from a parental antibody binding to one of the antigen targets CD3 or BCMA, and the $VL_B$ and $VH_B$ are variable domains from a different parental antibody binding to the other of the antigen targets CD3 or BCMA.

8. The binding protein or FIT-Fab fragment of embodiment 7, wherein

said first polypeptide chain comprises, from amino to carboxyl terminus, $VL_{CD3}$-CL-$VH_{BCMA}$-CH1-Fc wherein CL is directly fused to $VH_{BCMA}$, said second polypeptide chain comprises, from amino to carboxyl terminus, $VH_{CD3}$-CH1, and said third polypeptide chain comprises, from amino to carboxyl terminus, $VL_{BCMA}$-CL;

said first polypeptide chain comprises, from amino to carboxyl terminus, $VL_{BCMA}$-CL-$VH_{CD3}$-CH1-Fc wherein CL is directly fused to $VH_{CD3}$, said second polypeptide chain comprises, from amino to carboxyl terminus, $VH_{BCMA}$-CH1, and said third polypeptide chain comprises, from amino to carboxyl terminus, $VL_{CD3}$-CL;

said first polypeptide chain comprises, from amino to carboxyl terminus, $VH_{BCMA}$-CH1-$VL_{CD3}$-CL-Fc wherein CH1 is directly fused to VL$_{CD3}$, said second polypeptide chain comprises, from amino to carboxyl terminus, VL$_{BCMA}$-CL, and said third polypeptide chain comprises, from amino to carboxyl terminus, VH$_{CD3}$-CH1; or

said first polypeptide chain comprises, from amino to carboxyl terminus, VH$_{CD3}$-CH1-VL$_{BCMA}$-CL-Fc wherein CH1 is directly fused to VL$_{BCMA}$, said second polypeptide chain comprises, from amino to carboxyl terminus, VL$_{CD3}$-CL, and said third polypeptide chain comprises, from amino to carboxyl terminus, VH$_{BCMA}$-CH1;

wherein VL$_{CD3}$ is a light chain variable domain of an anti-CD3 antibody, CL is an antibody light chain constant domain, VH$_{CD3}$ is a heavy chain variable domain of an anti-CD3 antibody, CH1 is an antibody first heavy chain constant domain, VL$_{BCMA}$ is a light chain variable domain of an anti-BCMA antibody, VH$_{BCMA}$ is a heavy chain variable domain of an anti-BCMA antibody, and Fc is an antibody Fc region.

9. The binding protein or FIT-Fab fragment of embodiment 8, wherein, the domains VL$_{CD3}$-CL are the same as the light chain of an anti-CD3 parental antibody, the domains VH$_{CD3}$-CH1 are the same as the heavy chain variable and heavy chain first constant domains of an anti-CD3 parental antibody, the domains VL$_{BCMA}$-CL are the same as the light chain of an anti-BCMA parental antibody, and the domains VH$_{BCMA}$-CH1 are the same as the heavy chain variable and heavy chain first constant domains of an anti-BCMA parental antibody.

10. The binding protein or FIT-Fab fragment of embodiment 6, wherein

said first polypeptide chain comprises a sequence of amino acids of SEQ ID NO:50, said second polypeptide chain comprises a sequence of amino acids of SEQ ID NO:51, and said third polypeptide chain comprises a sequence of amino acids of SEQ ID NO:52;

said first polypeptide chain comprises a sequence of amino acids of SEQ ID NO:53, said second polypeptide chain comprises a sequence of amino acids of SEQ ID NO:54, and said third polypeptide chain comprises a sequence of amino acids of SEQ ID NO:55;

said first polypeptide chain comprises a sequence of amino acids of SEQ ID NO:80, said second polypeptide chain comprises a sequence of amino acids of SEQ ID NO:81, and said third polypeptide chain comprises a sequence of amino acids of SEQ ID NO:82;

said first polypeptide chain comprises a sequence of amino acids of SEQ ID NO:83, said second polypeptide chain comprises a sequence of amino acids of SEQ ID NO:84, and said third polypeptide chain comprises a sequence of amino acids of SEQ ID NO:85; or

said first polypeptide chain comprises a sequence of amino acids of SEQ ID NO:86, said second polypeptide chain comprises a sequence of amino acids of SEQ ID NO:87, and said third polypeptide chain comprises a sequence of amino acids of SEQ ID NO:88.

11. A pharmaceutical composition comprising at least one binding protein or FIT-Fab fragment according to any one of embodiments 6-10 and a pharmaceutically acceptable carrier.

12. Use of a binding protein or FIT-Fab fragment according to any one of embodiments 6-10 for preparation of a medicament for treating a disease or disorder in which CD3-mediated activity and/or BCMA-mediated activity is detrimental.

13. The use according to embodiment 12 wherein said disease is a cancer and optionally selected from: a multiple myeloma, a melanoma (e.g., metastatic malignant melanoma), a renal cancer (e.g., clear cell carcinoma), a prostate cancer (e.g., hormone refractory prostate adenocarcinoma), a pancreatic adenocarcinoma, a breast cancer, a colon cancer, a lung cancer (e.g., non-small cell lung cancer), an esophageal cancer, a squamous cell carcinoma of the head and neck, a liver cancer, an ovarian cancer, a cervical cancer, a thyroid cancer, a glioblastoma, a glioma, a leukemia, a lymphoma, and a primary bone cancer (e.g., osteosarcoma, Ewing sarcoma, malignant fibrous histiocytoma, or chondrosarcoma).

14. A method of treating a disorder wherein CD3-mediated and/or BCMA-mediated activity is detrimental, comprising administering to a subject in need thereof an effective amount of a binding protein or FIT-Fab fragment according to any one of embodiments 6-10, or a combination thereof.

15. The method according to embodiment 14 wherein said disease is a cancer and optionally selected from: a multiple myeloma, a melanoma (e.g., metastatic malignant melanoma), a renal cancer (e.g., clear cell carcinoma), a prostate cancer (e.g., hormone refractory prostate adenocarcinoma), a pancreatic adenocarcinoma, a breast cancer, a colon cancer, a lung cancer (e.g., non-small cell lung cancer), an esophageal cancer, a squamous cell carcinoma of the head and neck, a liver cancer, an ovarian cancer, a cervical cancer, a thyroid cancer, a glioblastoma, a glioma, a leukemia, a lymphoma, and a primary bone cancer (e.g., osteosarcoma, Ewing sarcoma, malignant fibrous histiocytoma, or chondrosarcoma).

16. The method according to embodiments 14 or 15, wherein said subject is a human.

17. An anti-BCMA antibody or an antigen-binding portion thereof, comprising a set of six CDRs, of CDR-H1, CDR-H2, CDR-H3, CDR-L1, CDR-L2, and CDR-L3, selected from the group of CDR sets as follows:

| CDR Set No. | CDR | CDR Amino Acid Sequence | SEQ ID NO: |
|---|---|---|---|
| 3 | CDR-H1 | NFWMH | SEQ ID NO:74 |
| | CDR-H2 | AFYPGNDDTYYNQKFK | SEQ ID NO:75 |
| | CDR-H3 | SGYYGSSDAMDY | SEQ ID NO:76 |
| | CDR-L1 | GASENIYGALN | SEQ ID NO:77 |
| | CDR-L2 | GATNLAD | SEQ ID NO:78 |
| | CDR-L3 | QSVLTTPWT | SEQ ID NO:79 |
| 4 | CDR-H1 | NYGLN | SEQ ID NO:68 |
| | CDR-H2 | WINTYSGHPTYVDDFKG | SEQ ID NO:69 |
| | CDR-H3 | EKDDGYRLGLDY | SEQ ID NO:70 |
| | CDR-L1 | SASSSVSYMY | SEQ ID NO:71 |
| | CDR-L2 | DTSNLVS | SEQ ID NO:72 |
| | CDR-L3 | LQYSGYPYT | SEQ ID NO:73 |

18. The anti-BCMA antibody or antigen-binding portion thereof according to embodiment 17, comprising VH and VL domains having amino acid sequences selected from the following VH/VL pairs:

| VH/VL Pair | VH/VL Pair |
|---|---|
| SEQ ID NO:29/SEQ ID NO:30 | SEQ ID NO:36/SEQ ID NO:40 |
| SEQ ID NO:31/SEQ ID NO:32 | SEQ ID NO:36/SEQ ID NO:41 |
| SEQ ID NO:34/SEQ ID NO:38 | SEQ ID NO:36/SEQ ID NO:42 |
| SEQ ID NO:35/SEQ ID NO:38 | SEQ ID NO:36/SEQ ID NO:43 |
| SEQ ID NO:36/SEQ ID NO:38 | SEQ ID NO:36/SEQ ID NO:44 |
| SEQ ID NO:37/SEQ ID NO:38 | SEQ ID NO:34/SEQ ID NO:45 |
| SEQ ID NO:34/SEQ ID NO:39 | SEQ ID NO:34/SEQ ID NO:46 |
| SEQ ID NO:35/SEQ ID NO:39 | SEQ ID NO:34/SEQ ID NO:47 |
| SEQ ID NO:36/SEQ ID NO:39 | SEQ ID NO:34/SEQ ID NO:48 |
| SEQ ID NO:37/SEQ ID NO:39 | SEQ ID NO:34/SEQ ID NO:49 |

19. A pharmaceutical composition comprising at least one anti-BCMA antibody or antigen-binding fragment thereof according to embodiments 17 or 18, and a pharmaceutically acceptable carrier.

20. Use of the anti-CD3 antibody or antigen-binding portion thereof according to embodiments 17 or 18 for preparation of a medicament for treating a disease or disorder in which CD3-mediated activity and/or BCMA-mediated activity is detrimental.

21. The use according to embodiment 20 wherein said disease is a cancer and optionally selected from: a multiple myeloma, a melanoma (e.g., metastatic malignant melanoma), a renal cancer (e.g., clear cell carcinoma), a prostate cancer (e.g., hormone refractory prostate adenocarcinoma), a pancreatic adenocarcinoma, a breast cancer, a colon cancer, a lung cancer (e.g., non-small cell lung cancer), an esophageal cancer, a squamous cell carcinoma of the head and neck, a liver cancer, an ovarian cancer, a cervical cancer, a thyroid cancer, a glioblastoma, a glioma, a leukemia, a lymphoma, and a primary bone cancer (e.g., osteosarcoma, Ewing sarcoma, malignant fibrous histiocytoma, or chondrosarcoma).

**Claims**

1. An anti-BCMA antibody or an antigen-binding portion thereof, comprising a set of six CDRs, of CDR-H1, CDR-H2, CDR-H3, CDR-L1, CDR-L2, and CDR-L3, selected from the group of CDR sets as follows:

| CDR Set No. | CDR | CDR Amino Acid Sequence | SEQ ID NO: |
|---|---|---|---|
| 3 | CDR-H1 | NFWMH | SEQ ID NO:74 |
| | CDR-H2 | AFYPGNDDTYYNQKFK | SEQ ID NO:75 |
| | CDR-H3 | SGYYGSSDAMDY | SEQ ID NO:76 |
| | CDR-L1 | GASENIYGALN | SEQ ID NO:77 |
| | CDR-L2 | amino acid residues 50-56 of SEQ ID NO: 48 | |
| | CDR-L3 | QSVLTTPWT | SEQ ID NO:79 |
| 4 | CDR-H1 | NYGLN | SEQ ID NO:68 |
| | CDR-H2 | WINTYSGHPTYVDDFKG | SEQ ID NO:69 |
| | CDR-H3 | EKDDGYRLGLDY | SEQ ID NO:70 |
| | CDR-L1 | SASSSVSYMY | SEQ ID NO:71 |
| | CDR-L2 | DTSNLVS | SEQ ID NO:72 |
| | CDR-L3 | LQYSGYPYT | SEQ ID NO:73 |

2. The anti-BCMA antibody or antigen-binding portion thereof according to Claim 1, comprising VH and VL domains having amino acid sequences selected from the following VH/VL pairs:

| VH/VL Pair |
|---|
| SEQ ID NO:29/SEQ ID NO:30 |
| SEQ ID NO:36/SEQ ID NO:43 |
| SEQ ID NO:34/SEQ ID NO:48 |

3. The anti-BCMA antibody or antigen-binding portion thereof according to claim 1 or claim 2, comprising a (i) VH domain having a sequence selected from SEQ ID NO: 29, SEQ ID NO: 34, or SEQ ID NO: 36 and a CH1-hinge-CH2-CH3 human constant IgG1 having a sequence of SEQ ID NO: 26; and (ii) a VL domain having a sequence selected from SEQ ID NO: 30, SEQ ID NO: 43, or SEQ ID NO: 48 and a CLκ having a sequence of SEQ ID NO: 27.

4. A pharmaceutical composition comprising at least one anti-BCMA antibody or antigen-binding fragment thereof according to any one of claims 1 to 3, and a pharmaceutically acceptable carrier.

5. An anti-BCMA antibody or antigen-binding portion thereof according to any one of claims 1 to 3, or a pharmaceutical composition of claim 4 for use in treating a disease or disorder in which BCMA-mediated activity is detrimental.

6. The anti-BCMA antibody or antigen-binding portion thereof or the pharmaceutical composition for use according to claim 5 wherein said disease is a cancer and optionally selected from: a multiple myeloma, a melanoma (e.g., metastatic malignant melanoma), a renal cancer (e.g., clear cell carcinoma), a prostate cancer (e.g., hormone refractory prostate adenocarcinoma), a pancreatic adenocarcinoma, a breast cancer, a colon cancer, a lung cancer (e.g., non-small cell lung cancer), an esophageal cancer, a squamous cell carcinoma of the head and neck, a liver cancer, an ovarian cancer, a cervical cancer, a thyroid cancer, a glioblastoma, a glioma, a leukemia, a lymphoma, and a primary bone cancer (e.g., osteosarcoma, Ewing sarcoma, malignant fibrous histiocytoma, or chondrosarcoma).

**7.** An anti-BCMA and anti-CD3 bi-specific antibody or an antigen-binding portion thereof, comprising a set of twelve CDRs, of CDR-H1-1, CDR-H2-1, CDR-H3-1, CDR-L1-1, CDR-L2-1, and CDR-L3-1, selected from the group of CDR sets as follows:

| CDR Set No. | CDR | CDR Amino Acid Sequence | SEQ ID NO: |
|---|---|---|---|
| 1 | CDR-H1 | NFWMH | SEQ ID NO:74 |
| | CDR-H2 | AFYPGNDDTYYNQKFK | SEQ ID NO:75 |
| | CDR-H3 | SGYYGSSDAMDY | SEQ ID NO:76 |
| | CDR-L1 | GASENIYGALN | SEQ ID NO:77 |
| | CDR-L2 | amino acid residues 50-56 of SEQ ID NO: 48 | |
| | CDR-L3 | QSVLTTPWT | SEQ ID NO:79 |
| 2 | CDR-H1 | NYGLN | SEQ ID NO:68 |
| | CDR-H2 | WINTYSGHPTYVDDFKG | SEQ ID NO:69 |
| | CDR-H3 | EKDDGYRLGLDY | SEQ ID NO:70 |
| | CDR-L1 | SASSSVSYMY | SEQ ID NO:71 |
| | CDR-L2 | DTSNLVS | SEQ ID NO:72 |
| | CDR-L3 | LQYSGYPYT | SEQ ID NO:73 |

and CDR-H1-2, CDR-H2-2, CDR-H3-2, CDR-L1-2, CDR-L2-2, and CDR-L3-2, selected from the group of CDR sets as follows:

| CDR Set No. | CDR | CDR Amino Acid Sequence | SEQ ID NO: |
|---|---|---|---|
| 3 | CDR-H1 | NYYVH | SEQ ID NO: 56 |
| | CDR-H2 | WISPGSDNTKYNEKFKG | SEQ ID NO: 57 |
| | CDR-H3 | DDYGNYYFDY | SEQ ID NO: 58 |
| | CDR-L1 | amino acid residues 24-40 of SEQ ID NO: 25 | |
| | CDR-L2 | WASTRES | SEQ ID NO: 60 |
| | CDR-L3 | KQSYILRT | SEQ ID NO: 61 |
| 4 | CDR-H1 | NYYIH | SEQ ID NO: 62 |
| | CDR-H2 | WINLGDVNTKFNEKFKD | SEQ ID NO: 63 |
| | CDR-H3 | DGYSFYYFDF | SEQ ID NO: 64 |
| | CDR-L1 | KASQSLFNSRTRKNYLA | SEQ ID NO: 65 |
| | CDR-L2 | WASTRES | SEQ ID NO: 66 |
| | CDR-L3 | IQSHTLRT | SEQ ID NO: 67. |

**8.** The anti-BCMA and anti-CD3 bi-specific antibody or an antigen-binding portion thereof, comprising VH and VL domains having amino acid sequences selected from the following VH/VL pairs:

| VH/VL Pair | VH/VL Pair |
|---|---|
| SEQ ID NO:29/SEQ ID NO:30 | SEQ ID NO:36/SEQ ID NO:43 |
| | SEQ ID NO:34/SEQ ID NO:48 |

and

| VH/VL Pair | VH/VL Pair |
|---|---|
| SEQ ID NO:8/SEQ ID NO:9 | SEQ ID NO:12/SEQ ID NO:25 |
| SEQ ID NO:11/SEQ ID NO:25 | SEQ ID NO:17/SEQ ID NO:25 |
| SEQ ID NO:15/SEQ ID NO:25 | SEQ ID NO: 18/SEQ ID NO:25. |

9. The anti-BCMA and anti-CD3 bi-specific antibody or an antigen-binding portion thereof according claim 7 or claim 8, comprising (i) a first VH domain having a sequence selected from SEQ ID NO: 29, SEQ ID NO: 34, or SEQ ID NO: 36 and a CH1-hinge-CH2-CH3 human constant IgG1 having a sequence of SEQ ID NO: 26; (ii) a first VL domain having a sequence selected from SEQ ID NO: 30, SEQ ID NO: 43, or SEQ ID NO: 48 and a CLκ having a sequence of SEQ ID NO: 27; (iii) a second VH domain having a sequence selected from SEQ ID NO: 8, SEQ ID NO: 11, SEQ ID NO: 12, SEQ ID NO: 15, SEQ ID NO: 17, or SEQ ID NO: 18 and a CH1-hinge-CH2-CH3 human constant IgG1 having a sequence of SEQ ID NO: 26; and (iv) a second VL domain having a sequence selected from SEQ ID NO: 9 or SEQ ID NO: 25 and a CLκ having a sequence of SEQ ID NO: 27.

10. The anti-BCMA and anti-CD3 bi-specific antibody or an antigen-binding portion thereof according to any one of claims 7 to 9, comprising three polypeptides, wherein the first polypeptide has a sequence of SEQ ID NO: 80, the second polypeptide has a sequence of SEQ ID NO: 81, and the third polypeptide has a sequence of SEQ ID NO: 82.

11. A pharmaceutical composition comprising at least one anti-BCMA and anti-CD3 bi-specific antibody or antigen-binding fragment thereof according to any one of claims 7 to 10, and a pharmaceutically acceptable carrier.

12. An anti-BCMA and anti-CD3 bi-specific antibody or antigen-binding fragment thereof according to any one of claims 7 to 10, or the pharmaceutical composition of claim for use in treating a disease or disorder in which CD3-mediated activity and/or BCMA-mediated activity is detrimental.

13. The anti-BCMA and anti-CD3 bi-specific antibody or antigen-binding fragment thereof or the pharmaceutical composition for use according to claim 12, wherein said disease is a cancer and optionally selected from: a multiple myeloma, a melanoma (e.g., metastatic malignant melanoma), a renal cancer (e.g., clear cell carcinoma), a prostate cancer (e.g., hormone refractory prostate adenocarcinoma), a pancreatic adenocarcinoma, a breast cancer, a colon cancer, a lung cancer (e.g., non-small cell lung cancer), an esophageal cancer, a squamous cell carcinoma of the head and neck, a liver cancer, an ovarian cancer, a cervical cancer, a thyroid cancer, a glioblastoma, a glioma, a leukemia, a lymphoma, and a primary bone cancer (e.g., osteosarcoma, Ewing sarcoma, malignant fibrous histiocytoma, or chondrosarcoma).

## Fig. 1

|  | mAbCD3-001 | mAbCD3-002 | control α-CD3 mAb |
|---|---|---|---|
| EC50 | 0.02800 | 0.01005 | 0.01868 |

## Fig. 2

cyno CD3e/g

|  | mAbCD3-001 | mAbCD3-002 | control α-CD3 mAb |
|---|---|---|---|
| EC50 | 0.02354 | 0.01093 | 0.02090 |

Fig. 3

Fig. 4

Fig 5

A

**VK1**

- HuEM0006-01-4
- HuEM0006-01-4
- hIgG

MFI

0.1

B

**VK1**

- HuEM0006-01-4
- hIgG

MFI

0.1

Antibody conc.(nM)

Fig. 5 cont'd

C

**VK1**

D

**VK1**

Fig. 5 cont'd

E

F

Fig. 5 cont'd

G

**VK1A**

H

**VK1A**

HuEM0006-01-14
HuEM0006-01-17
HuEM0006-01-20
HuEM0006-01C
hIgG

Fig. 6

| | EC50 |
|---|---|
| Anti-BAFF | 19.77 |
| TAB1 | 9.702 |
| TAB2 | 6.879 |
| mIgG anti-RAC | 53.03 |
| mAbBCMA-002 | 34.78 |
| mAbBCMA-003 | 16.71 |

Fig. 7

**BAFF-induced NF-kB phosphorylation (HEK293F-BCMA-NF-kB-luc)**

- ● TAB1
- ■ TAB2
- ◆ mAbBCMA-002
- ○ mAbBCMA-003
- □ murine α-Ro1 IgG(negative control)

| | TAB1 | TAB2 | mAbBCMA-002 | mAbBCMA-003 |
|---|---|---|---|---|
| EC50 | 4.163 | 5.417 | 20.05 | 3.869 |

Fig. 8

**APRIL-induced NF-kB phospharylation
(HEK293F-BCMA-NF-kB-luc)**

|  | TAB1 | TAB2 | mAbBCMA-003 |
|---|---|---|---|
| EC50 | 6.313 | 6.262 | 6.181 |

Fig. 9

Fig. 10

## FIT-Ig binding with CHOK1/CD3/TCR

Legend:
- ● FIT1006-4a
- ■ FIT1006-4a
- ▲ FIT1006-4a
- ▼

Fig. 11

## Jurkat-NFAT-luc and NCI-H929 Co-Culture

Legend:
- ◯ FIT1006-4b
- ▢ FIT1006-4a
- △ irrelevant FIT-Ig
- ▽ FIT1006-4a-Fab
- ◆ HuEM0006-01-24
- ◯ HuEM0006-01-24-Fab
- ▢ irrelevant human IgG

X-axis: Antibody conc.(n M)
Y-axis: RLU

Fig. 12

**Jurkat-NFAT-luc and NCI-H929 Co-Culture**

- ● FIT1006-3a-Fab
- ■ FIT1006-4a-Fab
- ▲ FIT1006-5a-Fab
- ▼ FIT1006-6a-Fab
- ◆ FIT1006-7a-Fab
- ○ FIT1006-8a-Fab
- □ anti-CD3 mAb +mAbBCMA-002
- △ FIT1006-1a-Fab
- ▽ FIT1002-5a-Fab

Fig. 13

- ●
- ■
- ▲
- ▼
- ◆ combo
- ○
- □ HuEM0006-01-24-Fab
- △

Fig. 14

Fig. 15

Fig. 16

## Cell binding with NCI-H929

- FIT1006-31b(D-T)
- FIT1006-31b(D-T)
- hIgG

Fig. 17

## Cell binding with Jurkat

- FIT1006-31b(D-T)
- FIT1006-35b(D-T)
- hIgG

Fig. 18

Fig. 19

Fig. 20

**NCI-H929 xenograft model**

Fig. 21

**B cell**

- ● vehicle male
- ■ vehicle female
- ▲ FIT1006-31b (D-T) male
- ▼ FIT1006-31b (D-T) female
- ◆ FIT1006-35b (D-T) male
- ● FIT1006-35b (D-T) female

Fig. 22

**T cell**

- ● vehicle male
- ■ vehicle female
- ▲ FIT1006-31b (D-T) male
- ▼ FIT1006-31b (D-T) female
- ◆ FIT1006-35b (D-T) male
- ● FIT1006-35b (D-T) female

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5648260 A
- US 5624821 A
- WO 9005144 A
- US 5641870 A
- WO 2015103072 A

- US 7262028 B
- US 8236308 B
- WO 2012163805 A
- WO 2014122143 A
- WO 2016020332 A

### Non-patent literature cited in the description

- **ANTHONY et al.** *Science,* 2008, vol. 320, 373-376
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **HOLLIGER et al.** *Proc. Natl. Acad. Sci. USA,* 1993, vol. 90, 6444-6448
- Antibody Engineering. Springer-Verlag, 2001, 790
- **ZAPATA et al.** *Protein Eng,* 1995, vol. 8 (10), 1057-1062
- **HOOGENBOOM, H.R.** *Trends Biotechnol.,* 1997, vol. 15, 62-70
- **AZZAZY ; HIGHSMITH.** *Clin. Biochem.,* 2002, vol. 35, 425-445
- **GAVILONDO ; LARRICK.** *BioTechniques,* 2000, vol. 29, 128-145
- **HOOGENBOOM ; CHAMES.** *Immunol. Today,* 2000, vol. 21, 371-378
- **TAYLOR et al.** *Nucl. Acids Res.,* 1992, vol. 20, 6287-6295
- **KELLERMANN ; GREEN.** *Curr. Opin. Biotechnol.,* 2002, vol. 13, 593-597

- **LITTLE et al.** *Immunol. Today,* 2000, vol. 21, 364-370
- **KABAT et al.** Sequences of Proteins of Immunological Interest. National Institutes of Health, 1987
- **KABAT et al.** Ann. NY Acad. Sci. 1971, vol. 190, 382-391
- **KABAT et al.** Sequences of Proteins of Immunological Interest. U.S. Department of Health and Human Services, 1991
- Protein Sequence and Structure Analysis of Antibody Variable Domains. **MARTIN.** Antibody Engineering. Springer-Verlag, 2001, 432-433
- **SAMBROOK et al.** Molecular Cloning: A Laboratory Manual. Cold Spring Harbor Laboratory Press, 1989
- **URLAUB ; CHASIN.** *Proc. Natl. Acad. Sci. USA,* 1980, vol. 77, 4216-4220
- **KAUFMAN ; SHARP.** *J. Mol. Biol.,* 1982, vol. 159, 601-621
- **SECKINGER, A. et al.** Target Expression, Generation, Preclinical Activity, and Pharmacokinetics of the BCMA-T Cell Bispecific Antibody EM801 for Multiple Myeloma Treatment. *Cancer Cell,* 2017, vol. 31 (3), 396-410